(19) Europäisches Patentamt / European Patent Office / Office européen des brevets

(11) **EP 4 023 262 A1**

(12) **EUROPEAN PATENT APPLICATION**
published in accordance with Art. 153(4) EPC

(43) Date of publication:
06.07.2022 Bulletin 2022/27

(21) Application number: 20857473.1

(22) Date of filing: 27.08.2020

(51) International Patent Classification (IPC):
$A61L\ 27/02^{(2006.01)}$  $A61K\ 47/02^{(2006.01)}$
$A61L\ 27/12^{(2006.01)}$  $A61L\ 27/56^{(2006.01)}$
$C01B\ 25/32^{(2006.01)}$  $C01F\ 11/18^{(2006.01)}$
$C12M\ 3/00^{(2006.01)}$

(52) Cooperative Patent Classification (CPC):
A61K 47/02; A61L 27/02; A61L 27/12; A61L 27/20;
A61L 27/40; A61L 27/56; C01B 25/32; C01F 11/18;
C12M 3/00

(86) International application number:
PCT/JP2020/032323

(87) International publication number:
WO 2021/039892 (04.03.2021 Gazette 2021/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.08.2019 JP 2019154607

(71) Applicant: GC Corporation
Sunto-gun, Shizuoka 410-1307 (JP)

(72) Inventors:
• TSURU, Kanji
Fukuoka-shi, Fukuoka 814-0164 (JP)
• TOITA, Riki
Ikeda-shi Osaka 563-0026 (JP)
• NAKASHIMA, Yasuharu
Fukuoka-shi Fukuoka 813-0017 (JP)
• ISHIKAWA, Kunio
Kasuya-gun, Fukuoka 811-2201 (JP)

(74) Representative: TBK
Bavariaring 4-6
80336 München (DE)

(54) **MEDICAL CALCIUM CARBONATE COMPOSITION, RELATED MEDICAL COMPOSITIONS, AND PRODUCTION METHODS THEREFOR**

(57) Provided is a medical calcium carbonate composition that highly satisfies 1) tissue affinity, 2) *in vivo* resorbability, 3) reactivity, and 4) mechanical strength required for medical materials to be implanted *in vivo,* a medical calcium phosphate composition, a medical carbonate apatite composition, a medical calcium hydroxide porous structure, a medical calcium sulfate setting granules, and a bone defect regeneration kit related to the medical calcium carbonate composition, and methods for producing these. The medical composition calcium carbonate that highly satisfies the above described elements, and related medical compositions can be produced by controlling the polymorph or structure of calcium carbonate.

**Fig. 3**

$2\theta(degree)$

**Description**

Technical Field

The present invention relates to a medical composition and a method for producing the same. Specifically, the present invention relates to a medical calcium carbonate composition to be implanted *in vivo,* a scaffold for cell culture for *ex vivo* use, and a medical calcium sulfate setting composition, a medical calcium phosphate composition, a medical calcium hydroxide composition, and a bone defect reconstruction kit related to the composition, and methods for producing these.

More specifically, the present invention relates to a medical calcium carbonate composition that highly satisfies 1) tissue affinity, 2) *in vivo* resorbability, 3) reactivity, and 4) mechanical strength, and related medical compositions, and methods for producing these.

Background Art

The skeletons of invertebrates are made of calcium carbonate, and the skeletons of vertebrates are made of carbonate apatite, a kind of calcium phosphate, having phosphoric acid component added to calcium carbonate. Calcium carbonate has been studied as a bone graft material, and calcium phosphate including carbonate apatite, calcium sulfate, and the like have been clinically applied to bone graft materials.

(Tissue affinity)

Medical compositions such as bone graft materials differ in required properties from industrial compositions, and *in vivo* reaction is most important. The *in vivo* implantation of powders evokes inflammatory reaction. Hence, medical compositions to be implanted *in vivo* are required to have a volume above a certain level from the viewpoint of tissue affinity. They may be required to have antimicrobial properties from the viewpoint of infection prevention. Substantial purity is also essential subject matter for the medical compositions.

(*In vivo* resorbability)

It may be expected for bone graft materials that medical calcium carbonate compounds and related medical compositions are resorbed *in vivo* and replaced with desired tissues. Tissue replacement requires both material resorption and tissue regeneration. Although calcium carbonate or some kinds of calcium phosphate is resorbed by osteoclasts, etc., the resorption requires the absence of a material that is not resorbed *in vivo.*

(Reactivity)

For medical calcium carbonate compositions, excellent tissues reactivity such as *in vivo* tissue replacement may be desired, or chemical reactivity may be desired. For the former, the infiltration or lysis of tissues, cells, or tissue fluids becomes an element, and pore control, polymorph, or crystallite size is important. For the latter, the infiltration or lysis of aqueous solutions becomes an element, and pore control, polymorph, or crystallite size is important.

As for the latter, medical calcium carbonate compositions are not only expected as bone graft materials but also useful as precursors in the production of medical calcium phosphate compositions such as medical carbonate apatite compositions. For example, when a calcium carbonate block is immersed in aqueous phosphoric acid salt solution, its composition becomes carbonate apatite through deposition reaction while keeping macrostructure so that a carbonate apatite block can be produced (Patent Literature 1). However, dissolution and deposition reaction proceeds from the surface of the calcium carbonate block. Therefore, the core may remain without complete compositional transformation into carbonate apatite, etc. when the calcium carbonate block is large or when the calcium carbonate block has low reactivity. Hence, there is a demand on production methods that involve medical calcium carbonate compositions having high reactivity or rapid addition of phosphoric acid component.

The reactivity of calcium carbonate compositions is largely influenced not only by composition or polymorph but also by structure. Particularly, interconnected porous structures are preferred structures because cells or tissues migrate into the inside. Cell or tissue migration requires macropores of size over a certain level, whereas micropores of smaller size are also important for expected tissue replacement.

(Mechanical strength)

In general, more macropores and micropores are more desirable. However, mechanical strength is reduced with increase in porosity. Hence, the balance between porosity and mechanical strength is important.

Medical calcium phosphate compositions produced from medical calcium carbonate compositions as well as medical calcium hydroxide compositions or medical calcium sulfate compositions necessary for medical calcium carbonate composition production, and bone defect reconstruction kits are also important as related medical compositions.

(Reactivity of calcium carbonate: polymorph, pore and density)

Various factors such as polymorph, pores, and density influence the reactivity of calcium carbonate. Vaterite is metastable at ordinary temperature and pressure and does not occur naturally, and however, is calcium carbonate having the highest reactivity. Its density is 2.64 (g/cm$^3$). Calcite is a stable phase at ordinary temperature and pressure and has lower reactivity than that of vaterite. Its density is 2.71 (g/cm$^3$). Aragonite is a stable phase at high temperature and pressure and is a metastable phase at ordinary temperature and pressure. Its density is 2.96 (g/cm$^3$). When a medical material is produced using calcium carbonate having a high density as a raw material, the produced medical material may have low reactivity due to its high density. Hence, calcium carbonate having a relatively small density may be desired.

Pores have the largest influence on the reactivity of calcium carbonate. In general, higher porosity gives higher reactivity. In this relation, in the case of using calcium carbonate as a raw material to produce another material, a material having high reactivity may be produced using calcium carbonate having a small density.

For these reasons, the calcium carbonate according to the present invention is limited to vaterite and calcite.

(Background of vaterite composition)

Vaterite is a metastable phase and has higher reactivity not only than the stable phase calcite but also than even the metastable phase aragonite, and as such, is very preferred as a medical calcium carbonate composition. Any medical vaterite composition containing 20 mass% or larger of vaterite and having a volume of 10$^{-12}$ m$^3$ or larger has been absent so far.

It is known that vaterite powders can be produced by, for example, a method for producing calcium carbonate through the reaction of water soluble calcium salt and carbonic acid salt in aqueous solution, comprising delaying transfer to calcite by adding divalent cation other than calcium (Patent Literatures 2 and 3), a method of controlling the Ca concentration, temperature, and pH of slurry in carbonating calcium chloride or calcium nitrate (Patent Literatures 4 and 5), a method of passing O/W emulsion consisting of a continuous aqueous phase containing calcium ion dissolved therein, and an organic phase through a porous membrane, followed by reacting with water containing carbonate ion solution (Patent Literature 6), a method of introducing carbon dioxide to alcohol-water mixed suspension of calcium hydroxide (Patent Literature 7), a method of adding an alkylamine salt type surfactant (Non Patent Literature 1), or a method of adding an organic material such as ethylene glycol (Non Patent Literature 2). However, such powders evoke inflammatory reaction *in vivo* and as such, cannot be used.

The present inventor has found that a method for producing calcite granules containing 17 mass% of vaterite by immersing calcium sulfate anhydrous granules in 50 mL of 2 mol/L aqueous sodium carbonate solution of 4°C for 14 days. However, in this production method, the content of vaterite was 17 mass% because calcite formation cannot be sufficiently inhibited (Patent Literature 8). As described in Patent Literature 8, it is considered that "when a product inorganic compound comprising vaterite-containing calcium carbonate is produced, it is essential to set a temperature of an electrolyte to be 10°C or less", presenting problems associated with products and production (Patent Literature 8).

Specifically, neither any medical calcium carbonate composition that contains 20 mass% or larger of vaterite and satisfies all of the aforementioned conditions of medical calcium carbonate compositions, nor any method for producing the same has been known. Any method for producing vaterite at a temperature exceeding 10°C has not been known. Specifically, any medical material of size over a certain level that highly inhibits calcite formation and contains 20 mass% or larger of vaterite has not been known. As a matter of course, any sintered body containing vaterite has not been known.

(Background of calcite composition)

Calcite, which is stable phase calcium carbonate, is relatively easy to produce as granules, blocks, etc., and, for example, a method of exposing a calcium hydroxide compact to carbon dioxide has been reported (Patent Literature 9).

On the other hand, the reactivity of the stable phase calcite is lower as compared with the metastable phase vaterite or aragonite. In the case of producing calcium phosphate by adding phosphoric acid salt to calcite, calcium phosphate formation reaction proceeds from the surface of a calcite composition. Hence, the core of calcium carbonate may remain, thereby making it substantially impossible to produce a calcium phosphate composition, requiring a reaction temperature higher than 100°C, or requiring a long time for production.

The preparation of interconnected porous structures is effective for enhancing the apparent reactivity of medical calcium carbonate compositions. For example, in the case of producing a medical calcium phosphate composition from medical calcite having a volume over a certain level, a precise body may cause the core to remain or may require a long production time, whereas an interconnected porous structure having the same volume enables a medical calcium phosphate composition to be produced in a short production time without the core remaining because the reaction proceeds from the surface of the porous structure. Such an interconnected porous structure permits invasion of cells or tissues to the inside. For example, carbonate apatite interconnected porous structures drastically enhance bone replacement.

Various studies have been conducted so far on medical calcium carbonate porous structures serving as precursors of medical carbonate apatite porous structures. For example, a method for producing a calcium carbonate porous structure by mixing calcium hydroxide with a pore forming substance such as sodium chloride, compacting and then carbonating the mixture, and removing the pore forming substance has been proposed (Patent Literature 9). Although reactivity is improved by porous structure formation, further improvement in reactivity has been desired due to calcite porous structures. Limitations on pore size are also very important from the viewpoint of mechanical strength and reactivity. However, any specific pore size was not known at that time.

Calcium carbonate is decomposed and therefore has previously been considered difficult to sinter. A method for conveniently producing sintered calcium carbonate by adding a foaming agent to dispersion containing calcium carbonate and a gelling agent, stirring the mixture, and sintering the foam has been proposed (Patent Literature 10). In this production method, interconnected pores are formed and however, are in a form where large pores from several hundreds of microns to 1 mm or larger have been sintered, and are also inferior in reactivity due to large wall thickness. Furthermore, reproducibility is poor because the pores are formed by foaming. As described in Reference Example 1 of Patent Literature 10, no porous sintered calcium carbonate is obtained in the first place without adding sintering aids potassium carbonate and lithium carbonate. Bone graft materials supplemented with potassium or lithium are unfavorable. Calcium carbonate porous sintered bodies can be produced by adding sintering aids or by using high-purity calcium carbonate and however, have small mechanical strength and poor clinical practicality.

A debindering method related to ceramic porous structure production, comprising heating thermal fusion type resin beads to a temperature equal to or higher than their decomposition start temperature at a heating rate of 30°C/h or faster has been proposed as debindering in producing a ceramic porous structure using thermal fusion type resin beads as porogen (Patent Literature 11). Although this method is useful for thermally stable ceramics such as alumina, its usefulness is limited for ceramics, such as calcium carbonate, which are decomposed at high temperature. Furthermore, a higher level of a debindering method is necessary for medical compositions expected to have high reactivity. An approach of mixing porogen with raw material ceramic accurately adjusts pore size. However, for forming interconnected porous structures, it is necessary to introduce a relatively large amount of porogen. The introduction of appropriate porogen is required because the mechanical strength of produced ceramic porous structures is markedly reduced with increase in porosity.

The present inventor has proposed a method for producing a calcium carbonate honeycomb structure by extruding polymer material containing-calcium hydroxide through a honeycomb structure formation mold, and debindering the polymer material, followed by carbonation treatment, or performing the debindering and carbonation treatment of the polymer material at the same time (Patent Literature 12) as a method for producing a calcium carbonate interconnected porous structure. The present inventor has also disclosed that a carbonate apatite honeycomb structure can be produced by adding phosphoric acid component to the calcium carbonate honeycomb structure of the invention. The calcium carbonate honeycomb structure and the carbonate apatite honeycomb structure exhibited osteoconductivity and exhibited excellent properties such as a high level of orientation of the conducted bones along the through-holes direction. As a result of making diligent research and development on the carbonate apatite honeycomb structure, the debindering of the calcium carbonate honeycomb structure has been found insufficient, and the possibility has been found that a highly functional medical calcium carbonate composition can be produced by improving the debindering level. Specifically, the production of the calcium carbonate honeycomb structure of the invention employed polymer material such as a wax-based organic binder and therefore involved debindering. At that time, the desired degree of debindering was judged as differing depending on the required degree of whiteness. Furthermore, neither was the debindering level required for medical calcium carbonate compositions elucidated, nor any method for quantifying the degree of debindering was devised. Moreover, it was inconceivable that remaining materials after acid dissolution depending on the degree of debindering had large influence on the reactivity or usefulness of calcium carbonate honeycomb structures.

Hence, a calcium carbonate honeycomb structure disclosed in Example 1 of Patent Literature 12 (Comparative Example 7 of the present specification) contained 1.2 mass% of remaining materials after acid dissolution. A carbonate apatite honeycomb structure (Example 11 of Patent Literature 12) produced by adding phosphoric acid component to the calcium carbonate honeycomb structure also contained 1.2 mass% of remaining materials after acid dissolution. At that time, these remaining materials after acid dissolution were not presumed to necessarily influence tissue affinity for a long period, though being responsible for coloring. In actuality, even the carbonate apatite honeycomb structure containing 1.2 mass% of remaining materials after acid dissolution was confirmed to have a certain level of osteoconductivity and excellent tissue affinity.

As a result of conducting diligent studies on further improvement in the functions of the carbonate apatite honeycomb structure, even a small amount of remaining materials after acid dissolution was found to influence osteoconductivity or bioresorbability. Hence, the present inventor has pursued diligent studies on medical calcium carbonate honeycomb structures wherein remaining materials after acid dissolution are 1 mass% or less, if possible, remaining materials after acid dissolution are 0 mass%.

For medical calcium carbonate honeycomb structures, not only macropores that form an interconnected structure, but also micropores are important. This is because not only are macropores useful for the invasion of tissues or cells, but micropores are useful for accelerating the resorption of medical calcium carbonate porous structures by cells, body fluids, etc. or reaction with aqueous solution. However, neither any method for identifying micropores useful for medical calcium carbonate compositions nor the effective range of the micropores has been found.

Citation List

Patent Literature

Patent Literature 1: Patent Republication No. WO 2004/112856
Patent Literature 2: Japanese Patent Laid-Open Publication No. S57-92520
Patent Literature 3: Japanese Patent Laid-Open Publication No. S60-90822
Patent Literature 4: Japanese Patent Laid-Open Publication No. S54-150397
Patent Literature 5: Japanese Patent Laid-Open Publication No. 2011-126741
Patent Literature 6: Japanese Patent Laid-Open Publication No. 2011-157245
Patent Literature 7: Japanese Patent Laid-Open Publication No. H11-314915
Patent Literature 8: International Publication No. WO 2016/035751
Patent Literature 9: Japanese Patent Laid-Open Publication No. 2016-552061
Patent Literature 10: Japanese Patent Laid-Open Publication No. 2018-140890
Patent Literature 11: Japanese Patent Laid-Open Publication No. H7-223871
Patent Literature 12: International Publication No. WO 2018/074429

Non Patent Literature 1: Journal of the Adhesion Society of Japan, Vol. 22, No. 11, 1986, pp. 573-579
Non Patent Literature 2: The Journal Of the Society Of Materials Science, Japan, Vol. 30, No. 336, 1986, pp. 6-10

Summary of Invention

Technical Problem

An object of the present invention is to provide a medical calcium carbonate composition that highly satisfies 1) tissue affinity, 2) *in vivo* resorbability, 3) reactivity, and 4) mechanical strength, a medical calcium sulfate setting composition, a medical calcium phosphate composition, a medical calcium hydroxide composition, and a bone defect reconstruction kit related to the composition, and methods for producing these.

Solution to Problem

The present inventor has conducted diligent studies and consequently completed the present invention by finding that a medical calcium carbonate composition that highly satisfies 1) tissue affinity, 2) *in vivo* resorbability, 3) reactivity, and 4) mechanical strength, a medical calcium sulfate hemihydrate composition, a medical calcium phosphate composition, a medical calcium hydroxide composition, and a bone defect reconstruction kit related to the composition, and methods for producing these can be provided.

Specifically, the present invention is as follows.

[1] A medical calcium carbonate composition that satisfies all conditions of the following (A) - (C), and at least one condition selected from the group consisting of (D) - (K):

(A) a volume is $10^{-12}m^3$ or larger;

(B) remaining materials after acid dissolution are 1.0 mass% or less.

(C) it is substantially a pure calcium carbonate as a medical composition, and mainly comprises vaterite or calcite;

(D) it contains 20 mass% or larger of vaterite;

(E) it is a honeycomb structure comprising a plurality of through-holes extending in one direction, wherein a volume of pores which pore diameter is 10 $\mu$m or smaller with respect to a mass of the honeycomb structure analyzed by mercury intrusion porosimetry is larger than 0.02 cm$^3$/g;

(F) it is a granule bonded-porous structure comprising a plurality of granules which maximum diameter is 50 $\mu$m or longer and 500 $\mu$m or shorter, formed by being bonded to each other, and comprising a plurality of through-holes extending in plural directions, wherein a volume of pores with a pore diameter of 10 $\mu$m or smaller analyzed by mercury intrusion porosimetry is 0.05 cm$^3$/g or more;

(G) it is a pore integrated-type porous structure wherein a plurality of pores which maximum diameter is 50 $\mu$m or longer and 400 $\mu$m or shorter is integrated to the whole medical composition, not containing pores which maximum diameter is 800 $\mu$m or longer, wherein a volume of pores which maximum diameter is 10 $\mu$m or smaller in the pore integrated-type porous structure analyzed by mercury intrusion porosimetry is 0.05 cm$^3$/g or more;

(H) a ratio of pore volume which pore diameter is 1 $\mu$m or larger and 6 $\mu$m or shorter with respect to a pore volume which pore diameter is 6 $\mu$m or shorter analyzed by mercury intrusion porosimetry is 10 % or more;

(I) a maximum compressive strength obtained at any one direction is higher than a standard compressive strength [S] that is calculated by the following equation (with the proviso that a honeycomb structure comprising a plurality of through-holes extending in one direction, wherein a volume of pores with a pore diameter of 10 $\mu$m or smaller with respect to a mass of the honeycomb structure analyzed by mercury intrusion porosimetry is 0.02 cm$^3$/g or smaller is excluded)

$$S = S_0 \times C \times \exp(-b \times P)$$

(wherein So and b are constant number, So is 500, b is 0.068, and C is a constant number based on polymorph of calcium carbonate; C is 0.01 when the calcium carbonate contains 20 mass% or larger of vaterite, and is 1 when the calcium carbonate does not contain 20 mass% or larger of vaterite; and P is a percentage of pores in the composition.)

(J) it is a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists;

(K) plural composition particles are connected with a fiber.

[2] The medical calcium carbonate composition according to [1], wherein the above described (D) condition is satisfied, and the medical calcium carbonate composition is a sintered body.

[3] The medical calcium carbonate composition according to [1] or [2], wherein calcium carbonate powders that satisfy at least one of the following (AJ1) to (AJ4) conditions, are bonded to form the calcium carbonate composition:

(AJ1) a mean particle diameter is 2 $\mu$m or larger, and 8 $\mu$m or smaller;

(AJ2) a sphericity is 0.9 or larger.

(AJ3) Mg content is $5 \times 10^{-4}$ mass% or larger, and $3 \times 10^{-3}$ mass% or smaller;

(AJ4) Sr content is $3 \times 10^{-3}$ mass% or larger, and $1.5 \times 10^{-2}$ mass% or smaller.

[4] The medical calcium carbonate composition according to [1] or [3], wherein the above described (E) condition is satisfied, which is a bended honeycomb structure wherein a diameter of the circle that passes through both ends of any one of the through-holes and a center of the through-hole, is 1 cm or longer, and 50 cm or shorter.

[5] A method for producing the medical calcium carbonate composition according to [1] or [3] that satisfies the above described (D) condition, wherein:

in a process of exposing raw material calcium composition which volume is $10^{-12}$m$^3$ or larger to carbon dioxide or carbonate ion, at least one of the following conditions selected from (D1) to (D8) group is satisfied, and optionally comprises the following (D9) to (D12) process:

(D1) a process of inhibiting calcite formation or calcite crystal growth, and relatively promoting vaterite formation;

(D2) a process of exposing of raw material calcium composition to carbon dioxide or carbonate ion, and at least one selected from the group consisting of organic solvent, water soluble organic material, ammonia, and ammonium salt;

(D3) a process of exposing raw material calcium composition that contains at least one selected from the group consisting of organic solvent, water soluble organic material, ammonia, and ammonium salt, to carbon dioxide or carbonate ion, and at least one selected from the group consisting of organic solvent, water soluble organic material, ammonia, and ammonium salt;

(D4) a process of exposing raw material calcium composition to carbon dioxide or carbonate ion, and at least one selected from the group consisting of methanol, ethanol, glycerin, ethylene glycol, and ammonium carbonate.

(D5) a process of exposing raw material calcium composition that contains at least one selected from the group consisting of methanol, ethanol, glycerin, ethylene glycol, and ammonium carbonate, to carbon dioxide or carbonate ion, and at least one selected from the group consisting of methanol, ethanol, glycerin, ethylene glycol, and ammonium carbonate;

(D6) a process of inhibiting transfer from vaterite to calcite;

(D7) a process of removing water from the raw material calcium composition;

(D8) a process of circulating carbon dioxide or carbonate ion containing organic solvent around the raw material calcium composition;

(D9) a process of partial carbonation by exposing raw material calcium composition to carbon dioxide or carbonate ion under gas phase, followed by exposing the raw material calcium composition to carbon dioxide or carbonate ion under liquid phase; (D10) a process of exposing raw material calcium composition in a mold to carbon dioxide or carbonate ion;

(D11) a process of exposing raw material calcium composition that contains porogen to carbon dioxide or carbonate ion;

(D12) a process of exposing raw material calcium compositions that are connected with a fiber to carbon dioxide or carbonate ion;

[6] A method for producing the medical calcium carbonate composition according to [1] or [2] that satisfies the above described (D) condition, comprising:
compacting and sintering calcium carbonate powder that contains 20 mass% or larger of vaterite.

[7] A method for producing the medical calcium carbonate composition according to [1], [3] or [4] that satisfies the above described (E) condition, comprising:

the following process (E1) and one process selected from the group consisting of (E5) to (E9) as essential process, and optionally one process selected from the following (E2) to (E4), and (E10)

(E1) Extrusion process
a process of producing a raw honeycomb structure comprising a plurality of through-holes extending in one direction, having a volume of $3 \times 10^{-11} m^3$ or larger by extruding a raw material calcium composition comprising polymer material through a honeycomb structure forming die;

(E2) Forming process after extrusion process
A process of forming honeycomb structure consisting of a raw material calcium composition comprising polymer material to a desired form by softening by a thermal treatment, followed by pressure loading;

(E3) Removal process of peripheral wall
A process of removing peripheral wall after the extrusion process or the forming process after the extrusion process, and before a debindering and carbonation process; (E4) Forming process after removal process of peripheral wall
A process of forming a honeycomb structure consisting of a raw material calcium composition comprising polymer material to a desired form through softening by thermal treatment, after removal process of peripheral wall;

(E5) Debindering and calcium carbonate sintering process
a process of heat debindering of polymer material-containing calcium carbonate so that remaining materials after acid dissolution is 1 mass% or smaller, and sintering the calcium carbonate;

(E6) Debindering and carbonation process
a process of heat debindering of a polymer material containing-calcium hydroxide porous structure so that remaining materials after acid dissolution is 1 mass% or smaller under an oxygen concentration of less than 30%, and carbonation at the same time;

(E7) Debindering and carbonation process via calcium oxide
a process of heat debindering a polymer material containing-calcium hydroxide porous structure or polymer material containing-calcium carbonate porous structure so that remaining materials after acid dissolution is 1 mass% or smaller, and to be calcium oxide porous structure, followed by exposing the calcium oxide porous structure to carbon dioxide to be a calcium carbonate porous structure;

(E8) Debindering and carbonation process via calcium carbonate and calcium oxide

a process of heat treatment of a polymer material-containing calcium hydroxide under carbon dioxide atmosphere to be a polymer material containing-calcium carbonate porous structure, followed by heat debindering so that remaining materials after acid dissolution is 1 mass% or smaller, and to be a calcium oxide porous structure, followed by exposing the calcium oxide porous structure to carbon dioxide to be a calcium carbonate porous structure;

(E9) Debindering and carbonation process of calcium sulfate

a process of heat debindering of a polymer material containing-calcium sulfate so that remaining materials after acid dissolution is 1 mass% or smaller, followed by adding carbon dioxide or carbonate ion to the produced calcium sulfate porous structure to be a calcium carbonate;

(E10) a process of structure finishing process after debindering and carbonation processes.

[8] A method for producing the medical calcium carbonate composition according to [7], that satisfies at least one of the conditions selected from the following conditions (E11) to (E14):

(E11) in the above-described "(E1) Extrusion process", honeycomb structure is extruded so that thickness of peripheral wall of the honeycomb structure is thicker than that of the partition wall, and cross-sectional area vertical to the through-holes is 1 cm$^2$ or larger;

(E12) in at least one of the processes of "(E1) Extrusion process", "(E2) Forming process after extrusion process", "(E4) Forming process after removal process of peripheral wall", and "(E10) Structure finishing process after debindering and carbonation processes", a heat softened honeycomb structure comprising raw material calcium composition containing polymer material is bended by applying a pressure so that the diameter of the circle that passes through both ends and the center of one of the through-holes, is 1 cm or longer, and 50 cm or shorter;

(E13) the "(E3) Removal process of peripheral wall" is done with a cutter grinder, and the "(E10) process of structure finishing process after debindering and carbonation processes" is done by polishing;

(E14) a raw material calcium composition of the "(E1) Extrusion process" is calcium sulfate anhydrous.

[9] A method for producing the medical calcium carbonate composition according to [1] that satisfies the above described (F) condition, comprising:

the following (F1) and (F2) processes, and at least one of the (F3) or (F4) process. (F1) Placement-closing process

placing calcium oxide granules in a reaction vessel, and closing the opening of the vessel so that the granules are not escaped from the reaction vessel;

(F2) Porous structure producing process

a process of producing a porous structure by adding water or acetic acid to the calcium oxide granules inside the reaction vessel to make calcium hydroxide or calcium acetate;

(F3) Carbonation process

a carbonation process to produce a calcium carbonate porous structure by adding carbon dioxide to calcium hydroxide porous structure at the same time or after the porous structure producing process, or a carbonation process to produce a calcium carbonate porous structure by heat treatment of calcium acetate after porous structure producing process;

(F4) Calcium oxide carbonation process

a carbonation process producing a calcium carbonate porous structure by heat treatment of at least one selected from a group consisting of calcium hydroxide porous structure, calcium carbonate porous structure, and calcium acetate porous structure, followed by exposing the calcium oxide porous structure to carbon dioxide.

[10] A method for producing the medical calcium carbonate composition according to [1], that satisfies the above described (F) condition, and comprising the following (F5) and (F6) processes; or comprising the following (F5), (F7), and (F9) processes, and optionally comprising the (F8) process:

(F5) Placement process

a process of placing calcium sulfate granules in a reaction vessel;

(F6) Porous structure forming-carbonation process

a process of changing composition to calcium carbonate by reacting calcium sulfate granules in the reaction vessel with carbonate ion, and hardening granules each other to form porous structure;

(F7) Porous structure forming process

a process of forming calcium sulfate dihydrate porous structure by adding water to calcium sulfate hemihydrate granules or calcium sulfate anhydrous granules;

(F8) Heat-treatment process

a process of producing calcium sulfate anhydrous porous structure by heat-treatment of calcium sulfate dihydrate porous structure;

(F9) Carbonation process

a process of changing composition to calcium carbonate by exposing calcium sulfate dihydrate porous structure or calcium sulfate anhydrous porous structure to water containing carbonate ion.

[11] A method for producing the medical calcium carbonate composition according to [1] that satisfies the above described (F) condition, comprising the following (F10), (F11) and one selected from the group of (F12) to (F16) as essential processes, and optionally comprising the (F17) process:

(F10) Placement process

a process of placing raw material calcium composition granules containing polymer having a volume of $10^{-12}$ $m^3$ or larger in a reaction vessel;

(F11) Porous structure forming process

A process of producing granules bonded-porous structure formed from a plurality of granules which maximum diameter is 50 $\mu$m or longer and 500 $\mu$m or shorter bonded to each another, and comprising a plurality of through-holes extending in plural directions, and having a volume of $3 \times 10^{-11}$ $m^3$ by bonding the granules in the reaction vessel by heat fusing, or by fusing of the surface of granules to bond the surface of the granules one to another, or by fusing the surface of granules one to another with a plasticizer.

(F12) Debindering and calcium carbonate sintering process

a process of heat debindering of polymer material containing-calcium carbonate so that remaining materials after acid dissolution are 1 mass% or smaller, and sintering the calcium carbonate;

(F13) Debindering and carbonation process

a process of heat debindering of polymer material containing calcium hydroxide porous structure so that remaining materials after acid dissolution are 1 mass% or smaller under oxygen concentration of less than 30%, and carbonation at the same time;

(F14) Debindering and carbonation process via calcium carbonate and calcium oxide a process of heat treatment of polymer material containing-calcium hydroxide porous structure or polymer material containing-calcium carbonate porous structure so that remaining materials after acid dissolution are 1 mass% or smaller, and to be calcium oxide porous structure, followed by exposing the calcium oxide porous structure to carbon dioxide to be calcium carbonate porous structure;

(F15) Debindering and carbonation process via calcium carbonate and calcium oxide Debindering and carbonation process via calcium carbonate and calcium oxide, comprising heat treatment of polymer material-containing calcium hydroxide porous structure in the presence of carbon dioxide to produce polymer material-containing calcium carbonate porous structure, followed by heat debindering so that remaining materials after acid dissolution are 1 mass% or smaller, and to be calcium oxide porous structure, followed by exposing the calcium oxide porous structure to carbon dioxide to be calcium carbonate porous structure;

(F16) Calcium sulfate debindering and carbonation process

a debindering and carbonation process of producing calcium carbonate by heat debindering of polymer material-containing calcium sulfate so that remaining materials after acid dissolution are 1 mass% or smaller, followed by adding carbon dioxide or carbonate ion to the produced calcium sulfate porous structure.

(F17) A process of structure finishing process after debindering and carbonation processes.

[12] A method for producing the medical calcium carbonate composition according to [1] that satisfies the above described (G) condition, comprising (G1) and at least one process selected the group consisting of (D1) to (D10) and (E5) to (E9) as essential process, and optionally comprising (G2), (G3), and (E10):

(G1) Mixing process

A process of mixing raw material calcium composition powder or raw material calcium composition paste, and porogen;

(G2) Compacting process

A process of compacting raw material calcium composition powder or raw material calcium composition paste, and porogen;

(G3) Porogen removal process

A process of removing porogen by dissolving the porogen into a solvent;

(D1) A process of inhibition of calcite formation or calcite crystals' growth, and promoting relative vaterite formation;

(D2) A process comprising exposing raw material calcium composition to carbon dioxide or carbonate ion, and at least one selected from a group of organic solvent, water soluble organic material, ammonia, and ammonium salts;

(D3) A process comprising exposing raw material calcium composition that contains at least one selected from the group consisting of organic solvent, water soluble organic materials, ammonia, and ammonium salts, to carbon dioxide or carbonate ion, and at least one selected from a group of organic solvent, water soluble organic materials, ammonia, and ammonium salts;

(D4) A process comprising exposing raw material calcium composition to carbon dioxide or carbonate ion, and at least one selected from the group consisting of methanol, ethanol, and ammonium carbonate;

(D5) A process comprising exposing raw material calcium composition that contains at least one selected from the group consisting of methanol, ethanol, and ammonium carbonate, to carbon dioxide or carbonate ion, and at least one selected from the group consisting of methanol, ethanol, and ammonium carbonate;.

(D6) A process comprising inhibiting transfer from vaterite to calcite;

(D7) A process comprising removing water from the raw material calcium composition;

(D8) A process comprising circulating carbon dioxide or carbonate ion containing organic solvent around the raw material calcium composition;

(D9) A process comprising partial carbonation by exposing raw material calcium composition to carbon dioxide or carbonate ion under gas phase, followed by exposing the raw material calcium composition to carbon dioxide or carbonate ion under liquid phase;

(D10) A process comprising exposure of raw material calcium composition in a mold to carbon dioxide or carbonate ion;

(E5) Debindering and calcium carbonate sintering process

A process of heat debindering of polymer material containing-calcium carbonate so that remaining materials after acid dissolution is 1 mass% or smaller, and sintering the calcium carbonate;.

(E6) Debindering and carbonation process

A process of heat debindering polymer material containing-calcium hydroxide so that remaining materials after acid dissolution are 1 mass% or smaller under oxygen concentration of less than 30%, and carbonation at the same time.

(E7) Debindering and carbonation process via calcium oxide

A process of heat debindering polymer material containing- calcium hydroxide or calcium carbonate so that remaining materials after acid dissolution are 1 mass% or smaller, and to be calcium oxide porous structure, followed by exposing the calcium oxide porous structure to carbon dioxide to be calcium carbonate porous structure.

(E8) Debindering and carbonation process via calcium carbonate and calcium oxide

A process of heat treatment of polymer material containing-calcium hydroxide under carbon dioxide atmosphere to be polymer material containing-calcium carbonate, followed by heat debindering so that remaining materials after acid dissolution is 1 mass% or smaller, and to be calcium oxide porous structure, followed by exposing the calcium oxide porous structure to carbon dioxide to be calcium carbonate porous structure.

(E9) Debindering and carbonation process of calcium sulfate

A process of heat debindering of polymer material-containing calcium sulfate so that remaining materials after acid dissolution are 1 mass% or smaller, followed by adding carbon dioxide or carbonate ion to the produced calcium sulfate porous structure to be calcium carbonate.

(E10) A process of structure finishing process after debindering and carbonation processes.

[13] A method for producing the medical calcium carbonate composition according to any one of [7], [8], [11] or [12], wherein:

the above described heat debindering is done at 200 °C or higher, and mass decrease of the polymer material of polymer material containing-calcium composition is smaller than 1 mass%/min,

[14] A method for producing the medical calcium carbonate composition according to any one of [5] to [13] comprising at least one process selected from a group of below described (L) to (Q) as essential process:

(L) A process of debindering done at an oxygen partial pressure of 30 KPa or higher;

(M) A process of debindering or carbonation done at carbon dioxide partial pressure of 30 KPa or higher;

(N) A process of debindering or carbonation done at 150 KPa or higher under atmosphere that contains oxygen or carbon dioxide;

(O) A process of increasing carbon dioxide concentration in the reaction vessel by replacing air in the reaction vessel partially or completely with carbon dioxide, followed by introduction of carbon dioxide in the reaction vessel;
(P) A process of supplying carbon dioxide so that the pressure of the closed reaction vessel is a constant value;
(Q) A carbonation process of mixing or circulating carbon dioxide in the reaction vessel.

[15] A method for producing the medical calcium carbonate composition according to any one of [5] to [9] and [11] to [14], wherein:
a composition of the raw material calcium composition is one selected from the group consisting of calcium oxide, calcium hydroxide, and calcium carbonate.

[16] A method for producing the medical calcium carbonate composition according to any one of [5] to [15], wherein:
at least one condition selected from the following (R1) to (R4) is satisfied:

(R1) Using calcium carbonate powder with an average particle diameter of 2 $\mu$m and larger, and 8 $\mu$m and smaller;
(R2) Using calcium carbonate powder with a sphericity of 0.9 or higher;
(R3) Using calcium carbon powder containing $5 \times 10^{-4}$ mass% or larger, and $3 \times 10^{-3}$ mass% or smaller of Mg;
(R4) Using calcium carbon powder containing $3 \times 10^{-3}$ mass% or larger, and $1.5 \times 10^{-2}$ mass% or smaller of Sr.

[17] A medical calcium sulfate setting composition that satisfies all the following (T1) to (T5) conditions.

(T1) The remaining materials after acid dissolution is 1.0 mass% or less;
(T2) The volume is $5 \times 10^{-1\,3}$m$^3$ or larger;
(T3) it is substantially a pure calcium sulfate as a medical composition;
(T4) content of calcium sulfate hemihydrate production is 50 mass% or more;
(T5) Forming a porous structure with compressive strength of 0.3MPa or higher upon setting reaction when immersed in water while the compositions are contacted one another.

[18] A method for producing the medical calcium sulfate setting composition according to [17],
comprising the following (U2) and (U3) as essential processes, and optionally comprising (U1) and/or (U4) to produce calcium sulfate hemihydrate production:

(U1) Polymer debindering process
A process of debindering polymer material-containing calcium sulfate granules or block by thermal treatment so that the remaining materials after acid dissolution are 1.0 mass% or less;
(U2) Calcium sulfate dihydrate production process
A process of producing calcium sulfate dihydrate granules or block by adding water to calcium sulfate anhydrous or hemihydrate granules or block produced by the polymer debindering process, or by adding water to calcium sulfate hemihydrate powder, and followed by hardening;
(U3) Calcium sulfate hemihydrate production process
A process of producing calcium sulfate hemihydrate granules or block by dehydration of calcium sulfate dihydrate granules or block under gaseous phase;
(U4) Granules size adjusting process
A process of adjusting granules size so that the volume is $5 \times 10^{-13}$ m$^3$ or larger.

[19] A medical calcium phosphate composition that satisfies all the following (V1) to (V3) conditions, and at least one condition selected from the group consisting of (V4) to (V10), and optionally satisfying (V11) or (V12).

(V1) a volume is $1 \times 10^{-12}$ m$^3$ or larger;
(V2) remaining materials after acid dissolution are 1.0 mass% or less;
(V3) it is substantially a pure calcium phosphate as medical composition and is one selected from the group consisting of carbonate apatite, apatite containing HPO$_4$ group, tricalcium phosphate, whitlockite, calcium hydrogen phosphate;
(V4) it is a honeycomb structure comprising a plurality of through-holes extending in one direction (with the proviso that a honeycomb structure that does not satisfy any of the following condition is excluded: a composition is tricalcium phosphate, wherein a volume of pores which pore diameter is 10 $\mu$m or smaller with respect to a mass of the honeycomb structure analyzed by mercury intrusion porosimetry is 0.01 cm$^3$/g or more; and a diameter of the circle that passes through both ends of any one of the through-holes and a center of the through-hole, is 1 cm or longer, and 50 cm or shorter; The surface roughness of the surface of partition wall of honeycomb structure along the through-holes direction in arithmetic average roughness (Ra) is 0.7 $\mu$m or larger.)
(V5) it is a granule bonded-porous structure comprising a plurality of granules which maximum diameter is 50

μm or longer and 500 μm or shorter, formed by being bonded to each other, and comprising a plurality of through-holes extending in plural directions, wherein a volume of pores with a pore diameter of 10 μm or smaller analyzed by mercury intrusion porosimetry is 0.05 cm$^3$/g or more;

(V6) it is a pore integrated-type porous structure wherein a plurality of pores which maximum diameter is 50 μm or longer and 400 μm or shorter is integrated to the whole medical composition, not containing pores which maximum diameter is 800 μm or longer, wherein a volume of pores which maximum diameter is 10 μm or smaller in the pore integrated-type porous structure analyzed by mercury intrusion porosimetry is 0.05 cm$^3$/g or more (with the proviso that one which composition is tricalcium phosphate is excluded).

(V7) a volume of pores having a pore diameter of 6 μm or shorter with respect to a volume of pores having a pore diameter of 1 μm or larger and 6 μm or shorter analyzed by mercury intrusion porosimetry is 5 % or more;

(V8) a maximum compressive strength obtained at any one direction is higher than a standard compressive strength [S] that is calculated by the following equation (with the proviso that a honeycomb structure comprising a plurality of through-holes extending in one direction, wherein a volume of pores with a pore diameter of 10 μm or smaller with respect to a mass of the honeycomb structure analyzed by mercury intrusion porosimetry is 0.02 cm$^3$/g or smaller is excluded)

$$S = S_0 \times C \times exp(-b \times P)$$

(wherein So and b are the constant, and So is 500, and b is 0.068, and C is the constant based on the composition; C is 1 for carbonate apatite, apatite containing $HPO_4$, tricalcium phosphate, and C is 0.5 for whitlockite, and C is 0.1 for calcium hydrogen phosphate; and P is the percentage of pores in the composition.)

(V9) it is a honeycomb structure granule which minor diameter is 1 mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists;

(V10) The plural composition granules are connected with a fiber.

(V11) Composition is apatite with carbonate content is 10 mass% or larger.

(V12) Composition is apatite with carbonate content is smaller than 10 mass%.

[20] A medical calcium phosphate composition that satisfies the following condition (AG1) or (AG2), and optionally satisfying the following conditions (AG3) to (AG10).

(AG1) Composition is selected from the group consisting of carbonate apatite, apatite containing $HPO_4$, whitlockite, and calcium hydrogen phosphate, and granules or block with volume of $10^{-12}$ m$^3$ or larger, and contains 0.01 mass% or larger and 3 mass % or lower silver or silver compound.

(AG2) Composition is selected from the group consisting of sintered hydroxyapatite, sintered tricalcium phosphate, carbonate apatite, apatite containing $HPO_4$, whitlockite, and calcium hydrogen phosphate, and has silver phosphate crystals bonded on the surface of calcium phosphate, and content of silver phosphate is 0.01 mass% or larger and 3 mass% or smaller.

(AG3) The above described silver compound is silver phosphate.

(AG4) Silver or silver compound is contained at the surface and inside the calcium phosphate composition, and ratio of the silver content at the surface by that at least 50 μm distant to interior direction is 1.2 or higher.

(AG5) Honeycomb structure comprising a plurality of through-holes extending in one direction

(AG6) Granules bonded porous structure comprising a plurality of granules 50 μm or longer and 500 μm or shorter in diameter at the longest, bonded to each other, and comprising a plurality of through-holes extending in plural direction.

(AG7) Pores integrated type porous structure with integrated plurality of pores which maximum diameter is 50 μm or longer and 400 μm or shorter, and without containing pores which maximum diameter is 800 μm or longer.

(AG8) a ratio of pore volume which pore diameter is 1 μm or larger and 6 μm or shorter with respect to a pore volume which pore diameter analyzed by mercury intrusion porosimetry is 5 % or more;

(AG9) Maximum compressive strength obtained at one of any direction is higher than the standard compressive strength [S] that is calculated by the following equation;

$$S = S_0 \times C \times exp(-b \times P)$$

(wherein So and b are the constant, and So is 500, and b is 0.068, and C is the constant based on the composition: C is 1 for carbonate apatite or apatite containing $HPO_4$, and C is 0.5 for whitlockite, and C is 0.1 for calcium hydrogen phosphate, and C is 2 for sintered hydroxyapatite and sintered tricalcium phosphate; and P is the percentage of pores in the composition)

(AG10) it is a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists.

[21] The medical calcium phosphate composition according to [19] or [20], that satisfies at least one condition selected from (W1) to (W7):

(W1) a honeycomb structure comprising a plurality of through-holes extending in one direction, wherein ratio of pore volume with pore diameter 10 $\mu$m or smaller against mass of the honeycomb structure analyzed by mercury intrusion porosimetry is 0.01 cm$^3$/g or more;

(W2) a honeycomb structure comprising a plurality of through-holes extending in one direction, wherein a diameter of the circle that passes through both ends of any one of the through-holes and the center of the through-hole, is 1 cm or longer, and 50 cm or shorter;

(W3) a honeycomb structure with surface roughness of the surface of partition wall of honeycomb structure along the through-holes direction in arithmetic average roughness (Ra) is 0.7 $\mu$m or larger;

(W4) an aggregate of calcium phosphate with average diameter of 2 $\mu$m or larger and 8 $\mu$m or smaller;

(W5) an aggregate of calcium phosphate with sphericity of 0.9 or larger;

(W6) Mg content is $5 \times 10^{-4}$ mass% or larger, and $3 \times 10^{-3}$ mass% or smaller;

(W7) Sr content is $3 \times 10^{-3}$ mass% or larger, and $1.5 \times 10^{-2}$ mass% or smaller.

[22] A method for producing a medical calcium phosphate composition wherein: the following (AH1) and (AH2) condition are essential condition, and one selected from the group consisting of (AH3) to (AH9) can be added as a selected process:

(AH1) Using raw material calcium composition, containing 0.01 mass% or larger, and 3 mass% or smaller silver or silver composition, that is one selected from the group consisting of calcium carbonate, calcium hydroxide, calcium oxide, calcium sulfate, calcium hydrogen phosphate, and is a granule or block with volume of $10^{-12}$ m$^3$ or larger,

and comprising a process of adding carbonate group to the raw composition when the raw material calcium composition is not calcium carbonate,

and comprising a process of compositional transformation reaction to any one selected from the group consisting of silver or silver compound containing carbonate apatite,

apatite with $HPO_4$ group, whitlockite, and calcium hydrogen phosphate by exposure to aqueous phosphoric acid salt solution or mixed aqueous solution of phosphoric acid salt and magnesium salt;

(AH2) A process of immersing the raw material calcium composition selected from the group consisting of apatite, tricalcium phosphate, whitlockite, octacalcium phosphate, calcium hydrogen phosphate; and being granules or block with volume of $10^{-12}$ m$^3$ in aqueous solution containing silver ion, leading formation of silver phosphate to raw material calcium composition, is included;

(AH3) A process of immersing raw material calcium composition with calcium phosphate composition to first aqueous solution containing silver ion to form silver phosphate to raw material calcium composition, followed by immersing to second aqueous solution containing higher concentrated silver ion than first aqueous solution is included;

(AH4) Raw material calcium composition is a honeycomb structure comprising a plurality of through-holes extending in one direction;

(AH5) Raw material calcium composition is a granule bonded porous structure comprising a plurality of granules which maximum diameter is 50 $\mu$m or longer and 500 $\mu$m or shorter, bonded to each other, and comprising a plurality of through-holes extending in plural directions;

(AH6) Raw material calcium composition which is a pore integrated type porous structure with integrated plurality of pores which maximum diameter is 50 $\mu$m or longer and 400 $\mu$m or shorter, and not containing pores which maximum diameter is 800 $\mu$m or longer;

(AH7) Using a raw material calcium composition that a ratio of pore volume which pore diameter is 1 $\mu$m or larger and 6 $\mu$m or shorter with respect to a pore volume which pore diameter is 6 $\mu$m or shorter analysis using mercury intrusion porosimetry is 5 % or more;

(AH8) Using a raw material calcium composition with maximum compressive strength obtained at one of any direction is higher than the standard compressive strength [S] that is calculated by the following equation

$$S = S_0 \times C \times \exp(-b \times P)$$

(wherein So and b are the constant, and So is 500, and b is 0.068, and C is the constant based on composition. C is 1 for carbonate apatite and apatite containing $HPO_4$ group; 0.5 for whitlockite; 0.1 for calcium hydrogen phosphate; 2 for other composition; and P is the percentage of pores in the composition)

(AH9) Using a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists.

[23] A method for producing the medical calcium carbonate composition according to any one of [19] to [21] wherein: one of the following (AI1) to (AI4) condition is satisfied:

(AI1) Using calcium carbonate powder with mean particle diameter is 2 $\mu$m or larger, and 8 $\mu$m or smaller;
(AI2) Using calcium carbonate powder with sphericity 0.9 or larger;
(AI3) Using calcium carbonate powder containing $5 \times 10^{-4}$ mass% or larger, and $3 \times 10^{-3}$ mass% or smaller Mg;
(AI4) Using calcium carbonate powder containing $3 \times 10^{-3}$ mass% or larger, and $1.5 \times 10^{-2}$ mass% Sr.

[24] A method for producing the medical phosphate calcium composition according to any one of [19] to [21], comprising adding phosphoric acid component to the medical calcium carbonate composition according to any one of [1] to [4], or to the medical calcium carbonate composition produced by the method according to any one of [5] to [16], wherein the medical calcium carbonate composition is immersed in at least one of the aqueous solution selected from the group of (X1) to (X5), to add phosphoric component to the medical calcium carbonate composition:

(X1) Aqueous solution containing phosphoric acid component with pH 8.5 or higher;
(X2) Aqueous solution containing phosphoric acid component with pH lower than 8.5;
(X3) Aqueous solution containing both phosphoric acid component and carbonate component at a concentration of 0.5 mol/L or lower of pH 8.5 or higher;
(X4) Aqueous solution containing both phosphoric acid component and carbonate component at a concentration of 0.5 mol/L or lower of pH lower than pH 8.5
(X5) Aqueous solution containing both phosphoric acid component and magnesium component.

[25] A method for producing the medical calcium phosphate composition according to any one of [19] to [21], by adding phosphorous acid component to the medical calcium carbonate composition according to any one of [1] to [4], or to the medical calcium carbonate composition produced by the method according to any one of [5] to [16] wherein:

the method comprises any one of the process satisfying at least one condition selected from the group of consisting of (Y1) to (Y6) condition.
(Y1) A process of partial or complete replacement of gas in the pore of medical calcium carbonate composition immersed in the aqueous solution containing phosphoric acid component, to aqueous solution containing phosphoric acid component;
(Y2) A process of partial or complete replacement of gas in the pore of medical calcium carbonate composition immersed in the aqueous solution containing phosphoric acid component, to aqueous solution containing phosphoric acid component, using vibration;
(Y3) A process of partial or complete replacement of gas in the pore of medical calcium carbonate composition immersed in the aqueous solution containing phosphoric acid component, to aqueous solution containing phosphoric acid component, by flowing the aqueous solution;
(Y4) A process of partial or complete replacement of gas in the pore of medical calcium carbonate composition immersed in the aqueous solution containing phosphoric acid component, to aqueous solution containing phosphoric acid component, by degasification;
(Y5) A process of partial or complete replacement of gas in the pore of medical calcium carbonate composition immersed in the aqueous solution containing phosphoric acid component, to a gas that has higher solubility in an aqueous solution containing phosphoric acid component;

(Y6) A process of partial or complete replacement of gas in the pore of medical calcium carbonate composition immersed in the aqueous solution containing phosphoric acid component, to a solvent that has smaller contact angle than water and has lower boiling temperature than water.

[26] A method of producing a medical calcium phosphate composition wherein:
the following (Z1) to (Z4), or (Z1), (Z3), (Z4) or (Z1) to (Z3) are performed successively in this order and all performed in a same vessel:

(Z1) A process of producing medical calcium carbonate by adding carbonate component to raw material calcium composition;
(Z2) A process of washing medical calcium carbonate composition;
(Z3) A process of adding phosphate component to medical calcium carbonate composition;
(Z4) A process of washing medical calcium phosphate.

[27] A medical calcium hydroxide composition that satisfies all the following (AB1) to (AB3) conditions, and at least one condition selected from the group consisting of (AB4) to (AB8):

(AB1) a volume is $10^{-12}m^3$ or larger.
(AB2) remaining materials after acid dissolution are 1.0 mass% or less;
(AB3) it is substantially a pure calcium hydroxide as medical composition;
(AB4) it is a honeycomb structure comprising a plurality of through-holes extending in one direction;
(AB5) granule bonded porous structure comprising a plurality of granules which maximum diameter is 50 μm or longer and 500 μm or shorter, bonded to each other, and comprising a plurality of through-holes extending in plural directions;
(AB6) pore integrated type porous structure with integrated plurality of pores which maximum diameter is 50 μm or longer and 400 μm or shorter, and not containing pores which maximum diameter is 800 μm or longer.
(AB7) it is a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists;
(AB8) plural composition granules are connected with a fiber.

[28] A method for producing the medical calcium hydroxide composition according to [27] that satisfies the above described (AB4) condition, wherein:
a raw material calcium composition is calcium hydroxide and comprising the following process (AD1) and one selected from the group consisting of (AD2) to (AD5) are the essential processes, and (AD6) to (AD8) can be added as selection condition:

(AD1) Extrusion process
a process of producing a raw honeycomb structure comprising a plurality of through-holes extending in one direction, having a volume of $3 \times 10^{-11}m^3$ or larger by extruding a raw material calcium composition comprising polymer material through a honeycomb structure forming die;
(AD2) Debindering process
A process of debindering of polymer containing calcium hydroxide porous structure by thermal treatment so that the remaining materials after acid dissolution are 1.0 mass% or less;
(AD3) Hydration process via calcium oxide
A process of producing calcium hydroxide porous structure by debindering of polymer containing calcium hydroxide porous structure by thermal treatment so that the remaining materials after acid dissolution are 1.0 mass% or less, and to be calcium oxide porous structure, followed by hydration;
(AD4) Hydration process via calcium carbonate and calcium oxide
A hydration process via calcium carbonate and calcium oxide for producing calcium hydroxide porous structure by heat treatment of polymer containing calcium hydroxide porous structure in the presence of carbon dioxide, followed by debindering so that the remaining materials after acid dissolution are 1.0 mass% or less, and to be calcium oxide porous structure, followed by hydration;
(AD5) A production process from calcium carbonate porous structure

A process of fabricating calcium hydroxide porous structure by debindering polymer containing calcium hydroxide porous structure so that the remaining materials after acid dissolution is 1.0 mass% or less, and to be calcium oxide porous structure, followed by hydration of calcium oxide porous structure;
(AD6) Forming process after extrusion process
A process of foaming honeycomb structure consisting of polymer containing raw material calcium composition to desired foam through softening by thermal treatment, followed by pressure loading;
(AD7) Removal process of peripheral wall
A process of removing peripheral wall after the extrusion process or the forming process after extrusion process, and before debindering and carbonation process; (AD8) Forming process after removal process of peripheral wall A process of foaming honeycomb structure consisting of polymer containing raw material calcium composition to desired foam through softening by thermal treatment, after removal process of peripheral wall.

[29] A method for producing the medical calcium hydroxide composition according to [27] that satisfies the above described (AB5) condition, comprising the following process (AE1), (AE2) and at least one selected from the group consisting of the following (AD2) to (AD5) as essential processes:

(AE1) Placement process
A process of placing polymer containing calcium hydroxide granules with a volume of $10^{-12} m^3$ or more in a reaction vessel;
(AE2) Granules bonding process
A process of producing granules bonded-porous structure comprising a plurality of through-holes extending in plural directions, formed by bonding a plurality of granules which maximum diameter is 50 $\mu$m or longer and 500 $\mu$m or shorter, and having a volume of $3 \times 10^{-11}$ $m^3$ or larger,
based on heat treatment of the granules in the reaction vessel so that the surface is softened and fused one another, or dissolution of the granule surface so that the surface is bonded to each other, or treatment with a plasticizer so that the granule surface is fused one another;
(AD2) Debindering process
A process of debindering of polymer containing calcium hydroxide porous structure by thermal treatment so that the remaining materials after acid dissolution are 1.0 mass% or less
(AD3) Hydration process via calcium oxide
A process of producing calcium hydroxide porous structure by debindering of polymer containing calcium hydroxide porous structure by thermal treatment so that the remaining materials after acid dissolution is 1.0 mass% or less, and to be calcium oxide porous structure, followed by hydration.
(AD4) Hydration process via calcium carbonate and calcium oxide
A hydration process via calcium carbonate and calcium oxide for producing calcium hydroxide porous structure by heat treatment of polymer containing calcium hydroxide porous structure in the presence of carbon dioxide, followed by debindering so that the remaining materials after acid dissolution is 1.0 mass% or less, and to be calcium oxide porous structure, followed by hydration.
(AD5) A production process from calcium carbonate porous structure
A process of fabricating calcium hydroxide porous structure by debindering polymer containing calcium hydroxide porous structure so that the remaining materials after acid dissolution is 1.0 mass% or less, and to be calcium oxide porous structure, followed by hydration of calcium oxide porous structure.

[30] A method for producing the medical calcium hydroxide composition according to [27] using calcium hydroxide porous structure or calcium carbonate porous structure, wherein:
calcium oxide porous structure is produced using calcium hydroxide porous structure or calcium carbonate porous structure, followed by producing calcium hydroxide porous structure by its hydration.
[31] A method for producing the medical calcium composition according to any one of [9] to [11], [14] and [29] that satisfies at least one condition from the following (AF1) to (AF3) in the placement-closing process or placement process:

(AF1) Sphericity of the granules is 0.9 or larger;
(AF2) The granules are hollow;
(AF3) granules with a bulk volume of 105% or more with respect to the reaction vessel are placed in the reaction vessel.

[32] A bone defect reconstruction kit comprising a solid portion that contains vaterite and $\alpha$-tricalcium phosphate and a liquid portion that contains phosphoric acid salt, and set to form carbonate apatite when the solid portion and liquid portion are mixed.

[33] The bone defect reconstruction kit according to [32] wherein: amount of vaterite in the solid portion is 10 mass% or larger and 60 mass% or smaller.

[34] The bone defect reconstruction kit according to [32] or [33], wherein the liquid portion contains at least one selected from, acid containing plural carboxy groups, hydrogen sulfite salt, cellulose derivative, dextran sulfate salt, chondroitin sulfate salt, alginic acid salt, glucomannan.

[35] The bone defect reconstruction kit according to any one of [32] to [34], wherein the volume of vaterite in the solid portion is $10^{-12}$ m$^3$ or larger.

[36] The bone defect reconstruction kit according to any one of [32] to [34], wherein the vaterite's average diameter is 6 $\mu$m or smaller.

Brief Description of Drawings

Figure 1 is a schematic view of a honeycomb structure having a peripheral wall.
Figure 2 shows mercury intrusion porosimetry results of a medical calcite composition produced at a final temperature of 480°C in Example 7.
Figure 3 shows a powder X ray diffraction (XRD) pattern of a medical vaterite composition according to Example 1.
Figure 4 shows an XRD pattern of a medical carbonate apatite composition according to Example 1.
Figure 5 shows an electron microscope image (SEM image) of a honeycomb structure according to Example 7.
Figure 6 shows particle size distribution analysis results according to Example 8.
Figure 7 shows a histopathological image obtained in histopathological examination using a medical carbonate apatite honeycomb structure according to Example 9.
Figure 8 shows an electron microscope image (SEM image) of a medical carbonate apatite honeycomb structure according to Example 12.
Figure 9 shows a histopathological image obtained in histopathological examination on week 4 after implantation of a medical carbonate apatite honeycomb structure according to Example 14.
Figure 10 shows a histopathological image obtained in histopathological examination on week 12 after implantation of the medical carbonate apatite honeycomb structure according to Example 14.
Figure 11 shows histopathological images obtained in histopathological examination on weeks 4 and 12 after implantation of a medical carbonate apatite composition of Example 15 and a hydroxyapatite composition of Comparative Example 10.
Figure 12 shows an electron microscope image (SEM image) of a medical vaterite porous structure according to Example 16.
Figure 13 shows a histopathological image obtained in histopathological examination on week 4 after implantation of a medical carbonate apatite porous structure according to Example 16.
Figure 14 shows an electron microscope image (SEM image) of a medical calcite porous structure according to Example 17.
Figure 15 shows an electron microscope image (SEM image) of a medical carbonate apatite honeycomb structure according to Example 22.
Figure 16 shows a histopathological image obtained in histopathological examination on week 4 after implantation of a medical carbonate apatite honeycomb structure according to Example 22.
Figure 17 shows an electron microscope image (SEM image) of a medical carbonate apatite honeycomb structure according to Example 24.

Description of Embodiments

(Definition of term)

Terms used in the present invention are defined as described below.

The "medical calcium carbonate composition" described in the present invention is a calcium carbonate composition that is used as a medical composition (medical material) or a calcium carbonate composition that is used as a raw material for medical compositions. Bone graft materials or drug delivery carriers to be implanted in living tissues are medical materials as a matter of course, while scaffolds for cell cultures for use outside living tissues are also included in the medical materials. When the composition is a porous structure, it is also referred to as a medical calcium carbonate porous structure. When the porous structure is a honeycomb structure, it is also referred to as a medical calcium carbonate honeycomb structure, etc.

The "medical calcium carbonate composition" described in the present invention may be used in the production of other medical compositions and in this relation, may contain porogen such as sodium chloride. In order to improve the handleability of medical calcium carbonate or other medical compositions, composition particles (particles) may be bonded to each other via a fiber. The material containing such porogen or a fiber is also defined as the "medical calcium carbonate composition".

The "volume" described in the present invention is bulk volume and is also called total volume. It is a volume including pores.

The "vaterite", the "aragonite", and the "calcite" described in the present invention are polymorph types of calcium carbonate crystals.

The "medical vaterite composition" described in the present invention is a medical calcium carbonate composition containing 20 mass% or larger of vaterite. In this context, the masses of porogen and a fiber are excluded from the calculation of the vaterite content.

The contents of vaterite and calcite in medical calcium carbonate compositions are calculated from their respective peak area ratios in powder X ray diffraction (XRD) analysis by a method mentioned later.

The "medical calcium phosphate composition" described in the present invention is a calcium phosphate composition that is medically used as an artificial bone graft material or the like.

The "calcium phosphate" described in the present invention is a compound comprising phosphoric acid and calcium. Examples thereof include calcium condensed phosphate compounds typified by calcium orthophosphate, calcium metaphosphate, amorphous calcium phosphate, and calcium pyrophosphate. The calcium orthophosphate is a salt of orthophosphoric acid and calcium. Examples thereof include tetracalcium phosphate, apatite including hydroxyapatite and carbonate apatite, $\alpha$-tricalcium phosphate, $\beta$-tricalcium phosphate, and calcium hydrogen phosphate. Although the $\beta$-tricalcium phosphate may include whitlockite, a form free from $HPO_4$ and a form containing $HPO_4$ are categorized as tricalcium phosphate and whitlockite, respectively, in the present invention. The $\alpha$-tricalcium phosphate is also abbreviated to $\alpha TCP$, and the $\beta$-tricalcium phosphate is also abbreviated to $\beta TCP$.

The "medical apatite composition" described in the present invention is a kind of medical calcium phosphate composition and is an apatite composition that is used as a medical material such as an artificial graft material.

The "medical carbonate apatite composition" described in the present invention is a carbonate apatite composition that is used for medical purposes. The carbonate content of carbonate apatite is not particularly limited and is preferably 0.5 mass% or larger, more preferably 3 mass% or larger, further preferably 6 mass% or larger. The carbonate apatite described in the present invention is defined as apatite that contains carbonate groups. In general, it is apatite in which the phosphoric acid groups or hydroxy groups of calcium phosphate-based apatite are partially or completely replaced with carbonate groups. In association with replacement with carbonate groups, Na, K, or the like is often contained in crystal structures in order to control the charge balance of apatite. In the present invention, carbonate apatite obtained by partially substituting carbonate apatite by other elements or voids is also defined as carbonate apatite.

Increased carbonate content facilitates resorption by osteoclasts and accelerates bone replacement rate. Depending on cases, medical materials that are slowly replaced with bones or medical materials that are not replaced with bones are desired. When a medical material that is slowly replaced with bones is desired, carbonate apatite having a small amount of carbonate groups is desired. When a medical material that is not replaced with bones is desired, apatite having less than 0.2 mass% of carbonate groups is desired.

The "honeycomb structure" described in the present invention is a porous structure shaped to have a plurality of through-holes having a polygonal or round cross-sectional shape and extending in one direction, as described in Japanese Patent Laid-Open Publication No. 2004-298407 or 2005-152006. The through-holes are arranged with substantially no space via partition walls, whereas some through-holes may be lost.

The term "one direction" described in the present invention is not limited to a straight line direction and means substantially the same direction. The through-holes of a bended honeycomb structure mentioned later are in a form having a plurality of through-holes extending in substantially the same direction, albeit not one-dimensionally, and this is also a honeycomb structure.

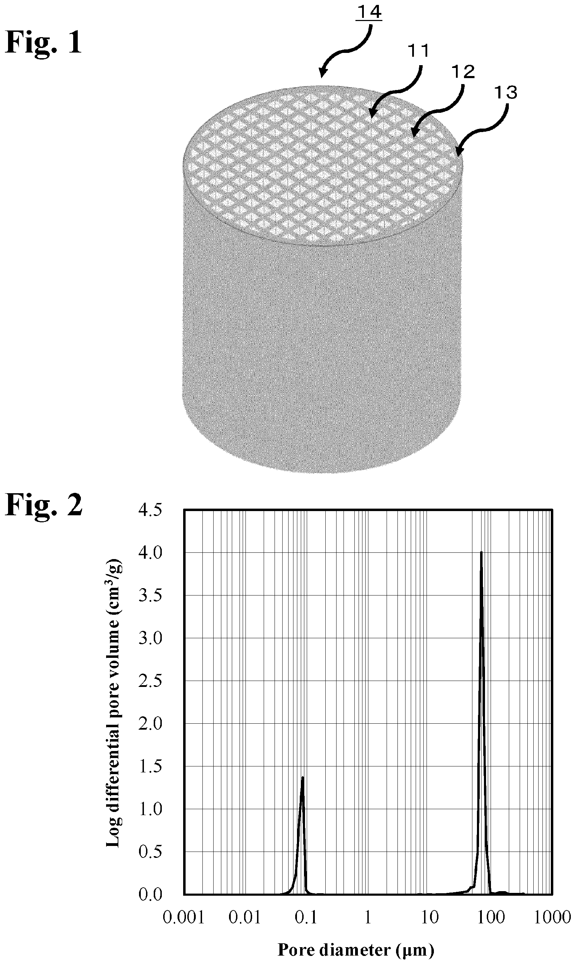

Now referring to Figure 1, one example of the honeycomb structure of the present invention will be described. As shown in Figure 1, honeycomb structure 14 is a structure having a plurality of through-holes 11 extending in one direction, and partition walls 12 which partition the through-holes. The honeycomb structure may have peripheral wall 13 which surrounds a honeycomb structure portion consisting of the through-holes or may be free from the peripheral wall partially or completely. Both the forms are included in the honeycomb structure.

The mercury intrusion porosimetry described in the present invention is a kind of porosimetry and is a method that exploits large surface tension of mercury and involves applying pressure to a powder material so that mercury infiltrates into its pores, and determining a pore distribution from the pressure and the amount of mercury intruded. In the present invention, the pores are calculated using 130° as the advancing contact angle and receding contact angle between mercury and the material and 485 mN/m as the surface tension of mercury.

In order to visualize a pore distribution, common logarithm of differential pore volume is plotted against pore diameters at measurement points. The pore volume is calculated from integrated data on intruded mercury among different pore diameters.

The pore diameter is basically a value that is calculated from mercury intrusion porosimetry results on the assumption that mercury is intruded to cylindrical pores, regardless of pore shape.

The "mean particle diameter (or average (particle) diameter)" described in the present invention means a particle diameter at an integrated value of 50% in a particle size distribution determined by a laser diffraction-scattering method. A powder is dispersed in 100 mL of distilled water and dispersed for 30 seconds with an ultrasonic washing machine with a frequency of 45 kHz-100 W, and measurement is performed within 1 minute thereafter.

The "surface roughness (arithmetic average roughness Ra)" described in the present invention is surface roughness (arithmetic average roughness Ra) measured under a 3D laser microscope.

The "sphericity" described in the present invention is Wadell's working sphericity. The Wadell's working sphericity is obtained by dividing the diameter of a circle equal to the projected area of a material by the diameter of the smallest circle circumscribed to the projected image of the material.

The "compressive strength" described in the present invention is a value obtained by dividing the maximum load by the original cross-sectional area of a columnar test piece subjected to a compression test at a crosshead speed of 10 mm/min. In the present invention, when a sample is not in a columnar shape and has a volume of $1 \times 10^{-8}$ $m^3$ or larger, the sample is processed into a columnar shape and subjected to a compression test. When a sample is not in a columnar shape and has a volume of smaller than $1 \times 10^{-8}$ $m^3$, the projected image area of the sample is regarded as the original cross-sectional area of a test piece.

The medical calcium carbonate composition of the present invention may be anisotropic. Anisotropic materials differ in compressive strength depending on directions. In the present invention, the largest compressive strength obtained in any direction is defined as the compressive strength of the composition.

In light of the fact that medical calcium carbonate compositions, etc. are ceramic materials, diametral tensile strength is measured, and a value obtained by increasing the diametral tensile strength 5-fold may be regarded as the compressive strength. If the compressive strength is different from the value obtained by increasing the diametral tensile strength 5-fold, a higher value is regarded as the value of the compressive strength according to the present invention.

The pressure of atmosphere used in the present invention is absolute pressure based on a pressure of 101.3 KPa around the ocean surface and is not relative pressure with reference to atmospheric pressure. Thus, atmosphere exceeding 101.3 KPa is in a pressurized state with respect to atmospheric pressure, and atmosphere less than 101.3 KPa is in a reduced pressure state with respect to atmospheric pressure.

The term "integrated" or "integration" described in the present invention is defined as a plurality of objects gathered. The phrase "pores are integrated" means that a plurality of pores is gathered, and the pores may or may not be bonded to each other. Thus, the pores do not have to be contacted with each other.

The "minor diameter" described in the present invention is a term related to the morphology of a composition and is defined as a size related to the presence or absence of passing through a sieve. Specifically, a minor diameter of 1 mm or larger and shorter than 5 mm is defined as the size of a composition that passes through a sieve having an opening of 5 mm and does not pass through a sieve having an opening of 1 mm.

The "closed reaction vessel" described in the present invention is defined as a reaction vessel that is not an open system. For example, when a calcium hydroxide compact in a reaction vessel is carbonated with carbon dioxide to produce calcium carbonate, the calcium hydroxide compact needs to be exposed to carbon dioxide. The case where carbon dioxide is discharged from a reaction vessel into the atmosphere is carbonation using an open reaction vessel, and the case where carbon dioxide is not discharged from a reaction vessel into the atmosphere is carbonation using a closed reaction vessel.

From this viewpoint, the case where carbon dioxide is discharged from an outlet or the like but is not discharged into the atmosphere is defined as carbonation using a closed reaction vessel. For example, the case where carbon dioxide discharged from an outlet is circulated to a reaction vessel via a pump or the like is defined as carbonation using a closed reaction vessel. Also, the case where a reaction vessel is connected to a carbon dioxide tank or the like is defined as carbonation using a closed reaction vessel.

In the present invention, hydrous methanol, hydrous ethanol, etc. is simply indicated in vol% of the solvent other than water. For example, methanol containing 10 vol% of water is referred to as 90% methanol.

The "granules" are generally defined as particles having a larger particle diameter than that of powders, particularly, large particles shaped by settling powders. The "granules" described in the present invention are particles having a larger particle diameter than that of powders and correspond to particles having a minor diameter of 50 $\mu$m or larger and 500 $\mu$m or shorter, or a volume of $1 \times 10^{-13}$ $cm^3$ or larger and $1 \times 10^{-7}$ $cm^3$ or smaller, unless the volume or the minor diameter is otherwise specified.

[I Medical calcium carbonate composition: essential condition]

First, [1] will be described.

<(A) A volume is $10^{-12}$ m$^3$ or larger>

An essential condition of the medical calcium carbonate composition of the present invention is that a volume is $10^{-12}$ m$^3$ or larger. Although medical compositions are required to be excellent in tissue affinity *in vivo,* both calcium carbonate powders and calcium phosphate powders evoke inflammatory reaction. On the other hand, a medical calcium carbonate composition having a volume of $10^{-12}$ m$^3$ or larger, or a medical calcium phosphate composition having a volume of $10^{-12}$ m$^3$ or lager produced from the medical calcium carbonate composition is excellent in tissue affinity.

In the case of using the medical calcium carbonate composition as a bone graft material or as a raw material for bone graft materials, A volume of $10^{-11}$ m$^3$ or larger, preferably $3 \times 10^{-11}$ m$^3$ or larger, is more preferred because the resulting composition when filled into a bone defect easily forms interconnected pores suitable for the invasion of cells. A volume of $10^{-10}$ m$^3$ or larger is further preferred because the resulting composition when filled into a bone defect forms pores effective for the invasion of tissues. A volume of $10^{-9}$ m$^3$ or larger is particularly preferred for clinical practice because of a feature that the resulting composition is easy to fill into a relatively large bone defect.

The upper limit of the volume of the medical calcium carbonate composition is not particularly limited and is preferably $10^{-3}$ m$^3$ or smaller because large volumes require time for production and are less demanded.

<(B) Remaining materials after acid dissolution are 1 mass% or less>

(B) is also related to a condition described below that it is substantially a pure calcium carbonate as a medical composition. A particularly essential condition is that remaining materials after acid dissolution are 1 mass% or less. This factor is very important for the usefulness of the medical calcium carbonate composition.

This is because the medical calcium carbonate composition of the present invention or carbonate apatite, etc. produced using the medical calcium carbonate composition as a raw material is expected to have excellent tissue affinity and *in vivo* resorbability. Calcium carbonate, carbonate apatite, or the like is resorbed by weakly acidic environment formed by osteoclasts, etc. *in vivo.* The presence of remaining materials after acid dissolution is synonymous with no resorption by osteoclasts, etc. and is therefore inappropriate for medical compositions.

The remaining materials after acid dissolution are problematic if the medical calcium carbonate composition is produced from a raw material containing polymer material. The polymer material is not soluble in acid, and the presence of remaining materials after acid dissolution is synonymous with remaining polymer material or decomposition products thereof.

The remaining materials after acid dissolution are remaining materials after calcium carbonate, etc. is dissolved in 1 mol/L hydrochloric acid having a volume of 20 molar equivalents with respect to the calcium carbonate, etc., and are indicated in % of dry mass with respect to the mass of the calcium carbonate, etc.

The remaining materials after acid dissolution are essentially 1 mass% or less, preferably 0.5 mass% or smaller, more preferably 0.3 mass% or smaller which decreases influence, further preferably 0.1 mass% or smaller which renders influence almost ignorable, ideally substantially 0 mass%.

The present medical calcium carbonate composition may contain porogen, or plural composition particles may be connected with a fiber. The porogen and the fiber that connects plural composition particles are not subject to the remaining materials after acid dissolution. Specifically, an essential condition of the present invention is that the remaining materials after acid dissolution of the calcium carbonate composition except for the porogen and the fiber that connects plural composition particles are 1 mass% or less.

<(C) It is substantially a pure calcium carbonate as a medical composition, and mainly comprises vaterite or calcite>

The medical calcium carbonate composition of the present invention is a medical material that is used for medical purposes, and cannot be used as a medical material if containing impurities other than the porogen and the fiber that connects plural composition particles. Thus, natural materials are not included in the present invention. Calcium carbonate compositions produced from calcium oxide-containing limestone discharged from boilers or from slag generated in iron and steel production processes also contain impurities and are therefore materials that are not included in the present invention. Organic gel, silicon-containing gel, metaphosphoric acid gel, or the like may be used in research on natural minerals, etc., and calcium carbonate compositions supplemented with these present problems associated with tissue affinity and are therefore materials that are not included in the present invention.

An essential condition of the medical calcium carbonate composition of the present invention is that it is substantially a pure calcium carbonate as a medical calcium carbonate composition, and mainly comprises vaterite or calcite, i.e., it is substantially a pure calcium carbonate, and its polymorph is mainly vaterite or calcite. It is required that impurities other than sodium, strontium and magnesium are substantially absent. In this context, in (C) of the present invention, the amount of impurities other than sodium, strontium and magnesium is preferably 1 mass% or smaller, more preferably 0.5 mass% or smaller, further preferably 0.1 mass% or smaller. Ideally, it contains no impurities.

In the medical calcium carbonate composition, sodium, strontium and magnesium, unlike other impurities, are less likely to evoke tissue irritancy. Although this mechanism is unknown, calcium carbonate chosen by invertebrates that emerged in the sea water containing sodium, strontium and magnesium contained sodium, strontium and magnesium and therefore evolutionistically became organisms that could tolerate sodium, strontium and magnesium according to analogy. However, magnesium, strontium and sodium are also impurities, and their content in "substantially a pure calcium carbonate as a medical composition" of the present invention is preferably 2 mass% or smaller, more preferably 1.0 mass% or smaller, further preferably 0.2 mass% or smaller.

As mentioned above, the medical calcium carbonate composition may contain porogen and a fiber that connects plural composition particles, and the porogen and the fiber that connects plural composition particles are not regarded as impurities. Specifically, an essential condition of the present invention is that the calcium carbonate composition except for the porogen and the fiber that connects plural composition particles is substantially a pure calcium carbonate.

As mentioned above, the polymorph of calcium carbonate includes vaterite and calcite as well as aragonite. Aragonite is essentially calcium carbonate and therefore, if coexisting, does not inhibit the advantageous effects of the present invention. The content of aragonite is preferably 20 mass% or smaller, more preferably 10 mass% or smaller, further preferably 5 mass% or smaller, from the viewpoint of the reactivity of a medical composition produced using the medical calcium carbonate composition of the present invention as a raw material. Specifically, the phrase "mainly comprises vaterite or calcite" of the present invention means that the proportion of vaterite or calcite is preferably more than 80 mass%, more preferably more than 90 mass%, further preferably more than 95 mass%, particularly preferably 100 mass%.

Calcium hydroxide and calcium oxide are converted into calcium carbonate through chronological reaction with carbon dioxide in the air and therefore do not have to be strictly eradicated. Hence, in "substantially a pure calcium carbonate as a medical calcium carbonate composition" described in the present invention, calcium hydroxide and calcium oxide are not regarded as impurities. However, it is preferred that neither calcium hydroxide nor calcium oxide should be contained. Thus, the content of calcium hydroxide and calcium oxide is also preferably 3 mass% or smaller, more preferably 2 mass% or smaller, further preferably 1 mass% or smaller. Ideally, it contains no calcium hydroxide or calcium oxide.

<(D) It contains 20 mass% or larger of vaterite>

Calcium carbonate compositions containing vaterite, which is metastable phase calcium carbonate, are preferred because of their high reactivity. Although a vaterite content of less than 20 mass% is also effective for improvement in reactivity, its effects are limited. Therefore, the present invention is limited by the content of 20 mass% or larger.

The content of vaterite is essentially 20 mass% or larger, preferably 30 mass% or larger because of a nearly sufficient amount of the active ingredient, more preferably 50 mass% or larger because of a sufficient amount of the active ingredient, further preferably 80 mass% or larger because the composition almost exhibits the properties of vaterite, particularly preferably 90 mass% or larger.

As mentioned above, the masses of the porogen and the fiber that connects plural composition particles are excluded from the calculation of the vaterite content because they are not involved in reactivity.

<(E) It is a honeycomb structure comprising a plurality of through-holes extending in one direction, wherein a volume of pores which pore diameter is 10 $\mu$m or smaller with respect to a mass of the honeycomb structure analyzed by mercury intrusion porosimetry is larger than 0.02 cm$^3$/g>

A honeycomb structure is formed, as shown in Figure 1, from a partition wall portion, a solid portion consisting of a peripheral wall, and a void space portion which is through-holes. Therefore, in the case of performing mercury intrusion porosimetry, a pore distribution attributed to the through-holes is always found. Figure 2 shows one example of mercury intrusion porosimetry results of the medical calcium carbonate honeycomb structure of the present invention. As is evident therefrom, there exist pores having a peak at which the pore diameter attributed to the macropores of the honeycomb structure is approximately 70 $\mu$m as well as a pore diameter of 1 $\mu$m or smaller attributed to the micropores of the honeycomb partition walls.

No medical calcium carbonate honeycomb structure has 10 $\mu$m or smaller through-holes. Thus, pores which pore diameter is 10 $\mu$m or smaller are pores present in partition walls. Since neither tissues nor cells infiltrate into the pores which pore diameter is 10 $\mu$m or smaller, these pores have not received attention so far. However, tissue fluids or aqueous solution is capable of infiltrating into the pores. Hence, the pores which pore diameter is 10 $\mu$m or smaller

have been found to play an important role in *in vivo* reaction or in producing a medical calcium phosphate honeycomb structure by adding phosphoric acid salt to the medical calcium carbonate honeycomb structure.

A larger pore diameter with respect to a mass of the honeycomb structure enhances reactivity and however, reduces mechanical strength. It is therefore necessary to control the balance therebetween. The volume of pores which pore diameter is 10 $\mu$m or smaller with respect to a mass of the honeycomb structure is essentially larger than 0.02 cm$^3$/g, preferably 0.03 cm$^3$/g or more, more preferably 0.10 cm$^3$/g or more, further preferably 0.15 cm$^3$/g or more, from the viewpoint of reactivity. It is preferably 0.15 cm$^3$/g or smaller, more preferably 0.10 cm$^3$/g or smaller, further preferably 0.03 cm$^3$/g or smaller, from the viewpoint of compressive strength.

<(F) It is a granule bonded-porous structure comprising a plurality of granules which maximum diameter is 50 $\mu$m or longer and 500 $\mu$m or shorter, formed by being bonded to each other, and comprising a plurality of through-holes extending in plural directions, wherein a volume of pores with a pore diameter of 10 $\mu$m or smaller analyzed by mercury intrusion porosimetry is 0.05 cm$^3$/g or more>

Depending on cases, porous structures having three-dimensional through-holes may be desired. Particularly, pore size is important for medical bone graft materials. Since tissues other than bone tissues, such as adipose tissues, invade large pores, the control of pore size is important.

A granule bonded-porous structure comprising a plurality of granules which maximum diameter is 50 $\mu$m or longer and 500 $\mu$m or shorter, formed by being bonded to each other, and comprising a plurality of through-holes extending in plural directions is characterized by high penetrability, as in through-holes formed by hexagonal closest packed structures, and easy migration and conduction of osteoblasts, osteoclasts, or bone tissues to the inside.

Pores in the granule portion play an important role in the tissue replacement of the granule bonded-porous structure. The pore volume of the granule portion needs to be 0.05 cm$^3$/g or more in terms of the volume of 10 $\mu$m or smaller pores in the granule bonded-porous structure analyzed by mercury intrusion porosimetry. The pore volume is preferably 0.1 cm$^3$/g or more, more preferably 0.2 cm$^3$/g or more, further preferably 0.3 cm$^3$/g or more.

<(G) It is a pore integrated-type porous structure wherein a plurality of pores which maximum diameter is 50 $\mu$m or longer and 400 $\mu$m or shorter is integrated to the whole medical composition, not containing pores which maximum diameter is 800 $\mu$m or longer, wherein a volume of pores which maximum diameter is 10 $\mu$m or smaller in the pore integrated-type porous structure analyzed by mercury intrusion porosimetry is 0.05 cm$^3$/g or more>

The above described granule bonded-porous structure is very useful for bone graft materials or the like from the viewpoint of penetrability, etc. and however, generally has disadvantages of small mechanical strength and difficult production. On the other hand, for example, a pore integrated-type porous structure having specific pores formed using porogen can be produced relatively easily by using polymer porogen, and is characterized by relatively excellent mechanical strength, albeit inferior in penetrability, and as such, is useful as medical calcium carbonate. Specifically, in the pore integrated-type porous structure, pores are integrated via pore walls made of calcium carbonate or via through-penetrations formed in a portion of the pore walls. When the through-penetrations penetrate the whole, the resulting porous structure having three-dimensional through-holes is particularly useful.

The pore size needs to be 50 $\mu$m or larger and 400 $\mu$m or smaller. This pore size is useful size from the viewpoint of the invasion of cells or tissues. The pore size is preferably 70 $\mu$m or larger and 350 $\mu$m or smaller, more preferably 90 $\mu$m or larger and 300 $\mu$m or smaller, further preferably 100 $\mu$m or larger and 300 $\mu$m or smaller.

Meanwhile, not containing pores which maximum diameter is 800 $\mu$m or longer is also a necessary condition. As mentioned above, adipose tissues, not bone tissues, migrate into large pores. Furthermore, compositions having large pores have small mechanical strength.

As not containing pores which maximum diameter is 800 $\mu$m or longer is also a necessary condition, the pore size to be excluded is preferably a maximum diameter of 700 $\mu$m or longer, more preferably a maximum diameter of 600 $\mu$m or longer.

The porosity is preferably 40 vol% or more and 80 vol% or less, more preferably 45 vol% or more and 78 vol% or less, further preferably 50 vol% or more and 77 vol% or less, from the viewpoint of the invasion of cells or tissues and the balance between resorbability and mechanical strength.

Not only the pores but also pores of calcium carbonate around the pores play an important role in the tissue replacement of the pore integrated-type porous structure. The volume of the pores needs to be 0.05 cm$^3$/g or more in terms of a volume of 10 $\mu$m or smaller pores in the pore integrated-type porous structure analyzed by mercury intrusion porosimetry. The volume of the pores is preferably 0.1 cm$^3$/g or more, more preferably 0.2 cm$^3$/g or more, further preferably 0.3 cm$^3$/g or more.

The pore integrated-type porous structure is classified into a porous structure containing porogen and a porous structure containing no porogen. The porous structure containing porogen is used directly as a raw material for medical composition production, and the porogen moiety is essentially pores because it is removed in the production process. Hence, the porogen is calculated as pores in the calculation of the porosity of the porous structure.

<(H) A ratio of pore volume which pore diameter is 1 $\mu$m or larger and 6 $\mu$m or shorter with respect to a pore volume which pore diameter is 6 $\mu$m or shorter analyzed by mercury intrusion porosimetry is 10% or more>

As mentioned above, not only macropores but also micropores are important for the reactivity of calcium carbonate porous structures. Although the absolute amount of micropores is important, the distribution of specific micropores may be useful. Specifically, a ratio of pore volume which pore diameter is 1 $\mu$m or larger and 6 $\mu$m or shorter with respect to a pore volume which pore diameter is 6 $\mu$m or shorter analyzed by mercury intrusion porosimetry is preferably 10% or more. The ratio of pore volume which pore diameter is 1 $\mu$m or larger and 6 $\mu$m or shorter with respect to a pore volume which pore diameter is 6 $\mu$m or shorter is more preferably 15% or more, further preferably 20% or more.

<(I) A maximum compressive strength obtained at any one direction is higher than a standard compressive strength [S] that is calculated by the following equation (with the proviso that a honeycomb structure comprising a plurality of through-holes extending in one direction, wherein a volume of pores with a pore diameter of 10 $\mu$m or smaller with respect to a mass of the honeycomb structure analyzed by mercury intrusion porosimetry is 0.02 cm$^3$/g or smaller is excluded)

$$S = S_0 \times C \times exp(-b \times P)$$

(wherein So and b are constant number, So is 500, b is 0.068, and C is a constant number based on polymorph of calcium carbonate; C is 0.01 when the calcium carbonate contains 20 mass% or larger of vaterite, and is 1 when the calcium carbonate does not contain 20 mass% or larger of vaterite; and P is a percentage of pores in the composition.)>

As mentioned above, the medical calcium carbonate composition of the present invention is preferably a porous structure, and its higher porosity enhances usefulness. On the other hand, the higher porosity reduces the mechanical strength of medical calcium carbonate.

It is known that the empirical equation (Duckworth equation) S = Soexp(-bP) cited in journal papers such as Journal of Biomedical Research, Vol. 29, pp. 1537-1543 and Journal of American Ceramics Society, Vol. 36, No. 2, pp. 68 holds between mechanical strength [S] and percentage [P] of pores. In this context, So is mechanical strength of a precise body, and b is empirical constant.

As mentioned above, reactivity is also influenced by composition. Therefore, even compositions having small porosity have high reactivity depending on their composition. A requirement for compressive strength is corrected with this compositional factor represented by C. C is 0.01 when the calcium carbonate contains 20 mass% or larger of vaterite, and is 1 when the calcium carbonate does not contain 20 mass% or larger of vaterite. P is a percentage of pores in the composition.

In the present invention, values reported as coefficients of similar ceramic hydroxyapatite in Journal of Biomedical Research, Vol. 29, pp. 1537-1543 are used as references.

Specifically, 500 is used as $S_0$, and 0.068 is used as b. Since higher compressive strength is more preferred, So is preferably 700, more preferably 900, further preferably 1000.

In the case of a honeycomb structure comprising a plurality of through-holes extending in one direction, a volume of pores with a pore diameter of 10 $\mu$m or smaller with respect to a mass of the honeycomb structure analyzed by mercury intrusion porosimetry is 0.02 cm$^3$/g or smaller is excluded from the present invention because its reactivity is low.

<(J) It is a honeycomb structure granule which minor diameter is 1 mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists>

This relates to the profile of honeycomb structure granules and is a condition as to compositions having round corners which do not damage the surrounding tissue. Compositions which minor diameter is shorter than 5 mm may compensate as granules for bone defects. A honeycomb structure as the medical calcium carbonate composition of the present invention is highly anisotropic. Hence, the honeycomb structure is pulverized into a spindle shape. When the minor diameter is shorter than 1 mm, the resulting granules have a lot of fluidity and are therefore less likely to have a sharp angle portion vertical to the surrounding tissue, such as the periosteum, which covers a defect. A larger minor diameter renders the surrounding tissue more susceptible to damage, for example, because the resulting granules have limited fluidity. A minor diameter of 5 mm or larger facilitates preventing the formation of sharp corners. Hence, the corners of a honeycomb structure granule which minor diameter is 1 mm or larger, and shorter than 5 mm can be rounded. Specifically, such a granule can be a granule which minor diameter is 1 mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists. The minor diameter is defined by whether to pass through a sieve. Specifically, compositions which minor diameter is 1 mm or larger, and shorter than 5 mm are compositions that pass through a sieve having an opening of 5 mm and do not pass through a sieve having an opening of 1 mm. The minor diameter is preferably 1 mm or larger and shorter than 5 mm, more preferably 1.2 mm or larger and shorter than 4 mm, further preferably 1.4 mm or larger and shorter than 3 mm.

It is preferred that "at a triangle formed by three points, no vertex point that the interior angle is 60° or smaller at the triangle should exist" as described above, from the viewpoint of round corners. The interior angle is more preferably 80° or smaller, further preferably 100° or smaller.

In the honeycomb structure granule as well, not only the profile of the granule that does not damage the surrounding tissue but also pores of the granule play an important role in tissue reaction. The volume of the pores is preferably 0.02 $cm^3$/g or more, more preferably 0.05 $cm^3$/g or more, further preferably 0.1 $cm^3$/g or more, in terms of a volume of 10 $\mu$m or smaller pores in the honeycomb structure granule analyzed by mercury intrusion porosimetry.

<(K) Plural compositions are connected with a fiber>

Medical calcium carbonate compositions or medical calcium phosphate compositions, etc. produced using the medical calcium carbonate compositions as raw materials may be implanted *in vivo* as bone graft materials or the like. When such compositions are particles such as granules, an operation of implanting the particles in bone defects is complicated. A composition wherein plural composition particles are connected in a beaded manner with a fiber has a lot of handleability. It is more preferred that the fiber should pass through the inside of the composition particles so as to connect plural composition particles, from the viewpoint of tissue affinity and strong connection.

The particle size is not particularly limited as long as the size enables the particles to be connected with a fiber. For example, the above-described granule which minor diameter is 1 mm or larger, and shorter than 5 mm is preferred.

The fiber is not limited by its type, and needs to be carbon fiber for medical compositions to be produced by production methods using a sintering operation, because of tissue affinity and no incineration. In the case of not performing a sintering operation, a bioresorbable fiber is preferred. Examples thereof include polyglycolic acid, polylactic acid, polycaprolactone, and copolymers thereof.

In this composition as well, pores play an important role in tissues reaction. The volume of the pores is preferably larger than 0.02 $cm^3$/g, more preferably 0.05 $cm^3$/g or more, further preferably 0.1 $cm^3$/g or more, in terms of a volume of 10 $\mu$m or smaller pores in the composition analyzed by mercury intrusion porosimetry.

In the present invention, an essential condition is that at least one selected from the group consisting of (D) to (K) is satisfied. It is preferred that two or more selected from these conditions should be satisfied.

(Shape of medical calcium carbonate composition)

The shape of the medical calcium carbonate composition of the present invention is not particularly limited. It can be an arbitrary shape such as a precise body, a porous structure, a block body, granules, or a plate, and a specific porous structure mentioned later is particularly preferred.

[I Medical calcium carbonate composition: sintered vaterite]

Next, [2] will be described.

Vaterite is one polymorph of calcium carbonate having high reactivity and is a metastable phase and a stable phase at low temperature. Therefore, it has not been expected that vaterite can be sintered, and no sintered vaterite has been found so far. However, it has been found that as mentioned later, medical sintered vaterite can be produced by compacting and sintering calcium carbonate powder that contains 20 mass% or larger of vaterite.

The medical calcium carbonate composition of the present invention and a medical composition produced using the composition as a raw material is used in wet environment such as the inside the body. Sintered bodies are objects obtained by settling powders. Vaterite compacts release powders when rubbed after attachment of water, and collapse and cannot keep their shapes while releasing powders when immersed in water. Hence, in the present invention, the presence or absence of sintering in medical sintered vaterite is determined from the shape retention of the sintered body in water. Saturated aqueous solution of calcium carbonate in 10 times the amount of the composition is placed in a glass container, and the composition is immersed therein. The glass container is placed in an ultrasonic washing machine with 28 kHz and an output of 75 W and ultrasonically irradiated for 1 minute. When the dry weight of the composition is 95% or more with respect to the dry weight before ultrasonic irradiation, the composition is confirmed to keep its shape even in water and defined as a sintered body. When the composition is partially broken by ultrasonic irradiation, the dry weight of a composition having the largest volume is used.

[I Medical calcium carbonate composition: specific fine structure and composition]

Next, [3] will be described.

The medical calcium carbonate composition according to [1] or [2], wherein calcium carbonate powders that satisfy at least one selected from the group consisting of (AJ1) to (AJ4) conditions, are bonded to form the calcium carbonate composition is preferred from the viewpoint of the balance between compressive strength and reactivity.

A larger mean particle diameter increases the number of formed interconnected pores and enhances reactivity and however, reduces compressive strength. For forming interconnected pores while maintaining compressive strength, particles having high sphericity are preferred because packing closer to the closest packing is attained.

From these viewpoints, the mean particle diameter is preferably 2 $\mu$m or larger and 8 $\mu$m or smaller, more preferably 3 $\mu$m or larger and 7 $\mu$m or smaller, further preferably 4 $\mu$m or larger and 6 $\mu$m or smaller. The sphericity is preferably 0.9 or larger, more preferably 0.95 or larger, further preferably 0.97 or larger. The mean particle diameter and the sphericity are measured and calculated by dividing particles at the grain boundary of the calcium carbonate composition.

Calcium carbonate compositions sintered too much cannot maintain interconnected pores. Hence, it is preferred that the calcium carbonate particles should contain a trace element that can control sinterability. This element must not influence biocompatibility. From these viewpoints, Mg and Sr are selected. The Mg content is preferably $5 \times 10^{-4}$ mass% or larger and $3 \times 10^{-3}$ mass% or smaller, more preferably $1 \times 10^{-3}$ mass% or larger and $2.5 \times 10^{-3}$ mass% or smaller, further preferably $1.5 \times 10^{-3}$ mass% or larger and $2.5 \times 10^{-3}$ mass% or smaller. The Sr content is preferably $3 \times 10^{-3}$ mass% or larger and $1.5 \times 10^{-2}$ mass% or smaller, more preferably $4 \times 10^{-3}$ mass% or larger and $1.3 \times 10^{-2}$ mass% or smaller, further preferably $5 \times 10^{-3}$ mass% or larger and $1 \times 10^{-2}$ mass% or smaller.

It is preferred that any one, more preferably two or more, further preferably all, of the conditions (AJ1) to (AJ4) should be satisfied.

[I: Medical calcium carbonate composition: bended honeycomb structure] Next, [4] will be described.

In the medical calcium carbonate composition wherein the above described (E) condition is satisfied, a specific condition related to the morphology of a medical carbonate apatite honeycomb structure is that "a diameter of the circle that passes through both ends of any one of the through-holes and a center of the through-hole, is 1 cm or longer, and 50 cm or shorter".

Bones include straight cylindrical bones as well as bended and curved cylindrical bones. A medical calcium carbonate honeycomb structure that is curved cylindrical in shape is useful for the reconstruction of such curved cylindrical bones. This is because bones are conducted to the inside of the through-holes of the medical calcium carbonate honeycomb structure, and because bones are not conducted to the inside of through-holes that are not contacted with the bones even if a curved cylindrical honeycomb structure is produced by processing a straight cylindrical honeycomb structure.

In the case of constructing a bone in a direction vertical to the surface of a bone, it is preferred that the through-holes of a honeycomb structure should open only on the bone surface without opening on the connective tissue around the bone. The connective tissue cannot invade the honeycomb structure having such a structure, whereas only bone tissues are conducted thereto. Thus, the honeycomb structure having such a structure can be produced by bending a honeycomb structure, and, if necessary, cutting the honeycomb structure such that its through-holes open only on one surface.

In the case of decreasing the angle of a rising portion of the bended honeycomb structure from the surface of a bone, it is useful to further bend the honeycomb structure in a direction that is not parallel to the surface formed by the circle that passes through both ends of any one of the through-holes and a center of the through-hole.

A preferred condition of the honeycomb structure is that a diameter of the circle that passes through both ends of any one of the through-holes and a center of the through-hole is 1 cm or longer, and 50 cm or shorter. The diameter of the circle is more preferably 2 cm or longer and 20 cm or shorter. The diameter of the circle is further preferably 3 cm or longer and 10 cm or shorter.

[II Method for producing medical calcium carbonate]

Next, a process of producing the medical calcium carbonate composition of the present invention will be described.

A raw material calcium composition may contain porogen and a fiber that connects plural composition particles. The porogen and the fiber that connects plural composition particles are excluded from, for example, the calculation of vaterite content.

The porogen is a material that forms pores by removal. The porogen does not have to be removed from an in-process medical calcium carbonate composition, and may be a material that is removed at a stage of producing other medical compositions using the medical calcium carbonate composition as a raw material. Examples thereof include salts such as sodium chloride, potassium chloride, and disodium hydrogen phosphate, and acrylic polymer beads. A water soluble inorganic material is preferred from the viewpoint of an easy removal process.

For example, in the case of using sodium chloride as porogen and calcium hydroxide as raw material calcium composition, both of them are mixed and then compacted, and carbon dioxide is added to the calcium hydroxide compact for compositional transformation into vaterite. Even if carbon dioxide is added to calcium hydroxide, sodium chloride is neither reacted nor removed. The porogen sodium chloride exists in a medical vaterite block. For example, in a process of immersing the block in an aqueous disodium hydrogen phosphate solution in order to produce a medical carbonate apatite block from the block, the porogen is dissolved at the same time with the compositional transformation of vaterite into carbonate apatite. As a result, a medical carbonate apatite porous structure is produced.

The fiber is used, as mentioned above, for connecting composition particles for improvement in the handleability of the composition. Since the fiber is used for improving the handleability of medical calcium carbonate granules, etc., or medical calcium phosphate granules, etc. produced using the composition as a raw material, it is preferred that the fiber should exist inside particles such as the granules.

[II Method for producing medical calcium carbonate: (D) vaterite composition]

First, [5] will be described.

For the production of a metastable phase vaterite composition, a production method is useful which involves relatively forming the metastable phase vaterite composition by inhibiting stable phase calcite formation, and inhibiting the transfer from the metastable phase vaterite to stable phase calcite. Organic materials inhibit stable phase calcite formation and relatively accelerate vaterite formation (although ammonia and ammonia salt, which are inorganic materials, are also useful for inhibiting transfer to calcite, the organic materials will be described for the sake of convenience).

Water functions both to accelerate the carbonation of raw material calcium composition and to accelerate transfer from vaterite to calcite. In the absence of water, the raw material calcium composition is not ionized. The solubility of carbon dioxide in organic solvents, which are organic materials, is also limited, and carbon dioxide does not form carbonate ion by dissolution in water. In the presence of water, calcium ion formed from raw material calcium reacts with carbonate ion formed from carbon dioxide. Therefore, water accelerates the formation reaction of calcium carbonate.

When carbon dioxide is added to raw material calcium composition, water is formed in the raw material calcium composition. For example, in a process of producing vaterite by exposing a calcium hydroxide compact to carbon dioxide, water having the same molar number as that of vaterite is formed in the calcium hydroxide compact. Water is necessary for vaterite formation and however, accelerates transfer from metastable phase vaterite to stable phase calcite. Therefore, an excess of water is unfavorable for vaterite production. In the production of vaterite powders using calcium hydroxide powders as raw materials, water is easily diffused into the environment and therefore is not problematic. However, the subject of the present invention is a medical calcium carbonate composition wherein "(A) a volume is $10^{-12}$ $m^3$ or larger", and diffusion from the inside of the composition into the outside of the composition is not easy. Hence, a process of discharging water formed in raw material calcium composition to the outside of the raw material calcium composition is important.

A medical calcium carbonate composition that satisfies all of the above described (A) to (C) conditions and the (D) condition is produced by exposing raw material calcium composition (which may contain porogen and a fiber that connects plural composition particles) which volume is $10^{-12}$ $m^3$ or larger to carbon dioxide or carbonate ion under a specific condition.

Specifically, a process of "(D1) inhibiting calcite formation or calcite crystal growth, and relatively promoting formation of calcium carbonate other than calcite" is useful for a method for producing the medical calcium carbonate composition that satisfies the (D) condition.

Since at least one selected from the group consisting of organic solvent, water soluble organic material, ammonia, and ammonium salt inhibits calcite formation or calcite crystal growth, a process of "(D2) exposing of raw material calcium composition to carbon dioxide or carbonate ion, and at least one selected from the group consisting of organic solvent, water soluble organic material, ammonia, and ammonium salt" is preferred.

The formation reaction of the medical calcium carbonate composition that satisfies the (D) condition from the raw material calcium composition needs to also proceed inside the raw material calcium composition. It may therefore be preferred to add the above described substance inhibiting calcite formation or calcite crystal growth to the raw material calcium composition.

Specifically, a process of "(D3) exposing raw material calcium composition that contains at least one selected from the group consisting of organic solvent, water soluble organic material, ammonia, and ammonium salt, to carbon dioxide or carbonate ion, and at least one selected from the group consisting of organic solvent, water soluble organic material, ammonia, and ammonium salt" may be preferred for a method for producing the medical calcium carbonate composition that satisfies the (D) condition. The raw material calcium composition that contains the organic solvent can be handled as paste and may therefore be useful from the viewpoint of handleability as well.

Methanol, ethanol, and ammonium carbonate are substances that vaporize, and are easy to remove from produced medical vaterite compositions. Glycerin and ethylene glycols are highly water soluble substances and are easy to remove from produced medical vaterite compositions. In this context, the ethylene glycols refer to ethylene glycol and polyethylene glycol.

These substances are preferred from the viewpoint of the balance between a convenient production process and a vaterite inhibitory effect. Specifically, a process of "(D4) exposing raw material calcium composition to carbon dioxide or carbonate ion, and at least one selected from the group consisting of methanol, ethanol, glycerin, ethylene glycols, and ammonium carbonate" is more preferred for a method for producing the medical calcium carbonate composition that satisfies the (D) condition, and "(D5) a process of exposing raw material calcium composition that contains at least one selected from the group consisting of methanol, ethanol, glycerin, ethylene glycols, and ammonium carbonate, to carbon dioxide or carbonate ion, and at least one selected from the group consisting of methanol, ethanol, and ammonium carbonate" is further preferred.

As mentioned above, metastable phase vaterite is transferred to stable phase calcite. Hence, "(D6) a process of inhibiting transfer from vaterite to calcite" is useful for the medical calcium carbonate composition that satisfies the (D) condition. As mentioned above, transfer from vaterite to calcite is accelerated by water. In the case of producing a medical vaterite composition through the reaction of raw material calcium composition with carbon dioxide, water in an equimolar amount with respect to calcium carbonate is secondarily produced inside the raw material calcium composition. Therefore, "(D7) a process of removing water from the raw material calcium composition" is useful for the medical calcium carbonate composition that satisfies the (D) condition.

For removing water secondarily produced in the raw material calcium composition, it is necessary to discharge water from the inside to the outside of the raw material calcium composition by diffusion (e.g., vaporization). Although a process of performing a pressure reduction operation for water removal, for example, is also possible amid the carbonation of the raw material calcium composition, "(D8) a process of circulating carbon dioxide or carbonate ion containing organic solvent around the raw material calcium composition" is useful because this process facilitates the vaporization of water inside the raw material calcium composition and can be continuously performed. Examples of the method for circulating carbon dioxide or carbonate ion containing organic solvent around the raw material calcium composition include the spraying of carbon dioxide or carbonate ion containing organic solvent to the raw material calcium composition using a fan or the like, the rotation of carbon dioxide or carbonate ion containing organic solvent around the raw material calcium composition.

Medical vaterite compositions are produced by adding carbon dioxide to raw material calcium composition under a specific condition. The raw material calcium composition is preferably calcium hydroxide. This is because any composition other than water is not secondarily produced. Although calcium hydroxide compacts are generally used, the calcium hydroxide compacts collapse and cannot keep their shapes when immersed in liquid phase such as an organic solvent. Hence, it is necessary to add carbon dioxide under gas phase. On the other hand, homogeneous reaction is less likely to occur under gas phase compared with liquid phase. Hence, a process of producing a medical vaterite composition under at least one condition selected from the group consisting of (D1) to (D8), and "(D9) a process of partial carbonation by exposing raw material calcium composition to carbon dioxide or carbonate ion under gas phase, followed by exposing the raw material calcium composition to carbon dioxide or carbonate ion under liquid phase" are useful.

Calcium hydroxide compacts, etc. collapse when immersed in liquid phase such as 90% ethanol, whereas calcium hydroxide paste, etc. can be placed in a mold and immersed in liquid phase without being disintegrated. Hence, a process of producing a medical vaterite composition under at least one condition selected from the group consisting of (D1) to (D8), and "(D10) a process of exposing raw material calcium composition in a mold to carbon dioxide or carbonate ion" are useful. The mold is not particularly limited by its shape and needs to be a mold that opens at least partially and permits reaction with external carbon dioxide or carbonate ion because the contents of the mold need to react with carbon dioxide or carbonate ion. Since the reaction proceeds from the whole surface, a mold produced from a breathing material is preferred. The raw material calcium composition in a mold does not have to be immersed in liquid phase and is also useful for reaction under gas phase from the viewpoint of the production of medical vaterite compositions in desired forms, etc.

Medical vaterite compositions are produced by exposing raw material calcium composition such as calcium hydroxide compact or calcium hydroxide paste to carbon dioxide or carbonate ion. If the raw material calcium composition contains porogen (e.g., sodium chloride, sodium dihydrogen phosphate, and polymer beads) or a fiber, the porogen or the fiber does not react. The porogen is useful in porous structure production, and the fiber is useful in the production of compositions excellent in handleability. For example, a medical calcium carbonate composition that contains porogen and satisfies the (D) condition can be produced by a process of producing a medical vaterite composition under at least one condition selected from the group consisting of (D1) to (D8), and "(D11) exposing raw material calcium composition that contains porogen to carbon dioxide or carbonate ion". The porogen can be removed to produce a medical calcium carbonate porous structure, a medical calcium phosphate porous structure, or the like. The porogen can be mixed into the raw material calcium carbonate.

A medical calcium carbonate composition, such as granules connected with a fiber, which satisfies the (D) condition can be produced by a process of producing a medical vaterite composition under at least one condition selected from the group consisting of (D1) to (D8), and "(D12) exposing raw material calcium compositions that are connected with a fiber to carbon dioxide or carbonate ion". As mentioned above, medical calcium carbonate compositions or medical calcium phosphate compositions which particles are connected with a fiber are excellent in handleability.

(Preferred raw material calcium composition)

In a process of producing the medical calcium carbonate composition that satisfies the (D) condition, the raw material calcium composition is not particularly limited as long as the composition contains calcium. Calcium hydroxide and calcium oxide are particularly preferred. This is because, as mentioned above, any composition other than water is not secondarily produced when calcium carbonate is formed in a process of exposing these compositions to carbonate or carbonate ion.

(Organic solvent)

The organic solvent described in the present invention is a solvent of an organic material and includes a hydrous organic solvent. Examples of the organic solvent include alcohols, ketone, and hexane.

An alcohol or ketone is desirable from the viewpoint of the ability to inhibit calcite formation, cost, the ability to contain water, and easy removal. A lower alcohol or lower alkyl ketone is more preferred, and an aliphatic alcohol having 1 to 4 carbon atoms or di-lower alkyl ketone having a total of 3 to 6 carbon atoms is further preferred.

Examples of the lower alcohol include methanol, ethanol, and propanol. Methanol, ethanol and propanol are preferred, and methanol and ethanol are more preferred.

Examples of the lower alkyl ketone include acetone, methyl ethyl ketone, diethyl ketone, and methyl isobutyl ketone. Particularly, acetone or methyl ethyl ketone is most suitable.

(Water soluble organic material)

The water soluble organic material described in the present invention is an organic material that can be dissolved in water, and includes a salt of the organic material. Examples thereof include nonionic surfactants, lignin sulfonic acid salt, saccharides such as saccharose, alkylamine salt type surfactants, glycerin, ethylene glycol, polyethylene glycol, propylene glycol, and polypropylene glycol.

The water soluble organic material needs to be completely removed after production of the medical calcium carbonate composition and may be inferior in usefulness to organic solvents, ammonia, and ammonium chloride which vaporize. Glycerin, ethylene glycol, and polyethylene glycol may be useful for extrusion forming and removal from products because their viscosity and solubility in water are high.

(Ammonia and ammonium salt)

The ammonia described in the present invention is $NH_3$ and includes ammonia water $NH_4OH$. The ammonium salt described in the present invention is a salt of ammonia. Examples thereof include ammonium carbonate, ammonium chloride, and ammonium nitrate.

Among them, ammonium carbonate is also used in a carbonation process and is therefore particularly useful.

[II Method for producing medical calcium carbonate composition: (D) sintered vaterite]

Next, [6] will be described.

As mentioned above, vaterite is a metastable phase and is known as a stable phase at low temperature. Therefore, it has not been considered that vaterite can be sintered. However, it has been found that medical sintered vaterite that contains 20 mass% or larger of vaterite can be produced by a process of "compacting and sintering calcium carbonate powder that contains 20 mass% or larger of vaterite". The compacting and sintering conditions are not particularly limited as long as a sintered body can be produced by settling powders under the conditions. The compacting pressure is preferably 100 MPa or higher, more preferably 130 MPa or higher, further preferably 160 MPa or higher.

The sintering temperature is preferably 200°C or higher, more preferably 220°C or higher, further preferably 240°C or higher. When a calcium carbonate compact that contains 20 mass% or larger of vaterite is sintered, the vaterite, albeit sintered, is decomposed into calcite at a temperature over a certain level. This temperature depends on the environment and is therefore not particularly limited. In the case of atmospheric sintering, the sintering temperature is preferably 600°C or lower, more preferably 550°C or lower, further preferably 500°C or lower. As mentioned above, transfer from metastable phase vaterite to stable phase calcite is accelerated by moisture. Hence, moisture-free sintering conditions are preferred, and carbon dioxide atmosphere is more preferred from the viewpoint of the suppression of decomposition. Thus, moisture-free carbon dioxide atmosphere is further preferred.

[II Method for producing medical calcium carbonate composition: (E) carbonate apatite honeycomb structure]

Next, [7] will be described.
A medical calcium carbonate honeycomb structure is produced by performing "(E1) Extrusion process" and one "debindering and carbonation process" selected from the group consisting of (E5) to (E9) as essential processes and optionally a process selected from the group consisting of (E2) to (E4) and (E10).

<(E1) Extrusion process>

An extrusion process comprises producing a raw material honeycomb structure comprising a plurality of through-holes extending in one direction, having a volume of $3 \times 10^{-11}$ m³ or larger by extruding a raw material calcium composition comprising polymer material through a honeycomb structure forming die.
A known polymer material is used as the polymer material. The polymer material is also called polymer binder or organic binder in the sense that powders are bonded. These terms have the same meaning in the present invention.
The polymer material is preferably a polymer material serving as a wax-acrylic resin-based (also simply referred to as wax-based) organic binder. This is because, unlike other forming methods, the forming of the honeycomb structure requires fluidity at the time of extrusion and hardenability after extrusion.

<(E2) Forming process after extrusion process>

It may be useful to perform a forming process after the essential process (E1).
This process comprises forming honeycomb structure consisting of a raw material calcium composition comprising polymer material to a desired form by softening by a thermal treatment, followed by pressure loading. The softening temperature is adjusted depending on the type of the polymer material, etc. and is generally 50°C or higher and 200°C or lower.
Forming into a desired form facilitates "(E3) Removal process of peripheral wall". The term "after extrusion process" means after the raw material calcium composition comprising polymer material passes through a honeycomb structure formation mold. The case of performing forming immediately after the raw material calcium composition comprising polymer material passes through a honeycomb structure formation mold, while extruding the raw material calcium composition comprising polymer material is also defined as after extrusion process.
In the case of producing a honeycomb structure wherein a diameter of the circle that passes through both ends of any one of the through-holes and a center of the through-hole is 1 cm or longer, and 50 cm or shorter, it is preferred to perform forming into this shape at this stage.
"(E3) Removal process of peripheral wall" comprises removing peripheral wall after (E1) or (E2), and before one "debindering and carbonation process" selected from the group consisting of (E5) to (E9).
"(E4) Forming process after removal process of peripheral wall" comprises forming a honeycomb structure consisting of a raw material calcium composition comprising polymer material to a desired form through softening by thermal treatment, followed by pressure loading. The softening temperature is adjusted depending on the type of the polymer material, etc. and is generally 50°C or higher and 200°C or lower.
A "debindering and carbonation process" is performed following the essential process (E1) and the optional processes (E2) to (E4). In this context, the "debindering and carbonation process" means a process of debindering and calcium carbonate formation or retention. When the raw material calcium composition is calcium carbonate, it is not necessary to add carbon dioxide. However, in the present invention, in the case of using calcium carbonate as a raw material, this process is also defined as a debindering and carbonation process. This process that is performed at the same time with a sintering process is also included in the "debindering and carbonation process".
The debindering is a process of removing the polymer material and is generally performed by heat treatment. In the case of preparing an alumina or cordierite honeycomb structure, the debindering is relatively easy because these ceramics are not thermally decomposed. In the production of the medical calcium carbonate composition of the present invention having high reactivity, the debindering is very difficult. This is because calcium carbonate or a raw material for calcium carbonate production is thermally decomposed or becomes poorly reactive calcium carbonate at high temperature. Hence, it is necessary to perform debindering under a specific condition so that the polymer material or remaining materials after acid dissolution, which are thermal decomposition products thereof, are 1 mass% or less. The remaining materials after acid dissolution after the debindering process are essentially 1 mass% or less, preferably 0.5 mass% or smaller, more preferably 0.3 mass% or smaller, further preferably 0.1 mass% or smaller, ideally substantially 0 mass%.

Various conditions such as composition, porosity, particle diameter, atmosphere, temperature, debindering time, and heating rate influence debindering. An index for debindering is remaining materials after acid dissolution of the calcium carbonate composition thus debindered, and the debindering cannot be evaluated from color, etc.

As mentioned above, it may be necessary to perform carbonation at the same time with debindering or after debindering.

The debindering and carbonation process is a process including at least one process selected from the group consisting of (E5) to (E9) given below. Debindering and carbonation are performed such that a volume of pores which pore diameter is 10 $\mu$m or smaller with respect to a mass of the honeycomb structure analyzed by mercury intrusion porosimetry is larger than 0.02 cm$^3$/g. A higher debindering temperature gives a smaller pore volume. When the pore volume is 0.02 cm$^3$/g or smaller, the pore volume can be increased by lowering the debindering temperature.

<(E5) Debindering and calcium carbonate sintering process>

As described in Patent Literature 11, it has been considered in relation to calcium carbonate honeycomb structure production that calcium carbonate has poor sinterability and is unsuitable as a raw material because of being thermally decomposed at high temperature; and methods using calcium hydroxide are useful. Specifically, honeycomb structure production requires a relatively large amount of a polymer material. In the case of producing a medical calcium carbonate honeycomb structure from a honeycomb structure of a polymer material containing-calcium composition, high temperature is necessary as compared with the sintering of ceramic compacts because pressure cannot be applied to between powders. However, calcium carbonate is thermally decomposed at high temperature. Furthermore, since calcium carbonate has poor sinterability, it has been considered impossible to sinter polymer material-containing calcium carbonate honeycomb structures produced using polymer material-containing calcium carbonate powders.

However, it has been found that, surprisingly, polymer material-containing calcium carbonate can be heat debindered under a specific condition so that remaining materials after acid dissolution is 1 mass% or smaller; as a result, calcium carbonate is sintered to produce a medical calcium carbonate porous structure having large mechanical strength.

An essential condition for producing a medical calcium carbonate honeycomb structure excellent in mechanical strength from a polymer material-containing calcium carbonate honeycomb structure is heat debindering under a specific condition described below so that remaining materials after acid dissolution is 1 mass% or smaller. At present, much remains to elucidate the detailed reason for this.

Calcium carbonate is difficult to thermally decompose up to approximately 500°C. Hence, calcium carbonate does not require controlling atmosphere up to approximately 500°C, and may be heated in the atmosphere. It starts to be thermally decomposed at a temperature exceeding approximately 500 in the atmosphere to be calcium oxide chronologically, and is stable up to 920°C in carbon dioxide atmosphere. Thus, heat debindering at a temperature exceeding approximately 500°C needs to be performed under a condition where calcium carbonate is not thermally decomposed, by elevating carbon dioxide partial pressure. The sintering temperature of calcium carbonate varies depending on powder size, etc. as mentioned later. In the case of producing a calcium carbonate honeycomb having high reactivity, it is necessary to perform debindering carbonation such that a volume of pores which pore diameter is 10 $\mu$m or smaller with respect to a mass of the honeycomb structure analyzed by mercury intrusion porosimetry is larger than 0.02 cm$^3$/g. Although heat debindering in the atmosphere is generally desirable in light of cost, etc., heat debindering at a carbon dioxide concentration higher than that in air is preferred from the viewpoint of the suppression of calcium carbonate decomposition.

As mentioned above, the presence or absence of sintering in the present invention is determined on the basis of whether or not to keep a shape without collapsing under a condition involving immersion in water and ultrasonic irradiation.

<(E6) Debindering and carbonation process>

In a process of heat debindering of a polymer material containing-calcium hydroxide porous structure so that remaining materials after acid dissolution is 1 mass% or smaller under an oxygen concentration of less than 30%, and carbonation at the same time, polymer material containing-calcium hydroxide is debindered by heating and carbonated at the same time. In this context, the same time means the same time during one process. In actuality, the debindering is performed first by heating. For carbonation at the same time with debindering of the polymer material, the control of heat debindering temperature and heat debindering atmosphere as well as heat debindering time is important. This is because calcium hydroxide is thermally decomposed into calcium oxide from approximately 345°C under a condition where carbon dioxide partial pressure is low. Although the carbon dioxide partial pressure does not have to be increased up to this temperature, the change of the carbon dioxide partial pressure during a process is complicated. Therefore, it is preferred to increase the carbon dioxide partial pressure from the beginning.

As described in Patent Literature 12, the incineration of a polymer material has been considered necessary for debindering. As a result of diligent studies, it has been found that incineration is one of the debindering methods and debindering can be achieved by depolymerization, vaporization, or the like without incineration. It has also been found that the incineration of a polymer material causes incomplete combustion and may allow carbon to remain as remaining materials after acid dissolution.

Hence, it has been found that, surprisingly, debindering under a condition without the incineration of a polymer material may be preferred. For debindering a polymer material by a method other than incineration, such as depolymerization or vaporization, an oxygen concentration needs to be less than 30% in terms of vol% of oxygen. The oxygen concentration is preferably less than 15%, more preferably less than 10%. An oxygen-free situation, i.e., an oxygen concentration of substantially 0%, is further preferred.

Debindering behavior differs between polymer material containing-calcium hydroxide and polymer material-containing calcium carbonate. In the case of heat debindering polymer material-containing calcium carbonate, remaining materials after acid dissolution are less likely to remain even in the presence of oxygen. On the other hand, as for polymer material containing-calcium hydroxide, remaining materials after acid dissolution are more likely to remain in the presence of oxygen. Although much remains to elucidate a cause thereof, it is probably due to the reactivity between calcium hydroxide and a polymer material. Specifically, since calcium carbonate has limited reactivity with a polymer material, the polymer material is easily debindered by depolymerization, vaporization, or the like. On the other hand, calcium hydroxide has relatively high reactivity with a polymer material and therefore probably interacts or reacts with the polymer material. The polymer material that has interacted or reacted with calcium hydroxide may be easily debindered by depolymerization, vaporization, or the like.

For preventing the thermal decomposition of calcium hydroxide and performing debindering and carbonation, it is preferred to perform debindering and carbonation at 600°C or higher and 800°C or lower using 50 vol% or more of carbon dioxide and less than 30 vol% of oxygen.

One of the essential conditions of the present invention is complete debindering of a polymer material. If complete debindering cannot be achieved by debindering and carbonation at 600°C or higher and 800°C or lower using 50 vol% or more of carbon dioxide and less than 30 vol% of oxygen, this production method is not included in the present invention. For honeycomb structures, it is essential to perform debindering and carbonation such that a volume of pores which pore diameter is 10 $\mu$m or smaller with respect to a mass of the honeycomb structure analyzed by mercury intrusion porosimetry is larger than 0.02 cm$^3$/g. Heat treatment is performed under a condition where a polymer material can be completely debindered, by studying appropriate heat treatment temperature, heat treatment atmosphere, and heat treatment time.

<(E7) Debindering and carbonation process via calcium oxide>

In the aforementioned "(E6) Debindering and carbonation process", a calcite porous structure can be produced relatively conveniently from a polymer material containing-calcium hydroxide porous structure. However, since calcite is formed at 600°C or higher and 800°C or lower, the calcite porous structure that can be produced often has poor reactivity. Although much remains to elucidate a cause thereof, it is probably due to generally necessary heat treatment at 600°C or higher and 800°C or lower which forms calcite having low reactivity.

From this viewpoint, the debindering process can be performed at high temperature, and calcite or vaterite can be formed after lowering of the temperature.

Calcium hydroxide is thermally decomposed into calcium oxide by heat debindering a polymer material containing-calcium hydroxide porous structure without forming calcite. Such high-temperature treatment has the advantage that complete debindering is achieved. At high heat treatment temperature, calcium carbonate having a low degree of crystallinity cannot be produced because a calcium oxide porous structure becomes precise or the degree of crystallinity is high. Hence, the heat treatment temperature for calcium oxide production is preferably 700°C to 1000°C, more preferably 750°C to 950°C, further preferably 800°C to 900°C.

Calcium carbonate is also thermally decomposed into calcium oxide by heat debindering a polymer material-containing calcium carbonate porous structure. Since calcium carbonate has lower reactivity as compared with calcium hydroxide, heat debindering can be achieved at low temperature. The heat treatment temperature for calcium oxide production from a polymer material-containing calcium carbonate porous structure is preferably 500°C to 1000°C, more preferably 530°C to 800°C, further preferably 550°C to 650°C.

After the debindering, the temperature is lowered, and carbon dioxide is then added to the calcium oxide porous structure to produce a calcite porous structure or a vaterite porous structure. A lower temperature at which carbon dioxide is added to the calcium oxide porous structure can produce a calcite porous structure having higher reactivity and however, requires more time for adding carbon dioxide. The temperature at which carbon dioxide is added to the calcium oxide porous structure is preferably 300°C to 500°C, more preferably 310°C to 400°C, further preferably 320°C to 380°C, from the viewpoint of the balance therebetween. In the case of producing a vaterite porous structure from the calcium oxide porous structure, carbon dioxide is added to the calcium oxide porous structure by a method such as the aforementioned (D1) to (D12).

<(E8) Debindering and carbonation process via calcium carbonate and calcium oxide>

In the above described "(E7) Debindering and carbonation process via calcium oxide", a calcite porous structure having relatively high reactivity, or a vaterite porous structure having very high reactivity can be produced. However, heat treatment of a polymer material-containing calcium hydroxide under carbon dioxide atmosphere to be a polymer material containing-calcium carbonate porous structure, followed by heat debindering to be a calcium oxide porous structure, followed by lowering the temperature and then adding carbon dioxide to the calcium oxide porous structure to produce a calcite porous structure or a vaterite porous structure can produce a calcium carbonate porous structure having larger mechanical strength or is less likely to yield a cracked calcium carbonate porous structure, as compared with the case of forming a calcium oxide porous structure directly from a polymer material containing-calcium hydroxide porous structure. Although much remains to elucidate a cause thereof, it is probably because the time for which a calcium hydroxide porous structure inferior in mechanical strength is heated can be decreased by adding carbon dioxide to calcium hydroxide. The temperature at which carbon dioxide is added to the calcium oxide porous structure is preferably 300°C to 500°C, more preferably 310°C to 400°C, further preferably 320°C to 380°C, as in "(E7) Debindering and carbonation process via calcium oxide".

<(E9) Debindering and carbonation process of calcium sulfate>

A calcite porous structure having more micropores can be produced by using calcium sulfate as compared with the case of using calcium carbonate or calcium hydroxide as described above. Although much remains to elucidate a cause thereof, it is, presumably, partly because calcium sulfate has a coarse crystal structure.
In a debindering and carbonation process of heat debindering of a polymer material containing-calcium sulfate so that remaining materials after acid dissolution is 1 mass% or smaller, followed by adding carbon dioxide or carbonate ion to the produced calcium sulfate porous structure to be a calcium carbonate, calcium sulfate is used as a raw material calcium composition. First, a process of debindering a polymer material-containing calcium sulfate porous structure by heat treatment at 700°C or higher so that remaining materials after acid dissolution is 1 mass% or smaller is performed. Next, carbonate ion is added to the produced calcium sulfate porous structure to produce a medical calcium carbonate porous structure.
Calcium sulfate is stable even at relatively high temperature. Debindering at 700°C or higher is an essential condition. The final heat treatment temperature is preferably 750°C to 1100°C, more preferably 800°C to 1000°C, further preferably 850°C to 950°C.

<(E10) A process of structure finishing process after debindering and carbonation processes>

After any one debindering and carbonation process described in (E5) to (E9), "(E10) a process of structure finishing process after debindering and carbonation processes" is performed. In the structure finishing process, structure finishing such as peripheral wall removal is performed.

[II Method for producing medical calcium carbonate composition: (E) specific calcium carbonate honeycomb structure]

Next, [8] will be described.
A method for producing the medical calcium carbonate composition that satisfies the above described (E), wherein the medical calcium carbonate composition satisfies at least one of the conditions selected from the following conditions (E11) to (E14), is useful.
(E11) is a process in which in the above-described "(E1) Extrusion process", honeycomb structure is extruded so that thickness of peripheral wall of the honeycomb structure is thicker than that of the partition wall, and cross-sectional area vertical to the through-holes is 1 $cm^2$ or larger.
This is because when the cross-sectional area vertical to the through-holes is 1 $cm^2$ or larger, shape retention is easy by extrusion so that thickness of peripheral wall of the honeycomb structure is thicker than the wall thickness of the honeycomb structure, from the viewpoint of shape retention at the time of extrusion, etc.

On the other hand, in a debindering and carbonation process of a honeycomb structure of a raw material calcium composition comprising polymer material wherein thickness of peripheral wall is thicker than the wall thickness of the honeycomb structure, and cross-sectional area vertical to the through-holes is 1 cm$^2$ or larger, cracks are easily formed between the peripheral wall and the partition wall or inside the honeycomb structure due to the difference in shrinkage between the peripheral wall and the partition wall. Hence, it is preferred to perform "(E3) Removal process of peripheral wall" before one debindering and carbonation process selected from the group consisting of the above described (E5) to (E9).

If the cross-sectional area vertical to the through-holes is less than 1 cm$^2$, problems associated with shape retention at the time of extrusion are limited. If the thickness of peripheral wall is the same as or smaller than that of the partition wall of the honeycomb structure, the need of "(E3) Removal process of peripheral wall" is limited because the shrinkage factor is the same or smaller. If the cross-sectional area vertical to the through-holes is less than 1 cm$^2$, cracks are less likely to occur because difference in the amount of shrinkage is limited, albeit different shrinkage factors. Thus, the need of "(E3) Removal process of peripheral wall" is limited.

(E12) is a production process in which in at least one selected from the group consisting of the processes of "(E1) Extrusion process", "(E2) Forming process after extrusion process", "(E4) Forming process after removal process of peripheral wall", and "(E10) Structure finishing process after debindering and carbonation processes", a heat softened honeycomb structure comprising raw material calcium composition containing polymer material is bended by applying a pressure so that the diameter of the circle that passes through both ends and the center of one of the through-holes, is 1 cm or longer, and 50 cm or shorter.

The softening temperature is adjusted depending on the type of the polymer material, etc. and is generally 50°C or higher and 200°C or lower.

(E13) is a production process in which the "(E3) Removal process of peripheral wall" is done with a cutter grinder, and the "(E10) process of structure finishing process after debindering and carbonation processes" is done by polishing, and is useful for a method for producing a medical calcium carbonate honeycomb excellent in profile morphology.

In an attempt to eliminate the peripheral wall of a raw material honeycomb structure formed from a raw material calcium composition comprising polymer material by polishing using a diamond point or the like, the peripheral wall is eliminated whereas a new peripheral wall is formed, which seems to be a phenomenon unique to the peripheral wall removal of the raw material honeycomb structure. This is probably due to the softening of the peripheral wall by heat generation ascribable to the polishing process. On the other hand, heat generation ascribable to a cutter grinder process is limited as compared with the polishing process. Thus, it is preferred to perform the removal process of peripheral wall using a cutter grinder such as a planer.

Chipping occurs by a peripheral wall finishing and removal process performed using a cutter grinder as to a calcium carbonate honeycomb structure produced by the debindering and carbonation of the raw material honeycomb structure. Hence, it is preferred to perform a peripheral wall removal and finishing process by polishing using a diamond point or the like, not a cutter grinder.

(E14) is a production process in which a raw material calcium composition of the "(E1) Extrusion process" is calcium sulfate anhydrous.

Any calcium sulfate honeycomb structure has not been produced so far. A mixture of a raw material calcium composition and a polymer material is heated in an extrusion process in a raw material honeycomb structure forming process. Calcium sulfate includes anhydrous, hemihydrate, and dihydrate. In the case of using hydrous calcium sulfate as a raw material calcium composition, a raw material honeycomb structure formed from polymer material-containing calcium sulfate swells by moisture vaporization so that the honeycomb structure is misshapen. Hence, it is essential to use calcium sulfate anhydrous as the calcium sulfate.

[II Method for producing medical calcium carbonate composition: (F) Swelling of calcium oxide granule]

Next, [9], i.e., a process of producing the medical calcium carbonate composition that satisfies the above described (F) condition using calcium oxide granules as a raw material, will be described.

For bonding granules with voids left, it is necessary to impart some bonding ability to the granules. Methods for imparting some bonding ability to the granules are divided to the case of using a polymer material and the case of using no polymer material. First, a production method using no polymer material will be described. In the case of using no polymer material, it is necessary to exploit the bonding of a raw material calcium composition, and swelling ascribable to the hydration, etc. of calcium oxide, and the hardening reaction of calcium sulfate are useful. First, a production method using calcium oxide granules as a raw material will be described.

Since calcium oxide swells by the addition of water, acetic acid, or the like to be calcium hydroxide or calcium acetate, granules are carbonated by bonding each other through the use of this reaction. This production method comprises the following (F1) and (F2) and comprises at least one of (F3) and (F4) as a necessary condition.

<(F1) Placement-closing process>

This process comprises placing calcium oxide granules in a reaction vessel, and closing the opening of the reaction vessel so that the granules are not escaped from the reaction vessel. This process is performed for the purpose of uniformly bonding the granules to each other in a reaction vessel or applying compressive stress to between the granules. The granules are not uniformly bonded to each other without a closing process, and this is unfavorable for medical porous structures. For forming a uniform porous structure, it is essential to close the opening of the reaction vessel. A placement process that does not involve closing the opening is not included in the present invention. Whether or not the granules are escaped from the reaction vessel is a criterion for determining the presence or absence of closing. If the opening opens so that the granules are not escaped from the reaction vessel, this is defined as being substantially closed. Even a mesh reaction vessel, when closed so that the granules are not escaped from the reaction vessel, is defined as having undergone the placement-closing process.

<(F2) Porous structure producing process>

After the placement-closing process, it is a process of adding water or acetic acid to the calcium oxide granules inside the reaction vessel. The calcium oxide granules swell through reaction with water or acetic acid to be calcium hydroxide or calcium acetate. Since the opening of the reaction vessel is closed by the placement-closing process, the degree of contact among the granules is high so as to form a calcium hydroxide porous structure or a calcium acetate porous structure having a uniform pore structure. Since the calcium oxide granules swell, the volume of 10 $\mu$m or smaller pores in the granule bonded-porous structure analyzed by mercury intrusion porosimetry is 0.05 cm$^3$/g or more.

<(F3) Carbonation process>

In the case of producing a calcium hydroxide porous structure, carbon dioxide is subsequently added to the calcium hydroxide porous structure at the same time with the calcium hydroxide porous structure producing process or after the calcium hydroxide porous structure producing process. The calcium hydroxide porous structure is carbonated into a calcium carbonate porous structure.
On the other hand, in the case of producing a calcium acetate porous structure, the calcium acetate porous structure is heat treated. Calcium acetate is thermally decomposed into a calcium carbonate porous structure. The heat treatment is performed at a temperature equal to or higher than 400°C which is the decomposition temperature of calcium acetate.

<(F4) Calcium oxide carbonation process>

A medical calcium carbonate porous structure can be produced by even a production process comprising all of the above described (F1) to (F3). However, the porous structure may have small compressive strength. A process of producing a calcium carbonate porous structure by heat treatment of calcium hydroxide porous structure, calcium carbonate porous structure, or calcium acetate porous structure, followed by exposing the calcium oxide porous structure to carbon dioxide is useful for increasing compressive strength.

[II Method for producing medical calcium carbonate composition:(F) hardening reaction of calcium sulfate granule]

Next, [10] will be described.
The medical calcium carbonate composition that satisfies the above described (F) can also be produced by hardening calcium sulfate granules of appropriate size.
Specifically, a porous structure may be produced through the hardening reaction of calcium sulfate granules with water containing carbonate ion, or through the hardening reaction of calcium sulfate hemihydrate granules or calcium sulfate anhydrous granules with water. In the former case, a calcium carbonate porous structure is directly produced. In the latter case, a calcium sulfate dihydrate porous structure is produced. Therefore, this porous structure is subjected to a carbonation process for the compositional transformation of calcium sulfate dihydrate into calcium carbonate. Specifically, the former production method comprises the (F5) and (F6) processes given below, and the latter production method comprises the (F5), (F7) and (F9) processes given below and optionally comprises the (F8) process. A method for producing the medical calcium carbonate composition that satisfies the above described (F) can thereby be provided.

<(F5) Placement process>

This process comprises placing calcium sulfate granules in a reaction vessel.

<(F6) Porous structure forming-carbonation process >

It is a process of reacting calcium sulfate granules placed in the reaction vessel with carbonate ion.

For example, the calcium sulfate granules placed in the reaction vessel can be immersed in aqueous sodium carbonate solution. In this process, the calcium sulfate granules are compositionally transformed into calcium carbonate while keeping their macrostructures. At the same time therewith, the granules are hardened together by the cross-linking of the formed calcium carbonate crystals to form a granule bonded-porous structure.

<(F7) Porous structure forming process>

When the calcium sulfate granules consist of calcium sulfate hemihydrate or calcium sulfate anhydrous, the granules placed in the reaction vessel are compositionally transformed into calcium carbonate while keeping their macrostructures, by adding water to the granules. At the same time therewith, the granules are hardened together by the cross-linking of the formed calcium carbonate crystals to form a granule bonded-porous structure.

<(F8) Heat-treatment process>

This process comprises producing a calcium sulfate anhydrous porous structure by heat treatment, if necessary, and dehydration of the calcium sulfate dihydrate porous structure produced by "(F7) Porous structure forming process". A medical calcium carbonate porous structure having large compressive strength can be produced by the heat-treatment process.

<(F9) Carbonation process>

In this process, the calcium sulfate dihydrate porous structure or the calcium sulfate anhydrous porous structure is exposed to water containing carbonate ion. The calcium sulfate dihydrate porous structure or the calcium sulfate anhydrous porous structure is compositionally transformed into calcium carbonate while maintaining macrostructure, to produce a medical calcium carbonate porous structure.

By the (F5) or (F9) process, the volume of 10 $\mu$m or smaller pores in the granule bonded-porous structure analyzed by mercury intrusion porosimetry is 0.05 cm$^3$/g or more.

[II Method for producing medical calcium carbonate composition: (F) process using polymer]

Next, [11], i.e., a method for producing the medical calcium carbonate composition that satisfies the above described (F) using a polymer material, will be described.

Use of a raw material calcium composition comprising polymer material, which is a mixture of a raw material calcium composition and a polymer material, enables the mixture granules to be bonded by the polymer material. On the other hand, since the polymer material is used, the production of the medical calcium carbonate composition of the present invention requires removing the polymer material by debindering, as described above. It is also necessary to perform debindering under a specific condition so that the polymer material or remaining materials after acid dissolution, which are thermal decomposition products thereof, are 1 mass% or less, as in the case of a medical calcium carbonate honeycomb structure.

A method for producing the medical calcium carbonate composition that satisfies the above described (F) can be provided by comprising the following (F10) and (F11) and one selected from the group consisting of the above described (E5) to (E9) as essential processes, and optionally comprising the above described (E10) process.

<(F10) Placement process>

It is a process of placing raw material calcium composition granules containing polymer having a volume of 10$^{-12}$ m$^3$ or larger in a reaction vessel.

<(F11) Porous structure forming process>

This process comprises producing granule bonded-porous structure formed from a plurality of granules which maximum diameter is 50 $\mu$m or longer and 500 $\mu$m or shorter bonded to each another, and comprising a plurality of through-holes extending in plural directions, and having a volume of 3 $\times$ 10$^{-11}$ m$^3$ or larger, wherein a volume of pores with a pore diameter of 10 $\mu$m or smaller analyzed by mercury intrusion porosimetry is 0.05 cm$^3$/g or more, by fusing the granules in the reaction vessel based on heat treatment so that the surface is softened and fused one another, or by fusing of the surface of granules to bond the surface of the granules one to another, or by fusing the surface of granules one to another with a plasticizer.

In the process of fusing the granules based on heat treatment so that the surface is softened and fused one another, the granules are heated. In the case of a thermoplastic polymer, the granules are softened, and the softened granules are fused by the self-weights of the granules or by compressive stress from the reaction vessel.

In the process of fusing of the surface of granules to bond the surface of the granules one to another, the surface of the granules is fused, for example, with a solvent such as acetone or dimethyl sulfoxide, to bond the granules one to another.

Examples of the process of fusing the surface of granules one to another with a plasticizer include a method of adding a plasticizer to the inside of the granules of the raw material calcium composition comprising polymer material so that the granules are contacted and thereby fused to each other, and a method of fusing the granules to each other by adding a plasticizer to the surface of the granules and thereby softening the surface of the granules.

[II Method for producing medical calcium carbonate composition: (G) pore integrated-type porous structure]

Next, [12], i.e., a method for producing the medical calcium carbonate composition that satisfies the above described (G), will be described.

The production method comprises the following (G1) and one selected from the group consisting of (D1) to (D10) and (E5) to (E9) as essential processes, and optionally comprises the following (G2) and (G3) and the above described (E10).

<(G1) Mixing process>

"(G1) Mixing process" is a process of mixing raw material calcium composition powder or raw material calcium composition paste, and porogen. In the case of raw material calcium, such as calcium hydroxide, calcium carbonate, or calcium sulfate, which has limited solubility in water, calcium carbonate paste is preferred because fluidity can be secured. On the other hand, in the case of a water soluble calcium compound such as calcium acetate, it is preferred to mix the raw material in a powder form. The raw material calcium composition is not particularly limited and is preferably calcium oxide, calcium hydroxide, or calcium carbonate, particularly preferably calcium hydroxide or calcium carbonate.

Examples of the porogen include, but are not particularly to, sodium chloride, sodium dihydrogen phosphate, and polymer beads, as mentioned above. Since the porogen controls pore diameter, not containing porogen which maximum diameter is 800 $\mu$m or longer is a necessary condition. Porogen which maximum diameter is 50 $\mu$m or longer and 400 $\mu$m or shorter is preferred.

<(G2) Compacting process>

"(G2) Compacting process" is a process of compacting raw material calcium composition powder or raw material calcium composition paste, and porogen. A known compacting method such as uniaxial pressurization including manual pressing, or pressurization to hydrostatic fluid pressure can be used as a compacting method without limitations. In the case of using raw material calcium composition paste, drying is performed, if necessary, after this process. This process is optional because the compacting process may be unnecessary owing to a constant pressure applied in the process of mixing raw material calcium composition paste and porogen.

<(G3) Porogen removal process>

"(G3) Porogen removal process" is a process of removing porogen by dissolving the porogen into a solvent. In the case of producing a medical calcium phosphate porous structure, etc. using a medical calcium carbonate composition as a raw material, porogen is removed by a process such as the immersion of the medical calcium carbonate composition in disodium hydrogen phosphate. Therefore, the porogen is not necessarily required to be removed from an in-process medical calcium carbonate composition. Thus, the (G3) process is optional.

In this production method, a polymer material may be used as porogen. In this respect, "(E10) a process of structure finishing process after debindering and carbonation processes" in the production of carbonate apatite honeycomb structures using a polymer material may be useful. Thus, the (E10) process is also optional.

For satisfying the (G) condition, the volume of 10 $\mu$m or smaller pores in the pore integrated-type porous structure analyzed by mercury intrusion porosimetry needs to be 0.05 cm$^3$/g or more. The pore volume is adjusted by the mixing ratio between the raw material calcium composition powder and a solvent in (G1) Mixing process, and compacting pressure in (G2) Compacting process.

[II Method for producing medical calcium carbonate composition: debindering condition]

Next, [13] will be described.
In a method for producing the medical calcium carbonate composition using a polymer material, the polymer material used needs to be removed. This process is called debindering. Specifically, the polymer material is removed by the depolymerization, vaporization, or the like of the polymer material. In the present invention, it is preferred to adjust the debindering rate of the polymer material so as not to increase the distance between calcium composition powders because the debindering of the polymer material in the production of a medical calcium carbonate honeycomb structure, etc. increases the distance between calcium composition powders in a polymer material containing-calcium composition. When the debindering temperature is lower than 200°C, the polymer material remains in a relatively large amount. Because of its high fluidity, the distance between calcium composition powders is relatively not long.
Hence, mass decrease of the polymer material of polymer material containing-calcium composition is preferably smaller than 1 mass%/min in a debindering process at 200°C or higher. However, the temperature at which the mass decrease in the polymer material containing-calcium composition is smaller than 1 mass%/min is preferably 150°C or higher, more preferably 100°C or higher, further preferably 50°C or higher.
The mass decrease of the polymer material is more preferably smaller than 0.9 mass%/min, further preferably smaller than 0.8 mass%/min.
The mass decrease rate of the polymer material is not constant with respect to temperature elevation, and the mass decrease occurs sharply at a specific temperature. For example, the depolymerization of acrylic resin occurs sharply at 250°C. Hence, the debindering condition can be optimized by thermal mass measurement. Although heating rate can basically be adjusted depending on a differential value of mass decrease in the thermal mass measurement, a temperature lower by approximately 20°C than the temperature at which sharp mass decrease starts may be maintained.

[II Method for producing medical calcium carbonate composition: specific production method]

Next, [14] will be described.
In a process of producing the medical calcium carbonate composition, the control of carbon dioxide and oxygen may be preferred.
"(L) A process of debindering done at an oxygen partial pressure of 30 KPa or higher" may be preferred for a debindering process. A polymer material is debindered by depolymerization, vaporization, thermal decomposition, or incineration based on heat treatment. Higher oxygen partial pressure may be more preferred because it may facilitate debindering and decreases the amount of remaining materials after acid dissolution. The oxygen partial pressure is approximately 20 KPa in air and is preferably 30 KPa or higher, more preferably 60 KPa or higher, further preferably 90 KPa or higher.
"(M) A process of debindering or carbonation done at carbon dioxide partial pressure of 30 KPa or higher" may be preferred for a debindering or carbonation process. This is because carbon dioxide is necessary for the carbonation of a raw material calcium composition; and debindering does not require the incineration of a polymer material, and the polymer material may be debindered by mere depolymerization, vaporization, or thermal decomposition so that remaining materials after acid decomposition are 1 mass% or less. Calcium carbonate is thermally decomposed into calcium oxide from approximately 340°C at carbon dioxide partial pressure of 0 KPa. Hence, a process of debindering or debindering and carbonation done at carbon dioxide partial pressure above a certain level may be preferred. The amount of carbon dioxide in the atmosphere is limited. For efficiently performing carbonation, the carbon dioxide partial pressure in the carbonation process is preferably 30 KPa or higher, more preferably 60 KPa or higher, further preferably 90 KPa or higher. Debindering done in environment equivalent to pure carbon dioxide environment, i.e., at carbon dioxide partial pressure of 101.3 KPa, may be preferred.
"(N) A process of debindering or carbonation done at 150 KPa or higher under atmosphere that contains oxygen or carbon dioxide" may be preferred for a debindering process and a carbonation process.
In a process of producing the medical calcium carbonate composition, debindering or carbonation may be performed. A raw material calcium composition is carbonated into a medical calcium carbonate composition by exposing the raw material calcium composition to carbon dioxide, for example, by flowing carbon dioxide. In the case of flowing carbon dioxide, the carbon dioxide is mostly discarded without being used in production. A problem of the carbonation of a porous structure of a raw material calcium composition or a compact of a raw material calcium composition is that carbon dioxide is difficult to introduce to the inside of the porous structure or the inside of the compact.
In such a case, it is preferred to create substantially a closed system, and to pressurize the inside of the closed system. Theoretically, such pressurization allows oxygen or carbon dioxide to be infiltrated to the inside of the porous structure or the compact so that the raw material calcium composition is exposed to oxygen or carbon dioxide. However, a process of debindering or carbonation done at 150 KPa or higher under atmosphere that contains oxygen or carbon dioxide is preferred from the viewpoint of efficiency. The pressure of atmosphere is preferably 150 KPa or higher, more preferably 200 KPa or higher, further preferably 300 KPa or higher. Theoretically, there is no upper limit on the pressure of atmosphere. The pressure of atmosphere is preferably 2 MPa or lower, more preferably 1 MPa or lower, further preferably 500 KPa or lower, because a pressurized and closed reaction apparatus is necessary.

For maintaining a pressurized state, a reaction apparatus needs to be substantially a closed system. However, an open system is also possible while pressurization is maintained transiently or continuously for the purpose of, for example, discharging the debindered polymer material component from the reaction apparatus.

"(O) A process of increasing carbon dioxide concentration in the reaction vessel by replacing air in the reaction vessel partially or completely with carbon dioxide, followed by introduction of carbon dioxide in the reaction vessel" may be useful for a carbonation process. This is because carbonation rate is accelerated by increasing carbon dioxide partial pressure.

A method for replacing air in the reaction vessel with carbon dioxide by introducing carbon dioxide from one port of the reaction vessel, and discharging the atmosphere from the other port is convenient and effective.

The degree of replacement of air with carbon dioxide is elevated by decreasing the amount of gas contained in the raw material calcium composition by a pressure reduction process, followed by introduction of carbon dioxide in the reaction vessel. Specifically, for producing a medical calcium carbonate composition, such as a medical calcium carbonate block, which contains no raw material calcium composition from a raw material calcium composition such as a calcium hydroxide compact, it is necessary to introduce carbon dioxide to the inside of the compact, etc. Diffusion alone may be time-consuming or may fail to carbonate the inside. Carbon dioxide can be introduced to the inside of the calcium hydroxide compact by decreasing the amount of air, etc. inside the calcium hydroxide compact by a pressure reduction process, followed by introduction of gas having higher carbon dioxide concentration than that of air in the reaction vessel.

For the pressure reduction of the reaction vessel, the pressure of the reaction vessel can be reduced to below 101.3 KPa which is atmospheric pressure, and is set to preferably 90 KPa or lower, more preferably 60 KPa or lower, further preferably 30 KPa or lower. It is necessary to decrease the amount of air, etc. inside the calcium hydroxide compact by a pressure reduction process, followed by introduction of gas having higher carbon dioxide concentration than that of air in the reaction vessel. The carbon dioxide concentration of the gas to be introduced can be higher than that of air in principle, and is preferably 10 vol% or more, preferably 50 vol% or more, more preferably 90 vol% or more. Substantially pure carbon dioxide is ideal. The pressure of the reaction vessel when carbon dioxide is introduced in the reaction vessel is not particularly limited. However, the pressure of the reaction vessel is preferably equal to or higher than 101.3 KPa which is atmospheric pressure, more preferably 150 KPa or higher, further preferably 200 KPa or higher, by the introduction of carbon dioxide from the viewpoint of increasing the reaction rate of the raw material calcium composition.

"(P) A process of supplying carbon dioxide so that the pressure of the closed reaction vessel is a constant value" is useful for a process of adding carbon dioxide to a medical calcium composition in the closed reaction vessel. The "closed reaction vessel" described in the present invention is, as mentioned above, a reaction vessel that is not an open system. In general, carbonation using the closed reaction vessel increases cost. The medical calcium carbonate composition of the present invention is a medical material, and its production in the closed system may be preferred from the viewpoint of preventing contamination by foreign materials.

Carbon dioxide or carbonate ion is necessary for adding carbonate group to a raw material calcium composition except for the case of producing a medical calcium carbonate composition from raw material calcium carbonate. For example, the production of 1 mol of calcium carbonate by adding carbon dioxide to 1 mol of calcium hydroxide requires 1 mol of carbon dioxide, and 1 mol of carbon dioxide is approximately 22.4 L in a normal state.

Thus, the production of 1 mol of a medical calcium carbonate composition requires a relatively large reaction vessel capable of containing at least 22.4 L of carbon dioxide in a normal state. On the other hand, production in a relatively small reaction vessel is possible by supplying carbon dioxide so that the pressure of the closed reaction vessel is a constant value. The pressure value is not particularly limited. The pressure in the reaction vessel is preferably larger than atmospheric pressure because a convenient process involves supplying carbon dioxide into the reaction vessel from a carbon dioxide tank using a pressure reducing valve so that the pressure in the reaction vessel is constant. The goal is the supply of carbon dioxide into the reaction vessel, and pressure larger than necessary in the reaction vessel requires an expensive reaction vessel and merely complicates operations. Hence, the pressure of carbon dioxide in the reaction vessel is preferably atmospheric pressure as well as 0.5 MPa or lower, more preferably 0.3 MPa or lower, further preferably 0.2 MPa or lower.

This process is useful not only for carbonation under gas phase but also for carbonation under liquid phase as described above in (D9). Since gas other than carbon dioxide is not consumed in the closed system, carbon dioxide concentration in the reaction vessel is low in this process alone. Hence, a process including this process and the "O" process is desirable.

"(Q) A carbonation process of mixing or circulating carbon dioxide in the reaction vessel" may be useful for a process of adding carbon dioxide to a medical calcium composition in the reaction vessel and may be useful, particularly, in the case of using the closed reaction vessel.

The process is also useful in "(D8)", which is a process in a method for producing the medical calcium carbonate composition that satisfies the above described "(D)", and may be useful for a method for producing the medical calcium carbonate composition of the present invention, without being limited by the method for producing the medical calcium carbonate composition that satisfies the above described "(D)".

[II Method for producing medical calcium carbonate composition: specific raw material calcium composition]

Next, [15] will be described. Compositions containing calcium are widely used as raw material calcium compositions. The composition of the raw material calcium composition is preferably one selected from the group consisting of calcium oxide, calcium hydroxide, and calcium carbonate. This is because any composition other than water is not secondarily produced in, for example, a process of producing a medical calcium carbonate composition by adding carbonate component or the like to the raw material calcium composition.

[II Method for producing medical calcium carbonate composition: specific raw material calcium composition]

Next, [16] will be described. [16] relates to, particularly, a method for producing the calcium carbonate composition according to [3] and is a production method that satisfies at least one condition selected from

(R1) Using calcium carbonate powder with an average particle diameter of 2 $\mu$m and larger, and 8 $\mu$m and smaller;
(R2) Using calcium carbonate powder with a sphericity of 0.9 or higher;
(R3) Using calcium carbon powder containing $5 \times 10^{-4}$ mass% or larger, and $3 \times 10^{-3}$ mass% or smaller of Mg;
(R4) Using calcium carbon powder containing $3 \times 10^{-3}$ mass% or larger, and $1.5 \times 10^{-2}$ mass% or smaller of Sr.

All of these conditions are for enhancing the reactivity of the medical calcium carbonate composition to be produced by controlling micropores. Preferred micropores are formed by using a specific raw material calcium powder.

(R1) relates to specific average particle diameter of calcium carbonate powder serving as a raw material calcium composition. The average particle diameter is preferably 2 $\mu$m or larger and 8 $\mu$m or smaller, more preferably 3 $\mu$m or larger and 7 $\mu$m or smaller, further preferably 4 $\mu$m or larger and 6 $\mu$m or smaller.

The sphericity of (R2) is preferably 0.9 or higher, more preferably 0.95 or higher.

The Mg content of (R3) is preferably $5 \times 10^{-4}$ mass% or larger and $3 \times 10^{-3}$ mass% or smaller, more preferably $1 \times 10^{-3}$ mass% or larger and $2.5 \times 10^{-3}$ mass% or smaller, further preferably $1.5 \times 10^{-3}$ mass% or larger and $2.5 \times 10^{-3}$ mass% or smaller.

The Sr content of (R4) is preferably $3 \times 10^{-3}$ mass% or larger and $1.5 \times 10^{-2}$ mass% or smaller, more preferably $4 \times 10^{-3}$ mass% or larger and $1.3 \times 10^{-2}$ mass% or smaller, further preferably $5 \times 10^{-3}$ mass% or larger and $1 \times 10^{-2}$ mass% or smaller.

It is preferred that any one, more preferably two or more, further preferably all, of the conditions (R1) to (R4) should be satisfied.

[III Medical calcium sulfate setting composition]

Next, [17], i.e., a medical calcium sulfate setting composition which may be used as a raw material for medical calcium carbonate compositions, will be described.

A medical calcium sulfate setting composition that satisfies all the following (T1) to (T5) conditions exhibits setting properties and is therefore a useful medical material.

(T1) The remaining materials after acid dissolution is 1.0 mass% or less;
(T2) The volume is $5 \times 10^{-13}$ m$^3$ or larger;
(T3) it is substantially a pure calcium sulfate as a medical composition;
(T4) content of calcium sulfate hemihydrate is 50 mass% or more;
(T5) Forming a porous structure with compressive strength of 0.3 MPa or higher upon setting reaction when compositions contacted one another are immersed in water.

Calcium sulfate hemihydrate powders have been known to set so far. However, it has not been known that calcium sulfate granules wherein the remaining materials after acid dissolution is 1.0 mass% or less; the volume is $5 \times 10^{-13}$ m$^3$ or larger; it is substantially a pure calcium sulfate as a medical composition, form a porous structure with compressive strength of 0.3 MPa or higher upon setting reaction.

The granules containing 50 mass% or larger of calcium sulfate hemihydrate become calcium sulfate dihydrate partially or completely when kneaded with water, and are bonded to each other by the cross-linking of deposited calcium sulfate dihydrate crystals to make a porous structure.

Even a volume of less than $5 \times 10^{-13}$ m$^3$ is useful because a porous structure can be formed upon setting reaction. However, since this usefulness is limited, the volume needs to be $5 \times 10^{-13}$ m$^3$ or larger. The volume is preferably $5 \times 10^{-13}$ m$^3$ or larger and $1 \times 10^{-9}$ m$^3$ or smaller, more preferably $4 \times 10^{-12}$ m$^3$ or larger and $5 \times 10^{-10}$ m$^3$ or smaller, further preferably $1.4 \times 10^{-11}$ m$^3$ or larger and $1.1 \times 10^{-10}$ m$^3$ or smaller, from the viewpoint of the formation of porous structures useful in the invasion of tissues, and compressive strength.

50 mass% or larger of calcium sulfate hemihydrate is essential from the viewpoint of setting properties. Since larger content of calcium sulfate hemihydrate gives higher setting properties, the content of calcium sulfate hemihydrate is preferably 70 mass% or larger, more preferably 80 mass% or larger, further preferably 90 mass% or larger.

Although calcium sulfate porous structure formation itself has usefulness, porous structures having small mechanical strength are less useful. An essential condition of the present invention is forming a porous structure with compressive strength of 0.3 MPa or higher upon setting reaction when immersed in water while the compositions are contacted one another. The compressive strength of the setting product is preferably 0.5 MPa or higher, more preferably 1.0 MPa or higher. The compressive strength differs depending on the degree of contact. Therefore, higher compressive strength is regarded as the compressive strength of the composition when different compressive strength values are obtained.

[IV Method for producing medical calcium sulfate composition]

Next, [18] will be described. A method for producing the above described "III Medical calcium sulfate setting composition", comprising the following (U2) and (U3) as essential processes, and optionally comprising (U1) and/or (U4) to produce medical calcium sulfate hemihydrate granules serving as the medical calcium sulfate setting composition, is useful.

"(U1) Polymer debindering process" is a process of debindering polymer material-containing calcium sulfate granules or block by thermal treatment so that the remaining materials after acid dissolution are 1.0 mass% or less.

This process is a process using polymer material-containing calcium sulfate block or granules as a raw material, and comprises debindering the raw material by thermal treatment so that the remaining materials after acid dissolution are 1.0 mass% or less. The remaining materials after acid dissolution are essentially 1.0 mass% or less, preferably 0.5 mass% or smaller, more preferably 0.3 mass% or smaller, further preferably 0.1 mass% or smaller, ideally 0 mass%. The thermal treatment is generally performed at 700°C or higher. Since calcium sulfate becomes anhydrous at 700°C or higher, this process produces a calcium sulfate anhydrous block or calcium sulfate anhydrous granules.

"(U2) Calcium sulfate dihydrate production process" is a process of producing calcium sulfate dihydrate granules or block by adding water to calcium sulfate anhydrous or hemihydrate granules or block produced by the polymer debindering process, or by adding water to calcium sulfate hemihydrate powder, and followed by hardening.

In the case of using polymer material-containing calcium sulfate, calcium sulfate anhydrous granules or block is produced by the polymer debindering process. Therefore, calcium sulfate dihydrate granules or block is produced by adding water.

When it is not necessary to use polymer material-containing calcium sulfate, calcium sulfate dihydrate granules or block is produced by mixing calcium sulfate hemihydrate powder with water, and followed by hardening.

"(U3) Calcium sulfate hemihydrate production process" is a process of producing calcium sulfate hemihydrate granules or block by dehydration of calcium sulfate dihydrate granules or block.

This process comprises producing calcium sulfate hemihydrate granules or block by dehydration of calcium sulfate dihydrate granules or block in gas phase so that calcium sulfate hemihydrate is 50 mass% or larger. In general, dehydration in gas phase, for example, in the atmosphere, is performed by known heat treatment, and the content of calcium sulfate hemihydrate can be easily controlled by optimizing heat treatment time and heat treatment temperature.

"(U4) Granules size adjusting process" is a process of adjusting granules size so that the volume is $5 \times 10^{-13}$ m$^3$ or larger.

This process comprises adjusting granules size so that the volume is $5 \times 10^{-13}$ m$^3$ or larger. This process may be performed at any stage during the whole processes. For example, spherical granules which volume is $5 \times 10^{-13}$ m$^3$ or larger can be prepared by mixing a calcium sulfate powder with a polymer material such as polyvinyl alcohol, and spray drying the mixture.

Alternatively, a calcium sulfate block can be pulverized and sifted.

As mentioned above, the volume needs to be adjusted to $5 \times 10^{-13}$ m$^3$ or larger. The volume is adjusted preferably to $5 \times 10^{-13}$ m$^3$ or larger and $1 \times 10^{-9}$ m$^3$ or smaller, more preferably to $4 \times 10^{-12}$ m$^3$ or larger and $5 \times 10^{-10}$ m$^3$ or smaller, further preferably to $1.4 \times 10^{-11}$ m$^3$ or larger and $1.1 \times 10^{-10}$ m$^3$ or smaller, from the viewpoint of the formation of porous structures useful in the invasion of tissues, and compressive strength.

[V Medical calcium phosphate composition]

Next, [19], i.e., a medical calcium phosphate composition produced from a medical calcium carbonate composition, will be described.

A medical calcium phosphate composition that satisfies all the following (V1) to (V3) conditions, and at least one condition selected from the group consisting of (V4) to (V10), and optionally satisfying (V11) or (V12) is highly useful.

The need of (V1), (V2), (V5), (V6), (V9), and (V10) is the same as that of the above described (A), (B), (F), (G), (J), and (K), respectively.

In "(V3) it is substantially a pure calcium phosphate as medical composition and is one selected from the group consisting of carbonate apatite, apatite containing $HPO_4$ group, tricalcium phosphate, whitlockite, calcium hydrogen phosphate", the condition that "it is substantially a pure calcium phosphate as medical composition" is common subject matter in the medical calcium composition of the present invention. Among others, one selected from the group consisting of carbonate apatite, apatite containing $HPO_4$ group, tricalcium phosphate, whitlockite, calcium hydrogen phosphate is particularly preferred because of its excellent reactivity. These calcium phosphates except for tricalcium phosphate cannot basically be produced by a sintering method and can be produced through dissolution-deposition reaction of compositional transformation using a precursor such as calcium carbonate in aqueous solution. Carbonate apatite is as mentioned in Patent Literature 1. Apatite containing $HPO_4$ group, whitlockite, and calcium hydrogen phosphate having high reactivity can also be produced in aqueous solution, whereas the $HPO_4$ group becomes pyrophosphoric acid by heating and therefore cannot be produced by a sintering method.

The condition that "(V4) it is a honeycomb structure comprising a plurality of through-holes extending in one direction (with the proviso that a honeycomb structure that does not satisfy any of the following condition is excluded: a composition is tricalcium phosphate, wherein a volume of pores which pore diameter is 10 μm or smaller with respect to a mass of the honeycomb structure analyzed by mercury intrusion porosimetry is 0.01 cm³/g or more; and a diameter of the circle that passes through both ends of any one of the through-holes and a center of the through-hole, is 1 cm or longer, and 50 cm or shorter; The surface roughness of the surface of partition wall of honeycomb structure along the through-holes direction in arithmetic average roughness (Ra) is 0.7 μm or larger.)" is similar to the above described condition that "(E) it is a honeycomb structure comprising a plurality of through-holes extending in one direction, wherein a volume of pores which pore diameter is 10 μm or smaller with respect to a mass of the honeycomb structure analyzed by mercury intrusion porosimetry is larger than 0.02 cm³/g". In the case of a composition other than tricalcium phosphate, there is no condition as to pore volume. However, the volume of pores which pore diameter is 10 μm or smaller with respect to a mass of the honeycomb structure analyzed by mercury intrusion porosimetry is preferably larger than 0.02 cm³/g from the viewpoint of the replacement of the medical calcium phosphate composition with bones, and tissue affinity. The volume is more preferably 0.04 cm³/g or more, further preferably 0.08 cm³/g or more.

The condition that "(V7) a volume of pores having a pore diameter of 6 μm or shorter with respect to a volume of pores having a pore diameter of 1 μm or larger and 6 μm or shorter analyzed by mercury intrusion porosimetry is 5 % or more" is similar to the above described "(H)". In the production of the medical calcium phosphate composition using a medical calcium carbonate composition as a raw material, the number of pores having small pore volume is decreased because phosphoric acid component is added. Hence, the volume of pores having a pore diameter of 1 μm or longer and 6 μm or shorter with respect to a volume of pores having a pore diameter of 6 μm or shorter is 5 % or more. The pore volume is more preferably 7% or more, further preferably 10% or more.

The condition that "(V8) a maximum compressive strength obtained at any one direction is higher than a standard compressive strength [S] that is calculated by the following equation (with the proviso that a honeycomb structure comprising a plurality of through-holes extending in one direction, wherein a volume of pores with a pore diameter of 10 μm or smaller with respect to a mass of the honeycomb structure analyzed by mercury intrusion porosimetry is 0.02 cm³/g or smaller is excluded)

$$S = S_0 \times C \times \exp(-b \times P)$$

(wherein So and b are the constant, and So is 500, and b is 0.068, and C is the constant based on the composition; C is 1 for carbonate apatite, apatite containing $HPO_4$, tricalcium phosphate, and C is 0.5 for whitlockite, and C is 0.1 for calcium hydrogen phosphate; and P is the percentage of pores in the composition.)" is similar to the above described (I). The constant C is a constant based on composition, not polymorph of calcium carbonate.

The condition that "(V11) Composition is apatite with carbonate content is 10 mass% or larger" is optional. The carbonate content is 10 mass% or larger, which means carbonate apatite. Carbonate apatite is highly useful because vertebrate bones are composed thereof.

The condition that "(V12) Composition is apatite with carbonate content is smaller than 10 mass%" is also optional. Apatite with carbonate content is smaller than 10 mass% becomes carbonate apatite and hydroxyapatite having small carbonate content. Slow bone replacement may be desirable depending on cases. A medical calcium phosphate composition wherein composition is apatite with carbonate content is smaller than 10 mass% is preferred for such cases. Since bone replacement rate is controlled by the amount of carbonate groups in an apatite structure, the carbonate content may be more preferably 6 mass% or smaller, further preferably 3 mass% or smaller. Alternatively, hydroxyapatite containing no carbonate group may be preferred.

[V Medical calcium phosphate composition: specific trace component]

Next, [20] will be described. Calcium phosphate compositions, particularly, apatite, have high adsorption ability. Calcium phosphate produced in aqueous solution has a high specific surface area. These calcium phosphate compositions, when infected, cannot be expected to have tissue affinity. Hence, a calcium phosphate composition containing trace component that prevents infection may be preferred. Silver or silver compound is useful as the trace component.

A medical calcium phosphate composition that satisfies (AG1) or (AG2) is a useful medical calcium phosphate composition from the viewpoint of infection prevention. A medical calcium phosphate composition that satisfies (AG1) or (AG2), and optionally satisfying the following conditions (AG3) to (AG10) may be a more preferred medical calcium phosphate composition.

The need of the requirements for volume in (AG1) and (AG2) is the same as that of (A).

An essential condition is that 0.01 mass% or larger and 3 mass % or lower silver or silver compound is contained in calcium phosphate compound in (AG1), and is that 0.01 mass% or larger and 3 mass% or smaller silver phosphate is contained therein in (AG2). Although silver may be taken up into calcium phosphate crystal structures, silver exerts antimicrobial properties, typically, as silver ion. Therefore, silver or silver compound that is not contained in calcium phosphate crystal structures is effective for preventing postoperative infection. The content of silver or silver compound is very important. Small content does not exert an antimicrobial effect, and large content has an adverse effect on tissue affinity. Hence, the medical calcium phosphate composition needs to contain 0.01 mass% or larger and 3 mass% or smaller silver or silver compound. The content is preferably 0.02 mass% or larger and 2 mass% or smaller, more preferably 0.03 mass% or larger and 1 mass% or smaller, further preferably 0.05 mass% or larger and 1 mass% or smaller. It is also preferred to secure such content except for the content of silver in calcium phosphate crystal structures.

The condition that composition is "selected from the group consisting of carbonate apatite, apatite containing $HPO_4$, whitlockite, and calcium hydrogen phosphate" in (AG1) is a condition as to calcium phosphate which cannot be produced by a sintering method and is produced in aqueous solution. Any calcium phosphate composition has not been known so far in which calcium phosphate compound which cannot be produced by a sintering method is allowed to contain silver or silver compound.

In (AG2), composition of calcium phosphate also includes sintered hydroxyapatite and sintered tricalcium phosphate which can be produced by a sintering method, and an essential condition is silver phosphate crystals bonded on the surface of calcium phosphate composition. The silver phosphate crystals bonded on the surface of calcium phosphate composition have a high antimicrobial effect because silver phosphate is dissolved from the surface of the composition. Since the dissolution of silver phosphate occurs from the surface that is not bonded to the calcium phosphate composition, the resulting calcium phosphate composition exhibits a relatively long-term antimicrobial function. The area ratio of the silver phosphate crystals bonded on the surface of calcium phosphate composition is preferably 20% or more, more preferably 30% or more, further preferably 40% or more, with respect to the surface area of the silver phosphate crystals. The area of silver phosphate bonded to calcium phosphate is preferably $2 \times 10^{-12}$ m$^2$ or larger, more preferably $6 \times 10^{-12}$ m$^2$ or larger, further preferably $1 \times 10^{-11}$ m$^2$ or larger.

The (AG3) to (AG10) conditions are optional and are not necessarily required to be satisfied. The condition (AG3) is optional for (AG1). Since silver phosphate having small solubility can be expected to have a long-term antimicrobial effect, other silver compounds may be more preferred.

The condition that "(AG4) Silver or silver compound is contained at the surface and inside the calcium phosphate composition, and ratio of the silver content at the surface by that at least 50 $\mu$m distant to interior direction is 1.2 or higher" is optional for a specific calcium phosphate composition having different silver concentrations inside the composition. Postoperative infection includes, for example, early infection which occurs shortly after operation, and slow infection which occurs, for example, after a lapse of 2 months or more from operation. Prevention of both of them is desirable. A relatively high silver concentration is preferred for an early stage after operation where infection prevention is given high priority as compared with tissue affinity. Hence, for tissue reconstruction with a calcium phosphate composition, it is desirable that a relatively high concentration of silver ion should be released around the composition. This can be achieved by a high concentration of silver or silver phosphate at the surface of the composition.

In the case of bioresorbable calcium phosphate such as carbonate apatite or tricalcium phosphate, silver or silver compound at the surface disappears upon resorption of the surface. Since the risk of infection is reduced after a lapse of a given postoperative period, the prevention of slow infection is necessary, though the priority of tissue affinity is increased. Thus, it is preferred that silver or silver compound should be contained both at the surface and inside the calcium phosphate composition, and the ratio of the silver content at the surface by that at least 50 $\mu$m distant to interior direction is preferably 1.2 or higher in the composition. The ratio is more preferably 2 or higher, further preferably 3 or higher.

The surface and the inside of the composition are essentially a surface that is resorbed by osteoclasts, and the inside except for the surface. In the case of a porous structure having 30 $\mu$m or larger interconnected pores that can be invaded by osteoclasts, a site except for not only apparent surface but also the surface of a site having no 30 $\mu$m or larger interconnected pores is regarded as the inside. The ratio of the concentration of silver or silver compound between the surface and the inside is quantified by analyzing the surface and the inside using an energy dispersive X-ray spectroscopy apparatus, an X-ray photoelectron spectroscopy apparatus, or the like. The surface refers to a site positioned less than 50 $\mu$m from a superficial aspect, and the inside refers to a site at least 50 $\mu$m distant from the superficial aspect. If silver concentration measurement at the surface and the inside is difficult by the above described measurement method, the calcium phosphate composition is subjected to the dissolution rate test stipulated by 9.3 of JIS-T0330-3 using aqueous solution of pH 5.5, and the concentration of silver component contained in the surface, which is calculated from the amount of silver component dissolved until the same weight decrease as in the case where 50 $\mu$m of the surface is dissolved, and the concentration of silver component contained in a sample are used to calculate the ratio therebetween.

The (AG5) to (AG10) conditions are optional for a specific calcium phosphate porous structure. Porous structures are predisposed to the focus of infection as compared with precise bodies, and these porous structures are useful as medical calcium phosphate compositions and are therefore medical calcium phosphate compositions for which infection prevention is desirable.

[V Medical calcium phosphate composition: specific honeycomb structure and specific fine structure, and composition]

Next, [21], i.e., a particularly useful composition among medical calcium phosphate compositions, will be described.

Medical calcium phosphate compositions are required to have high reactivity or characteristics appropriate for cases, as in medical calcium carbonate compositions. Hence, it is preferred to satisfy at least one condition selected from the following (W1) to (W7).

"(W1) A honeycomb structure comprising a plurality of through-holes extending in one direction, wherein ratio of pore volume with pore diameter 10 $\mu$m or smaller against mass of the honeycomb structure analyzed by mercury intrusion porosimetry is 0.01 cm$^3$/g or more" relates to micropores in the bone trabeculae. Composition is a factor involved in bone conduction or bone replacement, and macropores or micropores largely influence the usefulness of medical compositions. Specific micropores present in a specific amount influence useful properties such as the promotion of resorbability by osteoclasts. The pore volume with pore diameter 10 $\mu$m or smaller against mass of the honeycomb structure is preferably 0.01 cm$^3$/g or more, more preferably 0.03 cm$^3$/g or more, further preferably 0.05 cm$^3$/g or more.

"(W2) A honeycomb structure comprising a plurality of through-holes extending in one direction, wherein a diameter of the circle that passes through both ends of any one of the through-holes and the center of the through-hole, is 1 cm or longer, and 50 cm or shorter" is a honeycomb structure useful for specific cases. As mentioned above, bones include straight cylindrical bones as well as bended and curved cylindrical bones. A medical calcium phosphate honeycomb structure that is curved cylindrical in shape is useful for the reconstruction of such curved cylindrical bones. In the case of constructing a bone in a direction vertical to the surface of a bone, it is desirable for a honeycomb structure that the through-holes of the honeycomb structure should open only on the bone surface without opening on the connective tissue around the bone.

A preferred condition of the honeycomb structure is that a diameter of the circle that passes through both ends of any one of the through-holes and a center of the through-hole is 1 cm or longer, and 50 cm or shorter. The diameter of the circle is more preferably 2 cm or longer and 20 cm or shorter. The diameter of the circle is further preferably 3 cm or longer and 10 cm or shorter.

"(W3) A honeycomb structure with surface roughness of the surface of partition wall of honeycomb structure along the through-holes direction in arithmetic average roughness (Ra) is 0.7 $\mu$m or larger" is a honeycomb structure effective for cell adhesion, etc. In this context, the arithmetic average roughness (Ra) of the surface of partition wall of honeycomb structure along the through-holes direction is the arithmetic average roughness of the honeycomb surface independent of the partition wall. Larger arithmetic average roughness (Ra) in medical carbonate apatite honeycomb structures improves cell adhesion, etc. and as a result, also enhances osteoconductivity. The arithmetic average roughness (Ra) is more preferably 1.0 $\mu$m or larger, further preferably 1.5 $\mu$m or larger.

(W4) to (W7) correspond to (AJ1) to (AJ4) of [3], and the medical calcium phosphate composition that satisfies any of these conditions is excellent in tissue affinity and bone replaceability.

The average diameter of (W4) is preferably 2 $\mu$m or larger and 8 $\mu$m or smaller, more preferably 3 $\mu$m or larger and 7 $\mu$m or smaller, further preferably 4 $\mu$m or larger and 6 $\mu$m or smaller.

The sphericity of (W5) is preferably 0.9 or larger, more preferably 0.95 or larger.

The Mg content of (W6) is preferably $5 \times 10^{-4}$ mass% or larger and $3 \times 10^{-3}$ mass% or smaller, more preferably $1 \times 10^{-3}$ mass% or larger and $2.5 \times 10^{-3}$ mass% or smaller, further preferably $1.5 \times 10^{-3}$ mass% or larger and $2.5 \times 10^{-3}$ mass% or smaller.

The Sr content of (W7) is preferably $3 \times 10^{-3}$ mass% or larger and $1.5 \times 10^{-2}$ mass% or smaller, more preferably $4 \times 10^{-3}$ mass% or larger and $1.3 \times 10^{-2}$ mass% or smaller, further preferably $5 \times 10^{-3}$ mass% or larger and $1 \times 10^{-2}$ mass% or smaller.

The average diameter and the sphericity are measured and calculated by dividing particles at the grain boundary of the calcium phosphate composition.

It is preferred that any one, more preferably two or more, further preferably all, of the conditions (W1) to (W7) should be satisfied.

[VI Method for producing medical calcium phosphate composition: specific trace component]

Next, a method for producing a medical calcium phosphate composition will be described.

First, [22] will be described. The calcium phosphate composition according to [20], [21], etc. containing silver or silver phosphate can be produced under the (AH1) or (AH2) condition. A more preferred composition can be produced under a specific condition where any of (AH3) to (AH9) can be added, if necessary, as a selection condition.

The condition of "being granules or block with volume of $10^{-12}$ m$^3$ or larger", which is common in (AH1) and (AH2), is necessary for producing calcium phosphate compositions excellent in tissue affinity.

(AH1) relates to a method for producing a calcium phosphate composition which cannot be produced by a sintering method. By "using raw material calcium composition, containing 0.01 mass% or larger, and 3 mass% or smaller silver or silver composition, that is one selected from the group consisting of calcium carbonate, calcium hydroxide, calcium oxide, calcium sulfate, calcium hydrogen phosphate, and is a granule or block with volume of $10^{-12}$ m$^3$ or larger", the raw material calcium is subjected to "a process of adding carbonate group to the raw composition when the raw material calcium composition is not calcium carbonate" for the compositional transformation of the calcium composition to calcium carbonate. In the case of, for example, calcium hydroxide, the calcium hydroxide is exposed to carbon dioxide.

This process further comprises "a process of compositional transformation reaction to any one selected from the group consisting of silver or silver compound containing carbonate apatite, apatite with HPO$_4$ group, whitlockite, and calcium hydrogen phosphate by exposure to aqueous phosphoric acid salt solution or mixed aqueous solution of phosphoric acid salt and magnesium salt". The exposure to aqueous phosphoric acid salt solution or mixed aqueous solution of phosphoric acid salt and magnesium salt may be performed by simple immersion in the aqueous solution or may be performed by the spray exposure of the aqueous solution to the raw material calcium composition by spraying or the like. A calcium phosphate composition can be produced by the exposure process. The carbonation process and the process of exposure to aqueous phosphoric acid salt solution or mixed aqueous solution of phosphoric acid salt and magnesium salt may be performed at the same time.

(AH2) is a process of immersing the raw material calcium composition selected from the group consisting of apatite, tricalcium phosphate, whitlockite, octacalcium phosphate, calcium hydrogen phosphate, which are calcium phosphate compositions, in aqueous solution containing silver ion, leading formation of silver phosphate to raw material calcium composition, is included. For example, when tricalcium phosphate is immersed in aqueous silver nitrate solution, the tricalcium phosphate is partially dissolved to release phosphoric acid ion and calcium ion. Since the solubility of silver phosphate formed from the silver ion and the phosphoric acid ion is smaller than that of tricalcium phosphate, silver phosphate is deposited on the surface of the tricalcium phosphate composition. In the case of tricalcium phosphate having a porous structure so that aqueous silver nitrate solution infiltrates into the porous structure, silver phosphate is deposited on the tricalcium phosphate surface contacted with the aqueous silver nitrate solution. The type or concentration of the aqueous solution containing silver ion, immersion time, etc. is not particularly limited, and silver nitrate or silver carbonate is preferred in light of solubility.

It is essential to satisfy the (AG1) or (AG2) condition, and a more preferred medical calcium phosphate composition may be produced by further satisfying any of (AG3) to (AG10).

(AH3) is a production process related to (AG4) of [20]. It is possible to produce a calcium phosphate composition having different silver concentrations between the inside and the surface by adjusting the silver ion concentration of the aqueous solution, immersion time, etc. However, use of aqueous solutions differing in concentrated silver ion shortens a production time. In most cases, a raw material calcium phosphate composition is immersed in first aqueous solution to deposit a relatively low concentration of silver phosphate inside the composition, and then immersed in second aqueous solution to deposit a relatively high concentration of silver phosphate on the surface. Therefore, the second aqueous solution needs to contain higher concentrated silver ion than that of the first aqueous solution.

The (AH4) to (AH9) conditions relate to the raw material calcium composition necessary for producing (AG5) to (AG10) of [20].

[VI Method for producing medical calcium phosphate composition: specific raw material]

Next, [23] will be described. [23] relates to the medical calcium phosphate composition according to any one of [19] to [21] and, particularly, relates to (W4) to (W7) of [21]. The medical calcium phosphate composition that satisfies the (W4) to (W7) conditions is achieved by using a calcium carbonate powder that satisfies the (AI1) to (AI4) conditions as raw material calcium.

The mean particle diameter of (AI1) is preferably 2 $\mu$m or larger and 8 $\mu$m or smaller, more preferably 3 $\mu$m or larger and 7 $\mu$m or smaller, further preferably 4 $\mu$m or larger and 6 $\mu$m or smaller.

The sphericity of (AI2) is preferably 0.9 or larger, more preferably 0.95 or larger.

The Mg content of (AI3) is preferably $5 \times 10^{-4}$ mass% or larger and $3 \times 10^{-3}$ mass% or smaller, more preferably $1 \times 10^{-3}$ mass% or larger and $2.5 \times 10^{-3}$ mass% or smaller, further preferably $1.5 \times 10^{-3}$ mass% or larger and $2.5 \times 10^{-3}$ mass% or smaller.

The Sr content of (AI4) is preferably $3 \times 10^{-3}$ mass% or larger and $1.5 \times 10^{-2}$ mass% or smaller, more preferably $4 \times 10^{-3}$ mass% or larger and $1.3 \times 10^{-2}$ mass% or smaller, further preferably $5 \times 10^{-3}$ mass% or larger and $1 \times 10^{-2}$ mass% or smaller.

It is preferred that any one, more preferably two or more, further preferably all, of the conditions (AI1) to (AI4) should be satisfied.

Next, [24] will be described. A method for producing the medical calcium phosphate composition, comprising adding phosphoric acid component to the medical calcium carbonate composition of the present invention, or a medical calcium carbonate composition produced by the method for producing the medical calcium carbonate composition of the present invention, under a specific condition is useful.

The addition method is preferably a method of immersing the medical calcium carbonate composition in aqueous solution containing phosphoric acid component, such as aqueous phosphoric acid salt solution.

The type of calcium phosphate to be formed by the addition of phosphoric acid component to the calcium carbonate composition can be controlled by pH of solution, coexisting ion, etc. A medical calcium phosphate composition comprising carbonate apatite, hydroxyapatite, tricalcium phosphate, whitlockite, octacalcium phosphate, calcium hydrogen phosphate, calcium dihydrogen phosphate, or the like can be produced. For the production of such a medical calcium phosphate composition, a medical carbonate apatite composition containing carbonate groups is particularly useful because medical calcium carbonate comprises carbonate groups.

The amount of carbonate groups in the medical carbonate apatite composition can be controlled by pH. $Na_2HPO_4$ of pH 8.9 or $Na_3PO_4$ of pH 13.1 per mol/L has been used so far. However, it has been found that the amount of carbonate groups in the medical carbonate apatite composition can be controlled by setting pH to lower than 8.9. The medical carbonate apatite composition produced at pH lower than 8.9 can have a smaller carbonate content than that of previously produced carbonate apatite. However, pH 8.8 has a limited effect. Hence, in the present invention, aqueous solution containing phosphoric acid component with pH 8.5 or higher, and aqueous solution containing phosphoric acid component with pH lower than 8.5 are distinguished from each other.

Furthermore, it has been found that, surprisingly, lower pH of aqueous phosphoric acid salt solution accelerates the dissolution-deposition reaction of calcium carbonate into calcium phosphate and can shorten the production time of calcium phosphate. This is presumably because low pH in the dissolution-deposition reaction of calcium carbonate into calcium phosphate accelerates the dissolution reaction, though the details thereof have not yet been elucidated.

pH 5.5 or lower permits compositional transformation of calcium carbonate into calcium hydrogen phosphate, and tricalcium phosphate such as whitlockite can be produced by using aqueous solution containing both phosphoric acid component and magnesium component.

Although lower pH of phosphoric acid salt shortens the production time of calcium phosphate, the amount of carbonate groups is also decreased in the production of carbonate apatite. In order to shorten the production time of carbonate apatite without decreasing the amount of carbonate groups, carbonate component can be contained in phosphoric acid salt of low pH. Nonetheless, it has been found that carbonate apatite cannot be produced if the carbonate component concentration is higher than 1.0 mol/L. Also, a carbonate component concentration of 0.6 mol/L has been found to require time for the production of carbonate apatite. This is probably because in the presence of carbonate ion in aqueous solution, carbonate apatite becomes difficult to form due to reduced difference in the degree of supersaturation between calcium carbonate and carbonate apatite, though the details thereof have not yet been elucidated.

Specifically, aqueous solution containing both phosphoric acid component and carbonate component at a concentration of 0.5 mol/L or lower of pH lower than pH 8.5 is useful for this purpose.

Furthermore, it has been found that arithmetic average roughness (Ra) is increased by adding phosphoric acid component to the medical calcium carbonate composition using aqueous solution containing both phosphoric acid component and carbonate component at a concentration of 0.5 mol/L or lower. This is probably because carbonate groups contained in solution reduce the difference in the degree of supersaturation between calcium carbonate and carbonate apatite, resulting in limited core formation, though this mechanism has not yet been elucidated. As a result, the arithmetic average roughness (Ra) of the honeycomb structure is probably increased by the promoted growth of the formed carbonate apatite.

In light of combinations of these, at least one aqueous solution selected from the group consisting of

(X1) Aqueous solution containing phosphoric acid component with pH 8.5 or higher;
(X2) Aqueous solution containing phosphoric acid component with pH lower than 8.5;
(X3) Aqueous solution containing both phosphoric acid component and carbonate component at a concentration of 0.5 mol/L or lower of pH 8.5 or higher;
(X4) Aqueous solution containing both phosphoric acid component and carbonate component at a concentration of 0.5 mol/L or lower of pH lower than pH 8.5;
(X5) Aqueous solution containing both phosphoric acid component and magnesium component

is useful as the aqueous solution for adding phosphoric acid component to the medical calcium carbonate composition.

[VI Method for producing medical calcium phosphate composition: replacement method for gas inside composition]

Next, [25] will be described. As mentioned above, the medical calcium phosphate composition is produced by exposing the medical calcium carbonate composition to aqueous phosphoric acid salt solution, etc. by immersion or the like. As mentioned above, the method for producing the medical calcium phosphate composition from the medical calcium carbonate composition employs dissolution-deposition reaction. Thus, the medical calcium carbonate composition needs to be dissolved from the surface. Basically, the medical calcium carbonate composition can be immersed in aqueous phosphoric acid salt solution, etc. The medical calcium carbonate composition basically has internal pores which are macropores or micropores. Hence, the dissolution reaction may be inhibited by gas such as air in the internal pores. In this case, unreacted calcium carbonate remains in the resulting calcium phosphate composition, which may be unfavorable as the medical calcium phosphate composition. In order to prevent the inhibition of the reaction, gas in the pore of medical calcium carbonate composition can be replaced with aqueous solution containing phosphoric acid component.

Specifically, "(Y1) a process of partial or complete replacement of gas in the pore of medical calcium carbonate composition immersed in the aqueous solution containing phosphoric acid component, to aqueous solution containing phosphoric acid component" can be performed.

There are some useful processes for the partial or complete replacement of gas in the pore of medical calcium carbonate composition immersed in the aqueous solution containing phosphoric acid component, to aqueous solution containing phosphoric acid component. Since the medical calcium carbonate composition is basically a material that is wetted with aqueous solution containing phosphoric acid component, "(Y2) a process of partial or complete replacement of gas in the pore of medical calcium carbonate composition immersed in the aqueous solution containing phosphoric acid component, to aqueous solution containing phosphoric acid component, using vibration" is also effective. For example, an ultrasonic vibrator is effective for using vibration.

(Y3) A process of partial or complete replacement of gas in the pore of medical calcium carbonate composition immersed in the aqueous solution containing phosphoric acid component, to aqueous solution containing phosphoric acid component, by flowing the aqueous solution is also useful as a process similar to the process using vibration. The wetting of the surface of the pore inside the medical calcium phosphate composition with aqueous solution containing phosphoric acid component can be promoted by flowing the aqueous solution containing phosphoric acid component around the medical calcium carbonate composition.

"(Y4) A process of partial or complete replacement of gas in the pore of medical calcium carbonate composition immersed in the aqueous solution containing phosphoric acid component, to aqueous solution containing phosphoric acid component, by degasification" relates to so-called degasification. The amount of gas in the pore of medical calcium carbonate composition is decreased by pressure reduction, and its surrounding aqueous solution containing phosphoric acid component is introduced to the pore of medical calcium carbonate composition by canceling the pressure reduction. A repetition of pressure reduction and its cancelation is preferred from the viewpoint of improving the degree of replacement.

"(Y5) A process of partial or complete replacement of gas in the pore of medical calcium carbonate composition immersed in the aqueous solution containing phosphoric acid component, to a gas that has higher solubility in an aqueous solution containing phosphoric acid component" is also useful. For example, carbon dioxide has higher solubility in aqueous solution containing phosphoric acid component than that of air. When a medical calcium carbonate composition containing carbon dioxide replaced for gas in the pore is immersed in aqueous solution containing phosphoric acid component, air in the pore of medical calcium carbonate composition is replaced with the aqueous solution containing phosphoric acid component because the carbon dioxide is dissolved in the aqueous solution containing phosphoric acid component.

"(Y6) A process of partial or complete replacement of gas in the pore of medical calcium carbonate composition immersed in the aqueous solution containing phosphoric acid component, to a solvent that has smaller contact angle than water and is compatible with water" is also effective. For example, ethanol has smaller contact angle than that of water. Hence, ethanol infiltrates easily into the pore of medical calcium carbonate composition. When a medical calcium carbonate composition having pores partially or completely filled with ethanol is immersed in aqueous solution containing phosphoric acid component, the ethanol is compatibilized with the aqueous solution. The ethanol in the pores is replaced with the aqueous solution containing phosphoric acid component by diffusion. The solvent that has smaller contact angle than water and is compatible with water decreases the contact angle of the aqueous solution containing phosphoric acid component for the medical calcium carbonate composition, as in surfactants. Therefore, complete replacement of gas in the pore of medical calcium carbonate composition is not necessarily required, and only partial replacement functions sufficiently.

[VI Method for producing medical calcium phosphate composition: reaction vessel]

Next, [26] will be described. The medical calcium phosphate composition is produced by adding phosphoric acid component to the medical calcium carbonate composition of the present invention produced by adding carbonate component to the raw material calcium composition. In general, medical materials need to be prevented from contamination by foreign materials, and also from the viewpoint of a convenient production process, it is preferred that a production process should be performed using a same reaction vessel without taking a material out thereof.

The "reaction vessel" is not particularly limited, and a general vessel can be used. A vessel having an inlet and an outlet is preferred. This is because the addition of carbonate component, and the addition of phosphoric acid component need to be performed in the reaction vessel. The addition of carbonate component requires the inlet for introducing carbon dioxide, etc. into the reaction vessel. Also, the outlet is necessary for discharging air in the reaction vessel to the outside of the reaction vessel. Pressure may be applied into the reaction vessel from the inlet, and a solvent, etc. in the reaction vessel can be ejected from the outlet.

The vessel is preferably a column-shaped vessel for use in chromatography, or a reaction vessel capable of accommodating a mesh container that contains the raw material calcium composition or the medical calcium carbonate composition, from the viewpoint of ensuring homogeneous reaction. In the case of a column-shaped vessel, the homogeneous reaction of the composition in the column-shaped vessel is ensured by flowing gas or solution in the vessel. In the case of a reaction vessel capable of accommodating a mesh container, the homogeneous reaction of the composition in the mesh container is ensured by flowing gas or liquid into the mesh.

This process can prevent contamination and produce a medical calcium phosphate composition by a convenient operation. Therefore, "a process of producing a medical calcium phosphate composition wherein the following (Z1) to (Z4), or (Z1), (Z3), (Z4) or (Z1) to (Z3) are successively performed in this order in a same reaction vessel without taking a material out thereof" is a useful method for producing a medical calcium phosphate composition.

"(Z1) A process of producing medical calcium carbonate by adding carbonate component to raw material calcium composition" is an essential process and involves adding carbonate component to raw material calcium composition by exposure to carbon dioxide or carbonate ion. There is also a process of partial carbonation by exposing raw material calcium composition to carbon dioxide or carbonate ion under gas phase, followed by exposing the raw material calcium composition to carbon dioxide or carbonate ion under liquid phase, as in the above described (D9). In such a case, it is essential in principle to successively perform a process following (D9), and (Z2) or (Z3). It is preferred to perform the whole process of the above described (D9).

"(Z2) A process of washing medical calcium carbonate composition" is an optional process. This process is useful, for example, for producing a medical calcium carbonate composition by the carbonation of a raw material calcium composition that contains porogen, followed by washing off the porogen.

In the process of producing a medical calcium carbonate composition by adding carbonate component to raw material calcium composition, it is necessary neither to use a same reaction vessel nor to take a material out thereof for performing partial carbonation and subsequent carbonation. The carbonation subject to the partial carbonation is targeted. However, it is preferred to perform the partial carbonation and the subsequent carbonation using a same reaction vessel without taking a material out thereof.

"(Z3) A process of adding phosphate component to medical calcium carbonate composition" is an essential process. For example, when a medical calcium carbonate composition is immersed in aqueous phosphoric acid salt solution, the phosphoric acid component can be added to the medical calcium carbonate composition to produce a medical calcium phosphate composition.

"(Z4) A process of washing medical calcium carbonate composition" is an optional process. Although the medical calcium phosphate composition is produced by the above described (Z3), this composition contains phosphoric acid salt, etc. This process removes a substance other than the composition. The washing process is necessary for the production of the medical calcium phosphate composition and however, is optional because washing and size adjustment are performed at the same time and therefore, other containers such as a sieve may be used.

[VII Medical calcium hydroxide composition]

Next, [27], i.e., medical calcium hydroxide composition, will be described.

A medical calcium hydroxide composition that satisfies all the (AB1) to (AB3) conditions, and at least one condition selected from the group consisting of (AB4) to (AB8) is useful.

(AB1), (AB2), (AB4), (AB5), (AB6), (AB7), and (AB8) are the same as the above described (A), (B), (V4), (F), (G), (J), and (K), respectively.

The condition that "(AB3) it is substantially a pure calcium hydroxide as medical composition" is common subject matter in the medical calcium composition of the present invention, and the requirement of (AB3) is that composition is calcium hydroxide.

[XIII Method for producing medical calcium hydroxide composition: honeycomb structure]

Next, [28], i.e., a process of producing the medical calcium hydroxide composition that satisfies the above described (AB4) condition, will be described.

A medical calcium hydroxide honeycomb structure is produced by (AD1) and one selected from the group consisting of (AD2) to (AD5) as essential processes and (AD6) to (AD8) as optional processes.

(AD1), (AD6), (AD7), and (AD8) are the same as (E1), (E2), (E3), and (E4), respectively.

"(AD2) Debindering process" is a process of debindering of polymer containing calcium hydroxide porous structure by thermal treatment so that the remaining materials after acid dissolution are 1.0 mass% or less. The debindering needs to be performed under a condition where calcium hydroxide is neither decomposed into calcium oxide nor carbonated into carbonate apatite. For example, a polymer material that has become a monomer by depolymerization or the like under reduced pressure at a temperature that does not decompose calcium hydroxide can be removed.

"(AD3) Hydration process via calcium oxide" is a process of producing calcium hydroxide porous structure by debindering of polymer containing calcium hydroxide porous structure by thermal treatment so that the remaining materials after acid dissolution are 1.0 mass% or less, and to be calcium oxide porous structure, followed by hydration.

"(AD4) Hydration process via calcium carbonate and calcium oxide" is a process of producing calcium hydroxide porous structure by heat treatment of polymer containing calcium hydroxide porous structure in the presence of carbon dioxide, followed by debindering so that the remaining materials after acid dissolution are 1.0 mass% or less, and to be calcium oxide porous structure, followed by hydration.

"(AD5) A production process from calcium carbonate porous structure" is a process of fabricating calcium hydroxide porous structure by debindering polymer containing calcium hydroxide porous structure so that the remaining materials after acid dissolution is 1.0 mass% or less, and to be calcium oxide porous structure, followed by hydration of calcium oxide porous structure.

[XIII Method for producing medical calcium hydroxide composition: granule bonded-porous structure]

Next, [29], i.e., a process of producing the medical calcium hydroxide composition that satisfies the above described (AB5) condition, will be described.

A medical calcium hydroxide granule bonded-porous structure that satisfies the above described (AB5) condition is produced by the following (AE1) and (AE2) and at least one selected from the group consisting of the above described (AD2) to (AD5) as essential processes.

"(AE1) Placement process" is a process of placing polymer containing calcium hydroxide granules with a volume of $10^{-12}$ m$^3$ or more in a reaction vessel.

"(AE2) Granules bonding process" is process of producing granules bonded-porous structure comprising a plurality of through-holes extending in plural directions, formed by bonding a plurality of granules which maximum diameter is 50 $\mu$m or longer and 500 $\mu$m or shorter, and having a volume of $3 \times 10^{-11}$ m$^3$ or larger, based on heat treatment of the granules in the reaction vessel so that the surface is softened and fused one another, or dissolution of the granule surface so that the surface is bonded to each other, or treatment with a plasticizer so that the granule surface is fused one another.

In the heat treatment, the polymer material is softened, and the granules are bonded to each other.

For the dissolution of the surface of the granules, the granules can be exposed to a solvent that dissolves the polymer contained in the granules. When the polymer material is, for example, acrylic resin, the granules can be contacted with acetone or the like.

The plasticizer softens the polymer material. A known plasticizer can be used as the plasticizer without limitations.

[XIII Method for producing medical calcium hydroxide composition: production method using calcium oxide porous structure]

Next, [30], i.e., a process of producing a medical calcium hydroxide porous structure using a calcium hydroxide porous structure or a calcium carbonate porous structure as a raw material, will be described.
The medical calcium hydroxide porous structure can be produced by thermally decomposing a calcium hydroxide porous structure or a calcium carbonate porous structure into a calcium oxide porous structure, followed by hydration of the calcium oxide porous structure.

[IX Method for producing medical calcium composition: placement-closing process or placement process]

Next, [31] will be described. In the placement-closing process or placement process for the production of a granule bonded-porous structure formed from a plurality of granules bonded to each other, the shape of the granules is not particularly limited, and a sphere, a shape having asperities, a crushed material, or the like can be used. Also, a precise body, a porous structure, a hollow body, or the like can be used without limitations.
However, use of granules that satisfy the condition that "(AF1) sphericity of the granules is 0.9 or larger" or "(AF2) the granules are hollow" may be preferred for the placement-closing process or placement process from the viewpoint of improving the penetrability or compressive strength of the medical calcium carbonate porous structure to be produced.
"(AF1) Sphericity of the granules is 0.9 or larger" because use of spheric or nearly spheric granules improves the continuity of the pores to be formed between the granules or spherical granules are easy to contact with each other and therefore, a porous structure having high compressive strength is produced.
The sphericity is preferably 0.9 or larger, more preferably 0.92 or larger, further preferably 0.95 or larger.
In the case of granules that satisfy the condition that "(AF2) the granules are hollow", the granules having a hollow structure are bonded to form a granule bonded-porous structure, which in turn has a double pore shape. The porous structure in such a specific shape may be a particularly excellent porous structure from the viewpoint of the migration or conduction of cells or tissues or bone replacement.
When granules are placed in a reaction vessel, it may be preferred that "(AF3) granules with a bulk volume of 105% or more with respect to the reaction vessel should be placed in the reaction vessel".
In the production of a granule bonded-porous structure comprising a plurality of granules formed by being bonded to each other, and comprising a plurality of through-holes extending in plural directions, a placement-closing process or a placement process of placing granules in a reaction vessel, and closing the opening of the reaction vessel so that the granules are not escaped from the reaction vessel is performed. Following the placement-closing process or placement process, the granules are bonded by swelling or compositional transformation. Porous structure formation requires contacting or bonding the granules with each other by some approach. Increase in contact area among the granules is effective for improvement in the compressive strength of the porous structure. The application of compressive stress to between the granules is effective for the increase in contact area. An effective method for applying compressive stress to between the granules involves a placement-closing process or a placement process of placing, in a reaction vessel, the granules with a bulk volume equal to or more than the volume of the reaction vessel, and, if necessary, closing the opening of the reaction vessel so that the granules are not escaped from the reaction vessel.
In principle, compressive stress is applied to between the granules by placing, in a reaction vessel, the granules with a bulk volume more than 100% with respect to the reaction vessel. The bulk volume of the granules to be placed in the reaction vessel is preferably 105% or more, more preferably 110% or more, further preferably 120% or more, with respect to the reaction vessel from the viewpoint of increasing the compressive stress to be applied to between the granules.

[X Bone defect regeneration kit]

Next, [32], i.e., "a bone defect reconstruction kit comprising a solid portion that contains vaterite and $\alpha$-tricalcium phosphate and a liquid portion that contains phosphoric acid salt, and set to form carbonate apatite when the solid portion and liquid portion are mixed", will be described.
Medical vaterite compositions or medical carbonate apatite block compositions are useful for bone defect reconstruction. However, the forming of blocks into the form of the bone defect may be complicated, and granules may move from the bone defect. Hence, a bone defect reconstruction kit that sets to form carbonate apatite is useful.
When $\alpha$-tricalcium phosphate ($\alpha$TCP) and a liquid portion such as water are mixed, calcium ion and phosphoric acid ion are formed in solution by dissolution. The solution becomes supersaturated with respect to calcium deficient hydroxyapatite, and calcium deficient hydroxyapatite crystals are deposited from the solution to form cured calcium deficient hydroxyapatite by the entanglement of the crystals. However, calcium deficient hydroxyapatite is not bone composition and is inferior in osteoclast resorption to, for example, bone composition carbonate apatite. Calcium ion and phosphoric acid ion formed in the solution by the dissolution of $\alpha$TCP form carbonate apatite crystals, not calcium deficient hydroxyapatite crystals, in the presence of carbonate ion, and cured carbonate apatite is formed by the entanglement of the carbonate apatite crystals. Calcium carbonate is useful as a source of carbonate ion. However, calcite, which is stable phase calcium carbonate, has a small dissolution rate and cannot sufficiently supply carbonate ion into solution. Hence, in the case of mixing calcite and $\alpha$TCP in solution, the deposition of calcium deficient hydroxyapatite crystals becomes predominant, though carbonate apatite crystals are partially deposited.

On the other hand, metastable phase vaterite has a larger dissolution rate than that of calcite and can supply a large amount of carbonate ion into solution. Hence, in the case of mixing vaterite and $\alpha$TCP in solution, calcium deficient hydroxyapatite crystals are less deposited, and the deposition of carbonate apatite crystals becomes predominant. Hence, the solid portion essentially contains vaterite and $\alpha$TCP.

The liquid portion to be mixed with the solid portion essentially contains phosphoric acid salt. This is because calcium ion, carbonate ion, and phosphoric acid ion are eluted from vaterite and $\alpha$TCP into solution containing phosphoric acid ion so that the solution becomes supersaturated with respect to carbonate apatite to form carbonate apatite in a relatively short time and therefore, appropriate setting time is ensured.

The phosphoric acid salt is not particularly limited as long as the phosphoric acid salt can be dissolved to supply phosphoric acid ion into solution. $NaH_2PO_4$, $Na_2HPO_4$, $Na_3PO_4$, $KH_2PO_4$, $K_2HPO_4$, $K_3PO_4$, $(NH_4)H_2PO_4$, $(NH_4)_2HPO_4$, $(NH_4)_3PO_4$ and a mixed salt thereof are further preferred from the viewpoint of solubility, etc. The phosphoric acid salt concentration in the solution is preferably 0.1 mol/L or higher, more preferably 0.2 mol/L or higher, further preferably 0.4 mol/L or higher. This is because a higher phosphoric acid salt concentration increases the degree of supersaturation of the solution with respect to carbonate apatite.

pH of the solution is not particularly limited and is preferably 6.0 or higher and 9.0 or lower from the viewpoint of tissue affinity.

[X Bone defect regeneration kit: vaterite content]

Next, [33] will be described. An essential requirement for the solid portion of the above described defect reconstruction kit is that it contains vaterite and $\alpha$-tricalcium phosphate. The solid portion, when kneaded with the liquid portion, sets to form carbonate apatite. The amount of vaterite contained in the solid portion is preferably 10 mass% or larger and 60 mass% or smaller, more preferably 15 mass% or larger and 50 mass% or smaller, further preferably 20 mass% or larger and 40 mass% or smaller, from the viewpoint of the amount of carbonate apatite formed, the amount of carbonate groups in the carbonate apatite structure, the mechanical strength of a setting product, etc.

[X Bone defect regeneration kit: liquid portion]

Next, [34] will be described. The liquid portion essentially contains phosphoric acid salt and preferably contains at least one selected from, acid containing plural carboxy groups, hydrogen sulfite salt, cellulose derivative, dextran sulfate salt, chondroitin sulfate salt, alginic acid salt, and glucomannan. Since phosphoric acid salt is essential for the liquid portion, the acid containing plural carboxy groups is substantially the same as a salt of the acid containing plural carboxy groups.

Examples of the acid containing plural carboxy groups include dicarboxylic acid and tricarboxylic acid. Examples of the dicarboxylic acid include oxalic acid, malonic acid, succinic acid, malic acid, itaconic acid, phthalic acid, glutaric acid, and maleic acid. Examples of the tricarboxylic acid include aconitic acid.

The acid containing plural carboxy groups added to the liquid portion shortens setting time. This is probably because the acid containing plural carboxy groups is chelated with $\alpha$TCP or vaterite and therefore, the setting of the solid portion kneaded with the liquid portion proceeds through chelating reaction.

Examples of the hydrogen sulfite salt include sodium hydrogen sulfite and potassium hydrogen sulfite. Although much remains to elucidate the mechanism of action of the hydrogen sulfite salt, the liquid portion containing the hydrogen sulfite salt shortens setting time.

The cellulose derivative, dextran sulfate salt, chondroitin sulfate salt, alginic acid salt, or glucomannan added to the liquid portion produces viscosity. This improves handleability when the solid portion is kneaded with the liquid portion.

The cellulose derivative is cellulose chemically modified so as to possess solubility in solvents. Examples thereof include carboxymethylcellulose, methylcellulose, hydroxypropylcellulose, hypromellose and salts thereof. The cellulose derivative added to the liquid portion increases the viscosity of solution and as a result, improves handleability when the solid portion is kneaded with the liquid portion. The concentration of the cellulose derivative to be added differs depending on the degree of polymerization, etc. and is generally preferably 2 mass% or smaller.

Paste obtained by kneading the solid portion with the solution supplemented with such an additive may be able to suppress a property of collapsing upon contact of the paste with body fluids. This is probably because the infiltration of moisture into paste before setting causes collapse. Specifically, it is considered that disintegration ascribable to water can be suppressed by enhancing the viscosity of the paste and thereby suppressing the infiltration of moisture into the paste, or by accelerating the setting reaction.

[X Bone defect regeneration kit: volume of vaterite]

Next, [35] will be described. The volume of vaterite contained in the solid portion is not particularly limited. The bone defect reconstruction kit wherein the volume of vaterite contained in the solid portion is $10^{-12}$ m$^3$ or larger is preferred because a setting product may have large mechanical strength. Vaterite having a volume of $10^{-12}$ m$^3$ or larger presumably plays the same role as a filler in composite materials, though the mechanism underlying increased mechanical strength has not yet been elucidated. Vaterite having a volume of $10^{-12}$ m$^3$ or larger is not completely consumed through reaction with αTCP. Specifically, calcite coated with carbonate apatite is formed in a setting product. Calcium carbonate coated on the surface of carbonate apatite is useful because it may enhance osteoconductivity by releasing calcium ion. The solid portion contains preferably 10 mass% or larger, more preferably 20 mass% or larger, of vaterite having a volume of $10^{-12}$ m$^3$ or larger.

[X Bone defect regeneration kit: volume of vaterite]

Next, [36] will be described. For increasing the purity or content of formed carbonate apatite by imparting porousness to a setting product, the average diameter of vaterite contained in the solid portion is preferably 6 μm or smaller. The particle diameter is more preferably 4 μm or smaller, further preferably 2 μm or smaller. This is probably because a smaller particle diameter gives a larger specific surface area and increases the dissolution rates of carbonate ion and calcium ion in solution, thereby promoting carbonate apatite formation. On the other hand, the decreased particle diameter may deteriorate viscosity when the bone defect reconstruction kit is kneaded. Hence, the particle diameter may be preferably 0.5 μm or larger and 1 μm or smaller.
Hereinafter, the present invention will be described in more detail with reference to Examples. However, the scope of the present invention is not limited by Examples.
General conditions are as described below. Conditions different from these conditions are described in individual Examples or individual Comparative Examples.

<Raw material>

In the present Examples and Comparative Examples, the calcium hydroxide used was an ultra-high purity product (CSH) manufactured by Ube Material Industries, Ltd.; the calcium carbonate used was Calmaru (mean particle diameter: 5 μm) manufactured by Sakai Chemical Industry Co., Ltd.; the α-tricalcium phosphate used was α-TCP-B manufactured by Taihei Chemical Industrial Co., Ltd.; and the superhard plaster used was custom made from New Fujirock White manufactured by GC by the removal of components other than calcium sulfate, unless otherwise specified. Calcined plaster is calcium sulfate hemihydrate. All of them satisfied (B).
All of other reagents used were of special grade. Thus, all materials produced in the present Examples and Comparative Examples were artificial materials.
Calcium hydroxide spheres were produced by forming calcium hydroxide supplemented with 0.5 mass% of Kuraray Poval PVA-205C manufactured by Kuraray Co., Ltd. as a binder into a spherical shape by the spray drying method, and producing by sifting calcium hydroxide spheres that passed through a 200 μm sieve and did not pass through a 100 μm sieve. The spray draying produces spheres depending on surface tension. Hollow spheres were produced presumably because the spheres were dried from the circumference during the drying. The produced calcium hydroxide spheres were heated to 1000°C at 5°C/min in an electric furnace, heated at 1000°C for 6 hours, and furnace cooled for production.
In the production of honeycomb structures, a raw material calcium composition was mixed with a wax-based binder manufactured by Nagamine Manufacturing Co., Ltd. at a mass of 75:25. Then, a honeycomb structure formation mold was attached to Labo Plastomill manufactured by Toyo Seiki Seisaku-sho, Ltd., and extrusion forming was performed. Basically, a cylindrical honeycomb structure of a binder-containing raw material calcium composition which consisted of the polymer material-containing raw material calcium composition produced by extrusion forming, and had a peripheral wall was produced and subjected to debindering and, if necessary, carbonation, peripheral wall removal, etc.

<Reaction vessel>

A split reaction vessel having a diameter of 6 mm and a height of 3 mm was used unless otherwise specified.

The bottom face of the reaction vessel is closed with a glass plate, and the top face opens. A raw material is introduced to the reaction vessel from the top face that opens, which is then closed with a glass plate so that the raw material is not escaped from the reaction vessel. Water or water vapor can infiltrate into the reaction vessel through the gap. Hereinafter, this reaction vessel is referred to as a 6 mm (diameter)-3 mm (height) split reaction vessel for the sake of convenience.

All the volumes of calcium compositions, calcium phosphate compositions and raw material calcium compositions of Examples were $3 \times 10^{-11}$ m$^3$ or larger, so that the description of volumes may be omitted in Examples and Comparative Examples.

90% methanol is a mixture of 90 vol% of methanol and 10 vol% of water. 90% ethanol is a mixture of 90 vol% of ethanol and 10 vol% of water. 90% acetone is a mixture of 90 vol% of acetone and 10 vol% of water.

The "carbonation with 90% methanol of 4°C" means that carbon dioxide of 4°C containing 90% methanol is introduced at 100 mL/min into a container (capacity: 500 mL) of 4°C containing a raw material calcium composition. If a container having a different capacity was used, the amount of the material introduced was determined by proportional calculation from the above described amount. This flows 90% methanol of 4°C around the raw material calcium compound. An excess of carbon dioxide was discharged from the outlet of the container. This process is achieved by reaction for 7 days followed by drying.

<Powder X ray diffractometry: XRD analysis>

In the present invention, compositional analysis was conducted with powder X ray diffraction apparatus model D8 ADVANCE manufactured by Bruker Japan K.K. The output was 40 kV and 40 mA, and the X-ray source was CuK$\alpha$ ($\lambda$ = 0.15418 nm). Patterns obtained by XRD analysis are referred to as XRD patterns.

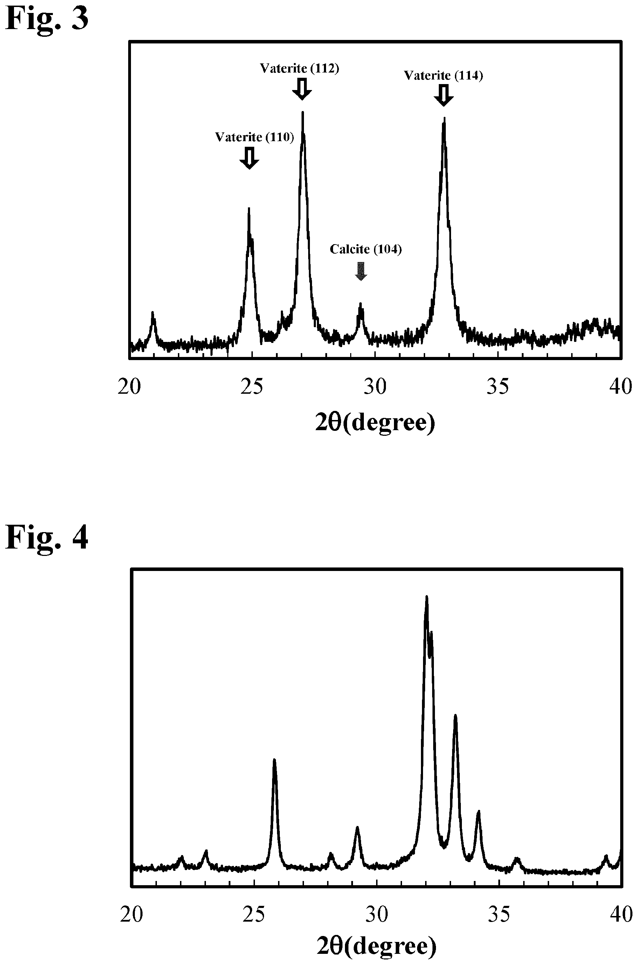

The contents of vaterite and calcite in medical calcium carbonate compositions are calculated from peak area ratios in XRD patterns. The peak areas of the 110, 112, and 114 planes of vaterite shown in Figure 3 are used as the peaks of vaterite, and the peak area of the 104 plane of calcite shown in Figure 3 is used as the peak of calcite.

If medical calcium carbonate compositions contain a substance other than vaterite and calcite, the contents of vaterite and calcite are calculated by the internal standard method.

<Mean particle diameter>

A powder was dispersed in 100 mL of distilled water and dispersed for 30 seconds with an ultrasonic washing machine with a frequency of 45 kHz-100 W, and a particle size distribution was determined within 1 minute thereafter using a laser diffraction particle size distribution measurement apparatus (manufactured by Shimadzu Corp., SALD-300V). A particle diameter at an integrated value of 50% in the particle size distribution was regarded as a mean particle diameter.

<Arithmetic average roughness (Ra)>

The arithmetic average roughness (Ra) of material surface was measured with Color 3D Laser Microscope VK-9710 manufactured by Keyence Corp.

<Mercury intrusion porosimetry>

The porosimetry was performed using AutoPore 9420 manufactured by Shimadzu Corp. The contact angle between mercury and a material used in calculation, etc. is as mentioned above.

<Remaining material after acid dissolution>

Remaining materials after acid dissolution were defined as remaining materials after calcium carbonate, etc. was dissolved in 1 mol/L hydrochloric acid having a volume of 20 molar equivalents with respect to the calcium carbonate, etc., and were indicated in % of dry mass with respect to the mass of the calcium carbonate, etc.

For example, a sample consisting of 0.2 g of calcium carbonate was dissolved in 40 mL of 1 mol/L aqueous hydrochloric acid solution, and the mixture was filtered and washed with water. Remaining materials after acid dissolution that did not pass through the filter were dried, followed by mass measurement. The ratio of the mass to the sample mass was indicated in percent.

Since the remaining materials after acid dissolution are numeric values of significance for raw material calcium compositions containing polymer material, the values may be omitted when raw material calcium compositions containing no polymer material were used.

<Compressive strength>

Compressive strength was measured as an index for mechanical strength. The compressive strength of calcium hydroxide block products was measured with a universal tester (model AGS-J) manufactured by Shimadzu Corp. A sample was broken at a crosshead speed of 10 mm/min, and the compressive strength was measured from the peak load at break.

<Volume>

The bulk volumes of produced cylindrical compositions were calculated by measuring diameters and heights. The same holds true for other forms. The volumes of all materials produced in Examples and Comparative Examples except for Comparative Example 3 were $10^{-12}$ m$^3$ or larger, so that the description of volumes may be omitted in Examples and Comparative Examples.

<Antimicrobial property and cytotoxicity test>

Antimicrobial properties were tested in accordance with JIS Z2801. The test strain used was *Staphylococcus epidermidis* (NBRC12993). Bacterial count was adjusted to $4.35 \times 10^4$ CFU/mL using 1/500 plain broth medium, and the bacterial solution was inoculated to sample surface. Then, one side of the sample was coated with a polyethylene film, followed by culture at 37°C at a relative humidity of 90% for 24 hours. The bacterial solution thus cultured was recovered and diluted, if necessary, and the bacterial count was measured by the agar plate medium method.
Cytotoxicity test was conducted in accordance with ISO10993-5: 2009. Specifically, $1 \times 10^4$ MC3T3-E1 cells were inoculated to sample surface, and a mixed solution of Minimum Essential Medium Eagle-a modification, 10% fetus bovine serum, and 1% antibiotic was used as a culture medium in an incubator of 37°C having a carbon dioxide concentration of 5 vol%. After culture for 24 hours, the cells were fixed in 2% glutaraldehyde and Hoechst stained, and cell count was measured. A cell survival rate of 70% or less was regarded as cytotoxicity present, and a cell survival rate exceeding 70% was regarded as cytotoxicity absent.

<Physical property evaluation of bone defect reconstruction kit>

The setting characteristics of bone defect reconstruction kits were measured at 37°C under a relative humidity condition of 100%. Setting time was measured by the Vicat needle method, and diametral tensile strength was measured 24 hours after setting. The composition of setting products was analyzed by XRD analysis and FT-IR analysis.

(Example 1)

A calcium hydroxide powder of guaranteed reagent manufactured by FUJIFILM Wako Pure Chemical Corp. was placed in a mold and uniaxially pressurized at a pressure of 20 MPa to produce a calcium hydroxide compact having a diameter of 6 mmΦ and a height of 9 mm.
Subsequently, 100 mL of 90% methanol was placed in a 500 mL reaction vessel, and the calcium hydroxide compact was placed on a mesh disposed so as not to contact the sample with the solvent. The temperature was set to 4°C.
Carbon dioxide was introduced at 100 mL/min to the 90% methanol portion of the reaction vessel through a bubbler and discharged from a discharge valve located at the upper part of the reaction vessel. The calcium hydroxide compact was thereby exposed to carbon dioxide containing 90% methanol while carbon dioxide containing 90% methanol was circulated around the calcium hydroxide compact.
Methanol inhibits calcite formation or calcite crystal growth, and relatively promotes formation of calcium carbonate other than calcite.
Calcium hydroxide was hardened to produce a block having a volume of $2.5 \times 10^{-4}$ m$^3$. Since it was produced by circulating carbon dioxide containing 90% methanol around the calcium hydroxide compact, water secondarily produced through the reaction of calcium hydroxide with carbon dioxide was removed by vaporization from the inside of the calcium hydroxide compact.
From XRD analysis (Figure 3), unreacted calcium hydroxide was found when the exposure period was 2 days, whereas the composition was confirmed to be pure calcium carbonate containing 97 mass% of vaterite and 3 mass% of calcite when the exposure period was 7 days. The remaining materials after acid dissolution were 0 mass%.
From these results, it was confirmed that the medical vaterite composition of the present invention was able to be produced.
The medical vaterite composition had porosity of 45%, and contained 20 mass% or larger of vaterite and therefore, a constant based on polymorph was 0.01. Although standard compressive strength was calculated to be 0.23 MPa, the compressive strength of the composition was 24 MPa and was thus also confirmed to be over the standard compressive strength.

Subsequently, the medical vaterite composition was immersed in 1 mol/L aqueous $Na_2HPO_4$ solution of pH 8.9 at 80°C for 3 days so as to add phosphoric acid salt to the medical vaterite composition.

The produced composition had a volume of $2.5 \times 10^{-4}$ m$^3$, and from carbonate group peaks detected in XRD analysis (Figure 4) and infrared spectroscopic spectra, the composition was confirmed to be pure carbonate apatite. As a result of elemental analysis, the amount of carbonate groups was 10.8 mass%. The remaining materials after acid dissolution were 0 mass%.

The porosity was 42%. Although standard compressive strength was calculated to be 29 MPa, the compressive strength of the composition was 32 MPa and was thus confirmed to be over the standard compressive strength.

These results demonstrated that a medical carbonate apatite composition is produced by adding phosphoric acid salt to a medical vaterite composition.

(Comparative Example 1)

A calcium hydroxide compact was exposed to carbon dioxide in the same manner as in Example 1 except that water was used instead of 90% methanol.

Calcium hydroxide was hardened by the exposure of the calcium hydroxide compact to carbon dioxide for 7 days to produce a block having a volume of $2.5 \times 10^{-4}$ m$^3$. From XRD analysis, the composition of the block was found to contain 98 mass% of calcite and 2 mass% of unreacted calcium hydroxide. The remaining materials after acid dissolution were 0 mass%.

The ratio of pore volume which pore diameter was 1 $\mu$m or larger and 6 $\mu$m or shorter with respect to a pore volume which pore diameter was 6 $\mu$m or shorter analyzed by mercury intrusion porosimetry was 0% in the composition.

The porosity was 44%. Although standard compressive strength was calculated to be 25 MPa, the compressive strength of the composition was 22 MPa and was thus less than the standard compressive strength. The produced calcite composition was a material that was not included in the present invention because any of (D) to (K) of [1] were not applicable thereto.

From the comparison of Example 1 to this Comparative Example, it was found that calcium carbonate containing 20 mass% or larger of vaterite can be produced by a process of inhibiting calcite formation or calcite crystal growth, and relatively promoting formation of calcium carbonate other than calcite; the organic solvent methanol is useful for the process; and vaterite formation proceeds faster than calcite formation.

Subsequently, the calcite block was immersed in 2 mol/L disodium hydrogen phosphate of 80°C for 3 days so as to add phosphoric acid salt to the medical vaterite composition.

The produced composition had a volume of $2.5 \times 10^{-4}$ m$^3$, and the remaining materials after acid dissolution were 0 mass%. However, from XRD analysis, the raw material calcite was confirmed to remain, though apatite was formed.

From the comparison of Example 1 to this Comparative Example, it was confirmed that a medical vaterite composition has a lot of reactivity and is useful in apatite production, as compared with a calcite composition.

(Comparative Example 2)

The same production method as in Example 11 of Patent Literature 8 was carried out. 1 to 2 mm sifted anhydrous granules of interconnected porous structure calcium sulfate were immersed in 50 mL of 2 mol/L aqueous sodium carbonate solution of 4°C for 14 days. As a result of compositional analysis by XRD analysis, the same XRD pattern as shown in Figure 3b) of Patent Literature 8 was obtained, and the polymorph of calcium carbonate was 83 mass% of calcite and 17 mass% of vaterite. The content of vaterite was 20 mass% or smaller. The produced calcium carbonate composition was a material that was not included in the present invention because any of (D) to (K) of [1] were not applicable thereto.

The comparison of Example 1 to this Comparative Example revealed the usefulness of a process of inhibiting calcite formation or calcite crystal growth, and relatively promoting formation of calcium carbonate other than calcite, typified by a process using methanol.

(Comparative Example 3)

The calcium hydroxide compact produced in Example 1 was immersed in 50 mL of 2 mol/L aqueous sodium carbonate solution of 4°C. The compact started to collapse immediately after immersion and eventually became a powder. The powder had a volume of smaller than $10^{-12}$ m$^3$. Thus, the produced calcite powder was not included in the present invention material.

As seen from the comparison among Example 1, Comparative Examples 1 and 2, and this Comparative Example, carbonation using 2 mol/L aqueous sodium carbonate solution of 4°C is limited by the content of formed vaterite, needs to employ a calcium sulfate block, etc. that does not collapse by immersion in aqueous solution, and fails to produce a vaterite block from a calcium hydroxide compact. These results demonstrated that the process of inhibiting calcite formation or calcite crystal growth, and relatively promoting formation of calcium carbonate other than calcite according to the present invention is excellent as a process of producing a medical vaterite composition.

(Example 2)

<1. Production of raw material calcium hydroxide composition>

A calcium hydroxide powder was placed in a mold and uniaxially pressurized at a pressure of 5 MPa to produce a calcium hydroxide compact having a diameter of 6 mmΦ and a height of 9 mm.

The calcium hydroxide powder and ethanol were kneaded at a liquid/powder ratio of 1.0, and the resulting calcium hydroxide paste was compacted at 5 MPa to produce a calcium hydroxide paste compact.

The raw material calcium hydroxide paste was injected to a silicon tube having an internal diameter of 5 mm and a length of 5 mm and serving as a mold to produce a calcium paste composition placed in the mold.

The calcium hydroxide paste was mixed with porogen sodium chloride granules that passed through a sieve having an opening of 100 μm and did not pass through a sieve having an opening of 50 μm, and the mixture was uniaxially pressurized at a pressure of 5 MPa to produce a sodium chloride granule-containing raw material calcium hydroxide paste compact having a diameter of 6 mmΦ and a height of 9 mm, which satisfied (AB6). All the specimens satisfied all the (AB1) to (AB3) conditions.

A portion of the calcium hydroxide compact was used to produce calcium hydroxide compact granules. Specifically, the calcium hydroxide compact was pulverized as poked with a surgical knife to produce calcium hydroxide compact granules that passed through a sieve having an opening of 2 mm and did not pass through an opening of 1.18 mm. The granules had a minor diameter of 1 mm or larger and shorter than 5 mm.

The calcium hydroxide powder was uniaxially pressurized at a pressure of 5 MPa such that a lactic acid-glycolic acid copolymer fiber was sandwiched thereby, to produce a calcium hydroxide compact having a diameter of 2 mmΦ and a height of 2 mm. Another calcium hydroxide compact was produced by a similar process such that its end was positioned at a site 2 mm distant from the calcium hydroxide compact. This process was repeated to produce a fiber-bonded raw material calcium hydroxide compact in a beaded form in which the fiber passed through the centers of the calcium hydroxide compacts having a diameter of 2 mmΦ and a height of 2 mm, and the raw material calcium hydroxide compacts were connected 2 mm away from each other with the fiber, which satisfied (AB8).

Since all the produced calcium hydroxide compositions satisfied (AB1) to (AB3), it was confirmed that a medical calcium hydroxide composition was able to be produced as to the above-described raw material calcium hydroxide composition that satisfied (AB6) or (AB8).

<2. Placement of raw material calcium composition, etc. in reaction vessel>

The reaction vessel used was a stainless pressurized vessel (TA125N) manufactured by AS ONE Corp. The reaction vessel has three screw holes (holes A, B, and C) and a top cover. Using a tube joint manufactured by Nihon Pisco Co., Ltd, a pipe was placed from the hole A so as to reach the bottom of the reaction vessel; a pipe was placed from the hole B to make 5 cm in a downward direction from the cover of the reaction vessel; and the power cord of an external fan was put in the reaction vessel from the hole C. The inside of the tube joint was sealed with epoxy resin. The holes A and B functioned as an inlet and an outlet.

90% ethanol and a stirrer piece were placed in the reaction vessel. Subsequently, a mesh container containing the raw material composition was placed in no contact with 90% ethanol. The fan was placed on the mesh container. The fan blew in on the mesh container side when turned on.

<3. Carbon dioxide replacement process>

In the covered state of the reaction vessel, carbon dioxide was introduced into the reaction vessel through the hole A from a carbon dioxide tank. Air in the reaction vessel was discharged from the hole B and replaced with carbon dioxide.

<4. Partial carbonation process>

Subsequently, the hole B was closed, and the carbon dioxide pressure in the reaction vessel was applied to atmospheric pressure to be 100 KPa using a pressure reducing valve of the carbon dioxide tank, followed by switch-on of the fan. Carbon dioxide containing 90% ethanol was thereby circulated around the raw material calcium composition.

When the hole A was blocked, carbon dioxide in the reaction vessel was consumed with the addition of carbonate component to the raw material calcium composition so that the pressure was lowered. The pressure reducing valve of the carbon dioxide tank supplied carbon dioxide when the pressure fell below the set pressure. Therefore, the carbon dioxide pressure in the reaction vessel was kept constant at 100 KPa in addition to atmospheric pressure.

As a result of compositional analysis by XRD 24 hours later, all the specimens were calcium hydroxide containing vaterite and a trace of calcite. The composition except for the porogen sodium chloride and the fiber was 72 mass% of vaterite, 3 mass% of calcite, and 25 mass% of calcium hydroxide for the calcium hydroxide compact, the calcium hydroxide compact granules, and the fiber-bonded raw material calcium hydroxide compact, and was 62 mass% of vaterite, 4 mass% of calcite, and 34 mass% of calcium hydroxide for the calcium hydroxide paste compact, the calcium paste composition placed in the mold, and the sodium chloride granule-containing raw material calcium hydroxide paste compact.

The raw material calcium composition was partially carbonated and did not collapse by immersion in 90% ethanol while keeping its shape, though unreacted calcium hydroxide remained.

<5. Carbonation process>

The reaction vessel was uncovered, and the fan was taken out thereof.

Subsequently, 90% methanol was further added in no contact with the pipe placed from the hole B such that the mesh container was completely immersed.

The above described <Carbon dioxide replacement process> was performed again, followed by closing of the hole B.

Subsequently, the reaction vessel was pressurized with the pressure reducing valve of the carbon dioxide tank such that the carbon dioxide pressure in the reaction vessel was 100 KPa in addition to the atmospheric pressure. The stirrer was rotated in the open state of the hole A.

6 days after the start of the carbonation process, the connection of the carbon dioxide tank connected with the hole A was canceled, and the hole B was opened to bring the pressurized state back to the atmospheric pressure state. Then, air was introduced to the reaction vessel from the hole B, and the solvent in the reaction vessel was discharged from the hole A. Subsequently, the hole A was closed, and the pressure in the reaction vessel was reduced using a pump from the screw hole B so as to dry the medical vaterite composition. After the drying, the pump was stopped, and air was introduced to the reaction vessel from the screw hole A to attain atmospheric pressure.

As a result of separate compositional analysis by XRD, the composition except for the porogen sodium chloride and the fiber was 95 mass% of vaterite and 5 mass% of calcite for the calcium hydroxide compact, the calcium hydroxide compact granules, and the fiber-bonded raw material calcium hydroxide compact, and was 93 mass% of vaterite and 7 mass% of calcite for the calcium hydroxide paste compact, the calcium paste composition placed in the mold, and the sodium chloride granule-containing raw material calcium hydroxide paste compact.

The integration of a plurality of 50 $\mu$m or larger and 100 $\mu$m or smaller pores was confirmed in the calcium carbonate composition produced from the sodium chloride granule-containing raw material calcium hydroxide paste compact, which however had no pores which maximum diameter was longer than 100 $\mu$m. The volume of 10 $\mu$m or smaller pores analyzed by mercury intrusion porosimetry was 0.53 cm$^3$/g.

No calcium hydroxide was detected in any of the specimens. The remaining materials after acid dissolution except for the fiber were 0 mass% in all the specimens.

Thus, it was confirmed that a medical vaterite block that satisfied (D) was produced from the calcium hydroxide compact, the calcium hydroxide paste compact, and the raw material calcium paste placed in the mold.

It was also confirmed that medical vaterite granules that satisfied the (D) and (G) conditions were produced from the sodium chloride granule-containing raw material calcium hydroxide paste compact.

It was further confirmed that medical vaterite granules that satisfied (K) were produced from the fiber-bonded raw material calcium hydroxide compact in a beaded form.

<6. Phosphoric acid component addition process>

1 mol/L aqueous Na$_2$HPO$_4$ solution of 80°C and pH 8.9 was introduced into the reaction vessel from the hole A such that the mesh container soaked.

The hole A was blocked, and the reaction vessel was degassed from the screw hole B using a diaphragm pump. Then, air was introduced from the hole B. By this operation, the pores of the medical calcium carbonate composition were degassed and also filled with the aqueous Na$_2$HPO$_4$ solution.

Subsequently, the temperature of the reaction vessel was kept at 80°C by the rotation of the stirrer piece.

<7. Washing and drying process>

7 days after the phosphoric acid component addition process, air was introduced from the hole B, and the aqueous $Na_2HPO_4$ solution was discharged from the hole A. Subsequently, water of 80°C was introduced to the reaction vessel from the hole A, and water was discharged from the hole B so as to wash the product.

Subsequently, air was introduced from the hole B, and water was discharged from the hole A. The hole A was blocked, and air was discharged from the hole B using a pump while the temperature of the reaction vessel was kept at 80°C so as to dry the product under reduced pressure.

From carbonate group peaks detected in XRD analysis and infrared spectroscopic spectra, the composition was confirmed to be pure carbonate apatite in all the specimens. As a result of elemental analysis, the amount of carbonate groups was 10.8 mass%. The remaining materials after acid dissolution were 0 mass%. All the specimens had a volume of $10^{-12}$ m$^3$ or larger.

From these analysis results, it was confirmed that a medical carbonate apatite block that satisfied all the (V1) to (V3) conditions was produced from the raw material calcium hydroxide compact, the raw material calcium hydroxide paste compact, and the raw material calcium paste placed in the mold.

The carbonate apatite composition produced from the calcium carbonate composition produced from the sodium chloride granule-containing raw material calcium hydroxide paste compact maintained pore morphology, and the integration of a plurality of 50 $\mu$m or larger and 100 $\mu$m or smaller pores was confirmed in this composition, which however had no pores which maximum diameter was longer than 100 $\mu$m. The volume of 10 $\mu$m or smaller pores analyzed by mercury intrusion porosimetry was 0.80 cm$^3$/g.

It was confirmed that a pore integrated-type medical carbonate apatite porous structure block that satisfied (V6) was produced by the dissolution of the porogen sodium chloride granules.

It was also confirmed that a fiber-bonded medical carbonate apatite block in a beaded form that satisfied (V10) was produced from the fiber-bonded raw material calcium hydroxide compact in a beaded form.

(Example 3)

The calcium hydroxide compact produced in <1. Production process of raw material calcium composition> of Example 2 was used. <2. Placement of raw material calcium composition, etc. in reaction vessel> and <3. Carbon dioxide replacement process> of Example 2 were performed, followed by carbonation with the reaction time in <4. Partial carbonation process> extended to 7 days.

As a result of compositionally analyzing the product by XRD analysis 7 days later, the composition was 95 mass% of vaterite, 4 mass% of calcite, and 1 mass% of calcium hydroxide.

From the comparison of Example 2 to this Example, it was found that a medical vaterite composition can be produced from a raw material calcium hydroxide compact by only carbonation under gas phase; and however, since 1 mass% of unreacted calcium hydroxide remained, a medical vaterite composition having higher purity can be produced by partial carbonation under gas phase and subsequent carbonation under liquid phase as compared with medical vaterite composition production by only carbonation under gas phase.

(Comparative Example 4)

The same process as in Example 3 was performed without rotating the fan.

As a result of compositionally analyzing the product by XRD analysis 7 days later, the composition was a mixture of 71 mass% of vaterite, 4 mass% of calcite, and 25 mass% of calcium hydroxide.

In Example 3 in which carbon dioxide containing 90% ethanol was circulated around the raw material calcium composition by the fan, 72 mass% of vaterite, 3 mass% of calcite, and 25 mass% of calcium hydroxide were obtained 1 day later. It was thus found that the carbonation of a raw material calcium compound is slow unless carbon dioxide or carbonate ion containing organic solvent is circulated around a raw material calcium composition in a closed reaction vessel.

It was also found that the content of vaterite is decreased due to transfer from vaterite phase to calcite phase because water formed inside a raw material calcium hydroxide compact is not removed.

In the production of a medical vaterite composition from a raw material calcium compound in a closed reaction vessel, it was thus found useful to circulate a substance, such as ethanol, which inhibits calcite formation or calcite crystal growth, and relatively promotes vaterite formation, together with carbon dioxide around the raw material calcium composition, and at the same time therewith, to remove water secondarily produced through the reaction of the raw material calcium composition with carbon dioxide by vaporization or the like.

(Example 4)

<1. Production of raw material calcium hydroxide composition>

Calcium hydroxide was mixed with porogen ammonium nitrate granules that passed through a 100 $\mu$m sieve and did not pass through a sieve having an opening of 50 $\mu$m such that the content of ammonium nitrate was 20 mass%. The mixture was uniaxially pressurized at a pressure of 5 MPa to produce an ammonium nitrate granule-containing raw material calcium hydroxide compact having a diameter of 6 mm$\Phi$ and a height of 9 mm, which satisfied (AB7).

<2. Placement of raw material calcium composition, etc. in reaction vessel>

The reaction vessel of Example 2 was used. However, approximately 10 g of ammonium carbonate manufactured by FUJIFILM Wako Pure Chemical Corp. was placed instead of 90% ethanol and the stirrer piece in the reaction vessel.

Subsequently, a mesh container containing the compact was placed in no contact with ammonium carbonate. A fan was placed on the mesh container.

<3. Carbon dioxide replacement process>

The same process as in Example 2 was performed.

<4. Carbonation process>

Subsequently, the hole B was closed, and the reaction vessel was heated to 70°C. Ammonium carbonate is known to be decomposed into carbon dioxide and ammonia at 58°C.

The carbon dioxide pressure in the reaction vessel was applied to atmospheric pressure to be 100 KPa using a pressure reducing valve of the carbon dioxide tank, followed by switch-on of the fan. Carbon dioxide containing ammonia was thereby circulated around the raw material calcium composition.

As a result of separately compositionally analyzing the product by XRD 7 days later, the composition was 91 mass% of vaterite and 9 mass% of calcite.

<5. Porogen removal process>

The product was taken out of the reaction vessel and immersed in 100 mL of ethanol at 25°C.

When ethanol was hourly replaced with fresh one five times, the porogen ammonium nitrate was completely removed. As a result of compositionally analyzing the product by XRD, the composition was 91 mass% of vaterite and 9 mass% of calcite. The volume of 10 $\mu$m or smaller pores analyzed by mercury intrusion porosimetry was 0.49 cm$^3$/g.

It was thus confirmed that a medical vaterite pore integrated-type porous structure that satisfied (G) was produced.

(Example 5)

A vaterite-containing composition was produced under the same conditions as in Example 1 except that 90% acetone was used instead of 90% methanol.

The composition produced by carbonation for 7 days had a volume of $2.5 \times 10^{-4}$ m$^3$. From XRD analysis, the vaterite content was 38 mass%, the calcite content was 62 mass%, and the remaining materials after acid dissolution were 0 mass%. The composition was therefore confirmed to be pure calcium carbonate.

The medical vaterite composition had porosity of 45%. Although standard compressive strength was calculated to be 0.23 MPa, the compressive strength of the composition was 14 MPa and was thus also confirmed to be over the standard compressive strength.

These results demonstrated that an organic solvent acetone is also effective for inhibiting calcite formation or calcite crystal growth, and relatively promoting formation of calcium carbonate other than calcite.

(Example 6)

50 g of a calcium hydroxide powder was suspended in a mixed solvent of 450 mL of methanol and 50 mL of water and stirred by bubbling carbon dioxide at 1000 mL/min at 4°C. 2 hours later, the bubbling of carbon dioxide was stopped. After being kept for 12 hours, the powder was isolated from the suspension by decantation and centrifugation and dried at 110°C for 2 hours. From XRD analysis, 100 mass% of a vaterite powder was confirmed to be produced. The mean particle diameter was 1 μm.

The vaterite powder was compacted at 300 MPa to produce a vaterite powder compact. The vaterite powder compact was heated to 200°C, 250°C, 300°C, 350°C, 400°C, or 450°C at 1°C/min using an electric furnace, sintered at this temperature for 6 hours, and furnace cooled to room temperature. As a result of XRD analysis, the sintered body obtained by sintering at 200°C, 250°C, 300°C, or 350°C contained 98 mass% of vaterite and 2 mass% of calcite. The sintered body obtained by sintering at 400°C contained 78 mass% of vaterite and 22 mass% of calcite. The vaterite powder compact collapsed and was unable to keep its shape when immersed in water, and got clouded when rubbed after attachment of water. On the other hand, all the sintered bodies neither collapsed by immersion in water nor got clouded when rubbed after attachment of water. The material thus heat treated was immersed in saturated aqueous solution of calcium carbonate in 10 times the amount of the material in a glass container and sonicated for 1 minute under conditions involving 28 kHz and an output of 75 W. As a result of comparing the dry weight of the composition thus irradiated to the dry weight before the ultrasonic irradiation, the ratio was 100% in all the specimens. Although the compressive strength of the vaterite powder compact was 3.2 MPa, the compressive strength of the sintered bodies obtained by sintering at 200°C, 250°C, 300°C, 350°C, 400°C, and 450°C was 4.6 MPa, 6.9 MPa, 11.3 MPa, 11.7 MPa, 12.1 MPa, and 11.8 MPa, respectively, in terms of estimations.

From these results, it was confirmed that sintered vaterite was produced.

(Example 7)

<Raw material calcium composition comprising polymer material>

A high-purity calcium carbonate powder having Mg content of $2 \times 10^{-5}$ mass%, Sr content of $1.6 \times 10^{-4}$ mass%, a mean particle diameter of 3 μm, and sphericity of 0.88 manufactured by Shiraishi Central Laboratories Co., Ltd. was mixed with a wax-based organic binder manufactured by Nagamine Manufacturing Co., Ltd. as a polymer material at a mass ratio of 75:25.

<(E1) Extrusion process>

A honeycomb structure formation mold was attached to Labo Plastomill manufactured by Toyo Seiki Seisaku-sho, Ltd., and extrusion forming was performed to prepare a polymer material-containing raw material calcium composition honeycomb structure in a cylindrical form having a peripheral wall as an intermediate.

<(E2) Forming process after extrusion process>

Aluminum angles on which release paper was placed was used as a mold for the purpose of straightening the polymer material-containing raw material calcium composition honeycomb structure. The polymer material-containing raw material calcium composition honeycomb structure was held in the mold, heat treated at 80°C for 24 hours, and cooled to room temperature. By this forming operation, the polymer material-containing raw material calcium composition honeycomb structure was straightened.

<(E3) Removal process of peripheral wall>

The peripheral wall of the polymer material-containing raw material calcium composition honeycomb structure in a cylindrical form was removed with an electric planer. The peripheral wall removal with an electric planer was easier for the polymer material-containing raw material calcium composition honeycomb structure smoothed by the forming process than for the polymer material-containing raw material calcium composition honeycomb structure without the forming process. Peripheral wall removal by polishing using a grindstone showed the evidence that a new peripheral wall was formed, where cutting with the electric planer showed no evident that a new peripheral wall was formed.

<(E4) Forming process after removal process of peripheral wall>

In order to completely remove deformation resulting from "(E3) Removal process of peripheral wall", the honeycomb structure was heat treated at 80°C for 24 hours and cooled to room temperature. By this forming operation, the deformation of the polymer material-containing raw material calcium composition honeycomb structure was removed.

<(E5) Debindering and calcium carbonate sintering process>

The honeycomb structure was heated to 250°C at 0.15°C/min, kept at 250°C for 1 hour, heated to 400°C to 510°C at 0.15°C/min, kept at this temperature (final temperature) for 24 hours, and then furnace cooled.

As a result of analyzing mass increase and mass decrease under this debindering and carbonation condition using a thermal mass analysis apparatus, the decrease in the amount of the polymer material was confirmed to be smaller than 1.0 mass%/min.

Figure 2 shows one example of mercury intrusion porosimetry results of the honeycomb structure produced at a final temperature of 480°C. As is evident therefrom, there exist pores having a peak at which the pore diameter attributed to the macropores of the honeycomb structure is approximately 70 $\mu$m as well as a pore diameter of 1 $\mu$m or smaller attributed to the micropores of the honeycomb partition walls.

The composition was confirmed by XRD analysis to be pure calcite in all the samples, regardless of the final temperature.

Table 1 summarizes the final temperature, and porosity, standard compressive strength with respect to the porosity, compressive strength, and the volume of pores which pore diameter is 10 $\mu$m or smaller with respect to a mass of the honeycomb structure analyzed by mercury intrusion porosimetry in the produced calcite honeycomb structure. The remaining materials after acid dissolution were 0 mass% in all the specimens.

[Table 1]

| Properties of calcite honeycomb structure and carbonate apatite honeycomb structure produced in Example 7 and Comparative Example 4 | | | | | | | | | | |
|---|---|---|---|---|---|---|---|---|---|---|
| | Calcite honeycomb structure | | | | | Carbonate apatite honeycomb structure | | | | |
| Sample | Final temperature (°C) | Porosity (%) | Standard compressive strength (Mpa) | Compressive strength (MPa) | Volume of 10 $\mu$m or smaller pore (cm$^3$/g) | Compositional transformation into apatite (days) | Porosity (%) | Standard compressive strength (Mpa) | Compressive strength (MPa) | Volume of 10 $\mu$m or smaller pore (cm$^3$/g) |
| Example 7 | 400 | 66 | 6 | 17 | 1.47 | <7 | 56 | 11 | 32 | - |
| Example 7 | 420 | 65 | 6 | 24 | 1.20 | <7 | 58 | 10 | 31 | 0.49 |
| Example 7 | 450 | 57 | 10 | 38 | 0.27 | <7 | 48 | 19 | 73 | - |
| Example 7 | 480 | 55 | 12 | 55 | 0.21 | <7 | 47 | 20 | 81 | 0.01 |
| Example 7 | 510 | 49 | 18 | 52 | 0.12 | 7-8 | 42 | 29 | 56 | - |
| Comparative Example 4 | 550 | 43 | 27 | 45 | 0.02 | $\times$ | - | - | - | - |

As shown in Table 1, the volume of pores which pore diameter was 10 $\mu$m or smaller with respect to a mass of the honeycomb structure analyzed by mercury intrusion porosimetry was a value larger than 0.02 cm$^3$/g in all the specimens. Thus, the medical calcite honeycomb structure of the present invention was able to be produced.

All the specimens exhibited compressive strength larger than standard compressive strength.

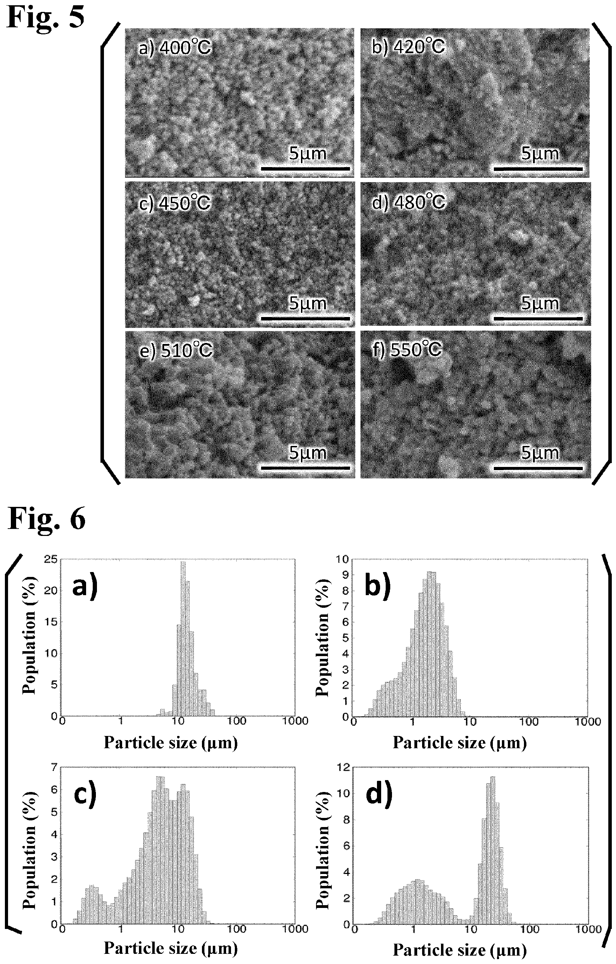

Figure 5 shows an electron microscope image of the surface of the produced medical calcite honeycomb structure. It was also confirmed that no remarkable grain growth was found up to the final temperature of 480°C, whereas grain growth was found for the final temperature of 510°C.

<Phosphoric acid component addition process>

Subsequently, the medical calcite honeycomb structure was immersed in 1 mol/L aqueous Na$_2$HPO$_4$ solution of 80°C and pH 8.9 for 7 days or 28 days. The presence or absence of complete compositional transformation into apatite was summarized in Table 1. When calcite remained on day 28, an x-mark is described in the table, which means failed compositional transformation into apatite. The remaining materials after acid dissolution were 0 mass% in all the specimens.

It was confirmed that by XRD analysis and FT-IR analysis that the medical calcium carbonate honeycomb structures produced at the final temperature of 480°C or lower and at the final temperature of 510°C were compositionally transformed into pure carbonate apatite by immersion for 7 days and for 28 days, respectively, which added phosphoric acid component to the medical calcite honeycomb structures. The carbonate content was 10.8 mass% in all the specimens.

The medical carbonate apatite honeycomb structure was thus confirmed to be produced. The medical carbonate apatite honeycomb structure was also found to exhibit compressive strength larger than standard compressive strength.

Unreacted calcite was found when the medical calcite honeycomb structure produced at 510°C, wherein the volume of 10 $\mu$m or smaller pores was 0.12 cm$^3$/g, was immersed in the aqueous solution for 7 days. These results demonstrated that provided that the volume of pores which pore diameter is 10 $\mu$m or smaller with respect to a mass of the honeycomb structure is any value larger than 0.02 cm$^3$/g, compositional transformation into carbonate apatite is achieved by immersion for 28 days; and however, the compositional transformation requires time and therefore, a larger pore volume is more preferred.

(Comparative Example 5)

A calcite honeycomb structure was produced by the same production method as in Example 7 except that the final temperature was set to 550°C. The remaining materials after acid dissolution were 0 mass% in all the specimens. Other results were summarized in Table 1. The volume of pores which pore diameter was 10 $\mu$m or smaller with respect to a mass of the honeycomb structure was 0.02 cm$^3$/g. The produced calcium carbonate composition was a material that was not included in the present invention because any of (D) to (K) of [1] were not applicable thereto.

The material was not compositionally transformed into apatite completely, albeit compositionally transformed into apatite partially, when immersed in 1 mol/L aqueous Na$_2$HPO$_4$ solution at 80°C for 28 days in the same manner as in Example 7. This partial compositional transformation into apatite revealed that immersion in the solution for a longer period seems to achieve complete compositional transformation into apatite and however, is not suitable for production.

From the comparison of Example 7 to this Comparative Example, it was confirmed that the micropores of a partition wall portion are also important for the reactivity of a medical calcite honeycomb composition; and an essential condition is that a volume of pores which pore diameter is 10 $\mu$m or smaller with respect to a mass of the honeycomb structure is larger than 0.02 cm$^3$/g.

(Example 8)

The same production as in Example 7 was performed using calcium carbonate samples having different mean particle diameters. All the calcium carbonate samples used had sphericity of 0.88, Mg content of $2.5 \times 10^{-5}$ mass% or smaller, and Sr content of $2 \times 10^{-4}$ mass% or smaller.

Figure 6 shows results of particle size distribution analysis using a laser diffraction particle size distribution measurement apparatus (manufactured by Shimadzu Corp., SALD-300V). Table 2 shows the properties of the medical calcite honeycomb structure and the medical carbonate apatite honeycomb structure. The remaining materials after acid dissolution were 0 mass% in all the specimens.

[Table 2]

| Properties of medical calcite honeycomb structure produced in Example 8 and medical carbonate apatite honeycomb structure | | | | | | | | | | |
|---|---|---|---|---|---|---|---|---|---|---|
| | | Medical calcite honeycomb structure | | | | Medical carbonate apatite honeycomb structure | | | | |
| Calcium carbonate | Mean particle diameter ($\mu$m) | Porosity (%) | Standard compressive strength (Mpa) | Compressive strength (MPa) | Ratio of pore volume (%) | Compositional transformation into apatite | Porosity (%) | Standard compressive strength (Mpa) | Compressive strength (MPa) | Volume of 10 $\mu$m or smaller pore (cm$^3$/g) |
| a | 14.3 | 65 | 6 | 8 | 0.24 | 7-28 | 49 | 18 | 18 | 0.37 |
| b | 1.6 | 63 | 7 | 19 | 0.25 | <7 | 58 | 10 | 16 | 0.57 |
| c | 4.3 | 78 | 2 | 27 | 0.28 | <7 | 66 | 6 | 30 | 0.82 |
| d | 6.5 | 55 | 12 | 55 | 0.21 | <7 | 47 | 20 | 84 | 1.08 |

Use of the calcium carbonate (a) having a mean particle diameter larger than 8 μm was found to produce a medical calcite honeycomb structure having relatively small compressive strength, albeit larger than standard compressive strength, and to relatively require time for compositional transformation into apatite in the phosphoric acid addition process. On the other hand, use of the calcium carbonate (b), (c), or (d) having a mean particle diameter of 8 μm or smaller was found to produce a medical calcite honeycomb structure having relatively large compressive strength. Use of the calcium carbonate (c) or (d) having a mean particle diameter of 2 μm or larger was found to produce a medical calcite honeycomb structure having larger compressive strength, and to take a relatively short time to complete compositional transformation into apatite in the phosphoric acid addition process.

These results demonstrated that for producing a medical calcium carbonate composition from polymer-containing calcium carbonate, it is preferred to use calcium carbonate having a mean particle diameter of 2 μm or larger and 8 μm or smaller.

(Example 9)

<Raw material calcium composition comprising polymer material>

A calcium hydroxide powder manufactured by FUJIFILM Wako Pure Chemical Corp. was pulverized into a mean particle diameter of 1 μm using a jet mill. The resulting calcium hydroxide was mixed with a wax-based organic binder manufactured by Nagamine Manufacturing Co., Ltd. as a polymer material at a mass ratio of 75:25.

<(E1) Extrusion process>

A honeycomb structure formation mold was attached to Labo Plastomill manufactured by Toyo Seiki Seisaku-sho, Ltd., and extrusion forming was performed to prepare a polymer material-containing raw material calcium composition honeycomb structure in a cylindrical form having a peripheral wall as an intermediate.

The cross-sectional area vertical to the through-holes of this honeycomb structure was 4.8 cm$^2$. The thickness of the peripheral wall was 220 μm, and the thickness of the partition wall was 75 μm.

<(E2) Forming process after extrusion process>

The same (E2) as in Example 7 was performed.

<(E3) Removal process of peripheral wall>

The same (E3) as in Example 7 was performed.

<(E6) Debindering and carbonation process>

The honeycomb structure was heated to 250°C at 0. 1°C/min under a stream of carbon dioxide at 200 mL/min (carbon dioxide partial pressure: approximately 101 KPa), kept at 250°C for 1 hour, heated to 450°C at 0.1°C/min, kept at 450°C for 1 hour, heated to 700°C at 0.1°C/min, kept at 700°C for 24 hours, and furnace cooled.

As a result of analyzing mass increase and mass decrease under this debindering and carbonation condition using a thermal mass analysis apparatus, the change was confirmed to be smaller than 1.0 mass%/hr. No crack was observed between the peripheral wall and the partition wall or inside the honeycomb structure.

<(E10) A process of structure finishing process after debindering and carbonation processes>

The produced honeycomb structure was subjected to final dimensional adjustment by polishing. Chipping occurred by cutting the medical calcite honeycomb structure after debindering, whereas polishing did not cause chipping, revealing that a finishing process done by polishing is preferred. Porosity and compressive strength were identical, regardless of the finishing process. The cross-sectional area vertical to the through-holes of the honeycomb structure was 3.9 cm$^2$, and the volume was $7.8 \times 10^{-6}$ m$^2$. The peripheral wall was removed and was thus 0 μm in thickness, and the honeycomb structure had a wall thickness of 78 μm. As a result of XRD analysis, the composition of the produced honeycomb structure was pure calcite. The remaining materials after acid dissolution were 0 mass%. The volume of pores which pore diameter was 10 μm or smaller with respect to a mass of the honeycomb structure analyzed by mercury intrusion porosimetry was 0.05 cm$^3$/g. Therefore, the production of a medical calcite honeycomb structure was confirmed.

The porosity was 48%. Although standard compressive strength was calculated to be 19 MPa, the compressive strength in the pore direction of the composition was 82 MPa.

A medical calcite honeycomb structure having a more arranged profile was produced by the finishing process.

<Phosphoric acid component addition process>

Following the finishing process, the medical calcite honeycomb structure was immersed in 1 mol/L aqueous $Na_2HPO_4$ solution of pH 8.9 at 80°C for 3 weeks. A small amount of calcite remained unreacted by immersion for 2 weeks, whereas it was confirmed by XRD analysis and FT-IR analysis that the honeycomb structure was compositionally transformed into pure carbonate apatite by immersion for 3 weeks, which added phosphoric acid component to the medical calcite honeycomb structure.

The volume was $7.8 \times 10^{-6}$ m$^2$, and the remaining materials after acid dissolution were 0 mass%. The volume of pores which pore diameter was 10 $\mu$m or smaller with respect to a mass of the honeycomb structure was 0.03 cm$^3$/g. Therefore, the production of a medical calcite honeycomb structure was confirmed. The porosity was 45%. Although standard compressive strength was calculated to be 23 MPa, the compressive strength of the composition was 89 MPa and was thus confirmed to be over the standard compressive strength. Thus, a medical carbonate apatite honeycomb structure was produced.

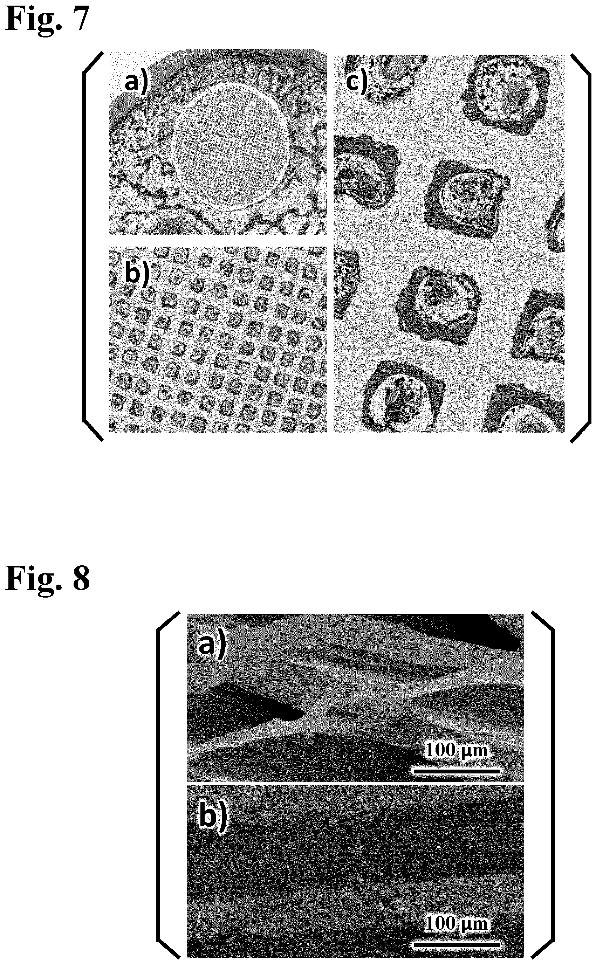

In order to verify the usefulness of the produced medical carbonate apatite honeycomb structure, wherein remaining materials after acid dissolution were 0 mass%, as a bone graft material in rabbits, a cylindrical medical carbonate apatite honeycomb structure having a diameter of 5.2 mm was separately produced in the same manner as in the above described Example 9, and implanted in the distal end of the rabbit femur. Figure 7 shows a histopathological image obtained by hematoxylin-eosin staining on week 4 after implantation. High osteoconductivity can be confirmed from bone conduction up to the central part. Bone formation was found inside all the through-holes, and the area of the formed bones with respect to the pore area was 32%.

In a histopathological image (Figure 18 of Patent Literature 12) related to a carbonate apatite honeycomb structure produced in Example 11 of Patent Literature 12, which is a material that is not included in the present invention because remaining materials after acid dissolution are 1.2 mass%, bones were formed in approximately 25% of pores, which is much lower than the value of bone conduction to the pores of the medical carbonate apatite honeycomb structure of the present invention, though bone conduction to the inside of the carbonate apatite honeycomb structure can be confirmed.

In a histopathological image (Figure 21 of Patent Literature 12) related to carbonate apatite honeycomb structure granules produced in Example 12 of Patent Literature 12, which are a material that is not included in the present invention because remaining materials after acid dissolution are 1.2 mass%, bones were formed in approximately 90% of through-holes and the area of the formed bones with respect to the pore area was 26%, both of which are lower than the values of bone formation in the through-holes of the medical carbonate apatite honeycomb structure of the present invention, though bone formation can be confirmed inside the through-holes of the carbonate apatite honeycomb structure granules.

From the comparison of this Example to Patent Literature 12, it was found that a carbonate apatite honeycomb structure is useful as a bone graft material excellent in osteogenic potential and however, differs in the degree of osteogenic potential depending on the presence or absence of remaining materials after acid dissolution; and the medical carbonate apatite honeycomb structure of the present invention wherein remaining materials after acid dissolution are 1 mass% or less is a medical material superior in osteoconductivity and osteogenic potential to a carbonate apatite honeycomb structure wherein remaining materials after acid dissolution are larger than 1 mass%, which is a material that is not included in the present invention.

(Comparative Example 6)

The same polymer material-containing raw material calcium composition as in Example 8 was used and extruded through a honeycomb structure formation mold that gave a 75 $\mu$m peripheral wall. The resulting honeycomb structure was not able to maintain its morphology, though some portions having a 75 $\mu$m peripheral wall and a partition wall thickness of 75 $\mu$m were confirmed. It was found that when a cross-sectional area vertical to the through-holes is less than 1 cm$^2$, even the same thicknesses of a peripheral wall and a partition wall permit extrusion and allow a honeycomb structure to be maintained. From the comparison of this Comparative Example to Example 8, it was found that when a cross-sectional area vertical to the through-holes is 1 cm$^2$ or larger, extrusion needs to be performed such that the peripheral wall of a honeycomb structure is thicker than the partition wall.

(Comparative Example 7)

A calcite honeycomb structure was produced by the method (heat treatment temperature: 450°C) of Example 1 of Patent Literature 12 and this method involving heat treatment temperatures of 600°C and 700°C.

Specifically, a calcium hydroxide powder manufactured by Nacalai Tesque, Inc. was pulverized into a mean particle diameter of 1 $\mu$m using a jet mill, and the resulting calcium hydroxide was mixed with a wax-based binder manufactured by Nagamine Manufacturing Co., Ltd. at a weight ratio of 75:25. Then, a honeycomb forming mold was attached to Labo Plastomill manufactured by Toyo Seiki Seisaku-sho, Ltd., and extrusion forming was performed. A cylindrical binder-containing calcium hydroxide honeycomb structure comprising the mixture of calcium hydroxide and the binder and having a peripheral wall was thus prepared. The peripheral wall of the cylindrical binder-containing calcium hydroxide honeycomb structure was removed with an electric planer. Then, the binder-containing calcium hydroxide honeycomb structure was debindered at 450°C, 600°C, or 700°C under a stream of oxygen containing 50% carbon dioxide.

The composition of the honeycomb structure thus debindered was analyzed using a powder X-ray diffraction apparatus model D8 ADVANCE manufactured by Bruker Japan K.K. under conditions involving an output of 40 kV and 40 mA, and CuKa ($\lambda$ = 0.15418 nm) as an X-ray source, and consequently found to be calcium carbonate in all the specimens.

The remaining materials after acid dissolution of the calcite honeycomb block debindered at 450°C, 600°C, or 700°C under a stream of oxygen containing 50% carbon dioxide were 1.2 mass%, 0.5 mass%, and 0 mass%, and the volume of pores which pore diameter was 10 $\mu$m or smaller with respect to a mass of the honeycomb structure analyzed by mercury intrusion porosimetry was 0.46 cm$^3$/g, 0.02 cm$^3$/g, and 0.01 cm$^3$/g. The produced calcium carbonate composition was a material that was not included in the present invention because any of (D) to (K) of [1] were not applicable thereto. Next, the produced calcium carbonate honeycomb structure was immersed in 1 mol/L aqueous disodium hydrogen phosphate solution of 80°C or 1 mol/L aqueous trisodium phosphate solution of 80°C for 7 days.

As a result of XRD analysis and FT-IR analysis, the calcium carbonate honeycomb structure produced at the debindering temperature of 450°C was compositionally transformed into carbonate apatite by phosphorylation with the aqueous disodium hydrogen phosphate solution and by phosphorylation with the aqueous trisodium phosphate solution to produce a carbonate apatite honeycomb structure. The remaining materials after acid dissolution were 1.2 mass% in both the specimens, revealing that in the case of phosphorylating a calcium carbonate composition containing remaining materials after acid dissolution, the remaining materials after acid dissolution remain.

On the other hand, the calcium carbonate honeycomb structure produced at the debindering temperature of 450°C was not compositionally transformed into carbonate apatite completely, albeit transformed into carbonate apatite partially, through immersion for 7 days by phosphorylation with the aqueous disodium hydrogen phosphate solution or by phosphorylation with the aqueous trisodium phosphate solution. These results demonstrated that the production method of Patent Literature 12 cannot produce the medical calcium carbonate composition of the present invention.

(Example 10)

The following debindering and carbonation process was performed using the polymer material-containing raw material calcium composition honeycomb structure (polymer material-containing calcium hydroxide honeycomb structure) used in Example 9.

<(E7) Debindering and carbonation process via calcium oxide>

The honeycomb structure was heated to 250°C at 0. 1°C/min under a stream of oxygen at 200 mL/min (oxygen partial pressure: approximately 101 KPa), kept at 250°C for 1 hour, heated to 450°C at 0. 1°C/min, and kept at 450°C for 24 hours. The composition at this stage was a mixture of calcium oxide and calcium hydroxide. Then, the honeycomb structure was heated to 850°C at 3°C/min and kept at 850°C for 3 hours. The composition at this stage was confirmed by XRD analysis to be calcium oxide. Subsequently, the honeycomb structure was furnace cooled to 350°C at 5°C/min. When the temperature reached 350°C, the gas in the reaction vessel was replaced with carbon dioxide and the vessel was hermetically sealed. Then, carbon dioxide was introduced to the vessel where carbonation was then performed at a pressure of 350 KPa for 14 days, followed by furnace cooling.

The cross-sectional area vertical to the through-holes of the produced honeycomb structure was 3.9 cm$^2$. The peripheral wall was removed and was thus 0 $\mu$m, and the honeycomb structure had a wall thickness of 69 $\mu$m.

As a result of XRD analysis, the honeycomb structure consisted of only calcite. The remaining materials after acid dissolution were 0 mass. The volume of pores which pore diameter was 10 $\mu$m or smaller with respect to a mass of the honeycomb structure analyzed by mercury intrusion porosimetry was 0.05 cm$^3$/g. Therefore, a pure medical calcite honeycomb structure was found to be produced.

The porosity was 52%. Although standard compressive strength was calculated to be 15 MPa, the compressive strength of the composition was 60 MPa and was thus confirmed to be over the standard compressive strength.

As a result of analyzing mass increase and mass decrease under this debindering and carbonation condition using a thermal mass analysis apparatus, the change was confirmed to be smaller than 1.0 mass%/hr.

<Phosphoric acid component addition process>

Subsequently, the medical calcite honeycomb structure was immersed in 1 mol/L aqueous $Na_2HPO_4$ solution of pH 8.9 at 80°C for 3 weeks. A small amount of calcite remained unreacted by immersion for 1 week, whereas it was confirmed by XRD analysis and FT-IR analysis that the honeycomb structure was compositionally transformed into pure carbonate apatite by immersion for 2 weeks, which added phosphoric acid component to the medical calcite honeycomb structure.

The volume was $7.8 \times 10^{-6}$ $m^2$, and the remaining materials after acid dissolution were 0 mass%. The volume of pores which pore diameter was 10 $\mu$m or smaller with respect to a mass of the honeycomb structure was 0.02 $cm^3$/g in terms of an estimation. Therefore, the production of a medical carbonate apatite honeycomb structure was confirmed.

The porosity was 45%. Although standard compressive strength was calculated to be 23 MPa, the compressive strength of the composition was 89 MPa and was thus also confirmed to be over the standard compressive strength.

From the comparison of this Example in which calcium carbonate was formed at 350°C to Example 9 in which calcium carbonate was formed at 700°C, it was found that phosphorylation reaction proceeded faster and formed calcium carbonate having higher reactivity in this Example.

(Example 11)

The following debindering and carbonation process was performed using the polymer material-containing raw material calcium composition honeycomb structure (polymer material-containing calcium hydroxide honeycomb structure) used in Example 9.

<(E8) Debindering and carbonation process via calcium carbonate and calcium oxide>

The honeycomb structure was heated to 250°C at 0.1°C/min under a stream of carbon dioxide at 200 mL/min (carbon dioxide partial pressure: approximately 101 KPa), kept at 250°C for 1 hour, heated to 450°C at 0.1°C/min, and kept at 450°C for 24 hours. Calcium hydroxide was confirmed by XRD analysis to become calcium carbonate at this stage. The honeycomb structure thus kept at 450°C for 24 hours was heated to 850°C at 3°C/min. After the temperature reached 850°C, the honeycomb structure was kept under a stream of oxygen at 200 mL/min (oxygen partial pressure: approximately 101 KPa) for 3 hours. Calcium carbonate was confirmed by powder X ray diffraction to become calcium oxide at this stage. Subsequently, the honeycomb structure was furnace cooled to 350°C at 5°C/min. When the temperature reached 350°C, the gas in the reaction vessel was replaced with carbon dioxide and the vessel was hermetically sealed. Then, carbon dioxide was introduced to the vessel where carbonation was then performed at a pressure of 350 KPa for 14 days, followed by furnace cooling.

The cross-sectional area vertical to the through-holes of the produced honeycomb structure was 3.7 $cm^2$. The peripheral wall was removed and was thus 0 $\mu$m, and the honeycomb structure had a wall thickness of 67 $\mu$m.

As a result of XRD analysis, the honeycomb structure consisted of only calcite. The remaining materials after acid dissolution were 0 mass. The volume of pores which pore diameter was 10 $\mu$m or smaller with respect to a mass of the honeycomb structure analyzed by mercury intrusion porosimetry was 0.04 $cm^3$/g. Therefore, a pure medical calcite honeycomb structure was found to be produced.

From the comparison of this Example in which calcium oxide was formed after carbonation of the polymer material-containing calcium hydroxide honeycomb structure to Example 10 in which calcium oxide was formed directly from the polymer material-containing calcium hydroxide honeycomb structure, the medical calcium carbonate honeycomb structure produced in this Example was found to have higher compressive strength.

<Phosphoric acid component addition process>

Subsequently, the medical calcite honeycomb structure was immersed in 1 mol/L aqueous $Na_2HPO_4$ solution of pH 8.9 at 80°C for 3 weeks. A small amount of calcite remained unreacted by immersion for 1 week, whereas it was confirmed by XRD analysis and FT-IR analysis that the honeycomb structure was compositionally transformed into pure carbonate apatite by immersion for 2 weeks, which added phosphoric acid component to the medical calcite honeycomb structure.

The volume was $7.8 \times 10^{-6}$ $m^2$, and the remaining materials after acid dissolution were 0 mass%. The porosity was 46%. Although standard compressive strength was calculated to be 22 MPa, the compressive strength of the composition was 120 MPa and was thus confirmed to be over the standard compressive strength. Thus, a medical carbonate apatite honeycomb structure was produced.

(Example 12)

Aqueous solution having a phosphoric acid concentration of 1 mol/L, a carbonate concentration of 0 mol/L or 0.5 mol/L, pH of 4.2, 7.2, 8.0 or 8.9, and a magnesium concentration of 0.1 mol/L was prepared using $NaH_2PO_4$, $Na_2HPO_4$, $NaHCO_3$, $Na_2CO_3$, or $MgCl_2$. Subsequently, the medical calcium carbonate honeycomb produced at the final temperature of 450°C in Example 7 was immersed in the aqueous solution of 80°C for 5 days, 7 days, or 28 days. Compositional transformation was analyzed by XRD analysis.

When immersed in the 1 mol/L aqueous $NaH_2PO_4$ solution (pH = 4.2) of 80°C for 5 days, the medical calcium carbonate honeycomb was compositionally transformed into calcium hydrogen phosphate while keeping its macrostructure. When immersed in the aqueous solution (pH = 7.2) of 80°C containing 0.1 mol/L $MgCl_2$ and having a phosphoric acid concentration of 1 mol/L for 7 days, the medical calcium carbonate honeycomb was compositionally transformed into a mixture of whitlockite and apatite while keeping its macrostructure. The remaining materials after acid dissolution were 0 mass% in both the specimens.

Table 3 shows results about the phosphoric acid concentration of 1 mol/L, the carbonate concentration of 0 mol/L or 0.5 mol/L, and pH 8.0 or 8.9. The remaining materials after acid dissolution were 0 mass% in all the specimens. An x-mark is described in the table, which means failed compositional transformation into pure apatite by immersion for 28 days. The carbonate content was a value obtained by increasing 5-fold carbon content obtained by elemental analysis. From these analysis results, a medical carbonate apatite honeycomb structure was found to be produced.

[Table 3]

| Properties of medical carbonate apatite honeycomb structure produced in Example 12 and results of Comparative Example 7 | | | | | | | | |
|---|---|---|---|---|---|---|---|---|
| | Calcite honeycomb structure | | | | | | | |
| Sample | pH | Carbonate concentration (mol/L) | Compositional transformation into apatite (days) | Carbonate content (mass%) | Porosity (%) | Standard compressive strength (Mpa) | Compressive strength (MPa) | Surface roughness Ra (μm) |
| Example 12 | 8.0 | 0.0 | <5 | 8.9 | 52 | 15 | 60 | 0.60 |
| Example 12 | 8.0 | 0.5 | 5 ~ 7 | 10.2 | 50 | 17 | 69 | 1.64 |
| Comparative Example 7 | 8.0 | 1.0 | × | - | - | - | - | - |
| Example 12 | 8.9 | 0.0 | 5 ~ 7 | 10.8 | 48 | 19 | 73 | 0.46 |
| Example 12 | 8.9 | 0.5 | 7 ~ 28 | 12.5 | 47 | 20 | 77 | 1.26 |
| Comparative Example 7 | 8.9 | 1.0 | × | - | - | - | - | - |

EP 4 023 262 A1

It was also found that use of aqueous solution of pH 8.9 serving as aqueous solution containing phosphoric acid component with pH 8.5 or higher produces a medical carbonate apatite honeycomb composition having carbonate content of 10 mass% or larger; and use of aqueous solution of pH 8.0 serving as aqueous solution containing phosphoric acid component with pH lower than 8.5 produces a medical carbonate apatite honeycomb composition having carbonate content of less than 10 mass%.

It was further found that use of aqueous solution of pH 8.0 serving as aqueous solution containing phosphoric acid component with pH lower than 8.5 allows addition of the phosphoric acid component to proceed faster and enables a medical carbonate apatite honeycomb structure to be produced with smaller carbonate content, as compared with use of aqueous solution of pH 8.9 serving as aqueous solution containing phosphoric acid component with pH 8.5 or higher.

It was further found that when 0.5 mol/L or lower carbonate component is allowed to coexist with aqueous solution containing phosphoric acid component, a medical carbonate apatite honeycomb structure having a larger amount of carbonate groups can be produced, though the addition of the phosphoric acid component is slowed down.

Figure 8 shows an electron microscope image when phosphoric acid component was added to the medical calcite honeycomb structure using aqueous solution of pH 8.9 containing 1 mol/L phosphoric acid component alone (a), and an electron microscope image when phosphoric acid component was added to the medical calcite honeycomb structure using aqueous solution of pH 8.9 containing 0.5 mol/L carbonate component coexisting with 1 mol/L phosphoric acid component (b). As is also evident from the surface roughness (Ra) in Table 2, it was found that when 0.5 mol/L or lower carbonate component is allowed to coexist in adding phosphoric acid component to a medical calcium carbonate composition, the produced medical carbonate apatite composition has larger surface roughness. Larger surface roughness enhances cell adhesion or osteoconductivity.

All the medical carbonate apatite honeycomb structures were also confirmed to exhibit compressive strength higher than standard compressive strength.

(Comparative Example 8)

Production was performed under the same conditions as in Example 12 except that the carbonate concentration of the aqueous solution to be prepared was set to 1.0 mol/L. As shown in Table 3, it was found that unlike the carbonate concentration of 0.5 mol/L, the carbonate concentration of 1.0 mol/L did not cause compositional transformation into pure apatite within 28 days.

From the comparison of Example 12 to this Comparative Example, a carbonate concentration of 0.5 mol/L or lower was found preferable for producing a medical carbonate apatite composition using aqueous solution containing phosphoric acid component and carbonate component.

(Comparative Example 9)

In order to evaluate the usefulness of the medical calcium carbonate composition of the present invention, the calcium carbonate block reportedly having high compressive strength disclosed in Example 1 of PCT/JP2018/00193 was prepared.

Specifically, calcium hydroxide (manufactured by FUJIFILM Wako Pure Chemical Corp.) and distilled water were mixed at a powder-water ratio of 1.13. The mixture was uniaxially pressed at 20 MPa using a mold to produce a calcium hydroxide compact having a diameter of 6 mm and a height of 3 mm.

Subsequently, the formed calcium hydroxide compact was carbonated with carbon dioxide with 100% relative humidity for 48 hours using a carbon dioxide contacting apparatus and then immersed in 1 mol/L aqueous sodium hydrogen carbonate solution of 80°C for 4 days.

From XRD analysis, the calcite content was 100 mass%, and the remaining materials after acid dissolution were 0 mass%.

Calcium hydroxide was hardened by the exposure of the calcium hydroxide compact to carbon dioxide for 7 days to produce a block having a volume of $2.5 \times 10^{-4}$ m$^3$. From XRD analysis, the composition of the block was found to contain 100 mass% of calcite. The remaining materials after acid dissolution were 0 mass%.

The ratio of pore volume which pore diameter was 1 $\mu$m or larger and 6 $\mu$m or shorter with respect to a pore volume which pore diameter was 6 $\mu$m or shorter analyzed by mercury intrusion porosimetry was 0% in the composition.

The porosity was 38%. Although standard compressive strength was calculated to be 38 MPa, the compressive strength of the composition was 32 MPa and was thus less than the standard compressive strength. The composition does not correspond to any of a honeycomb structure comprising a plurality of through-holes extending in one direction, a porous structure comprising a plurality of granules formed by being bonded to each other, and comprising a plurality of through-holes extending in plural directions, and a pore integrated-type porous structure wherein a plurality of pores is integrated to the whole medical composition.

Thus, the produced calcite composition was a material that was not included in the present invention.

From the comparison of this Comparative Example to Examples, it was found that the medical calcium carbonate composition of the present invention is a useful medical calcium carbonate composition excellent in compressive strength.

(Comparative Example 10)

In order to evaluate the usefulness of the medical calcium carbonate composition of the present invention, the calcium carbonate block porous structure reportedly having high compressive strength disclosed in Example 5 of PCT/JP2018/00193 was prepared.

Specifically, sodium chloride (manufactured by FUJIFILM Wako Pure Chemical Corp.) was sifted to produce 212 to 300 $\mu$m sodium chloride. Subsequently, calcium hydroxide (manufactured by FUJIFILM Wako Pure Chemical Corp.) and distilled water were mixed at a powder-water ratio of 1.0, and the resulting mixture was mixed with the sodium chloride at a mass ratio of 1:1.

Subsequently, the mixture was uniaxially pressed at 20 MPa using a mold to form a calcium hydroxide compact having a diameter of 6 mm and a height of 3 mm.

Subsequently, the produced calcium hydroxide compact was carbonated with carbon dioxide with 100% relative humidity for 1 hour using a carbon dioxide contacting apparatus and then immersed in 1 mol/L aqueous sodium hydrogen carbonate solution of 80°C for 4 days.

After the carbonation process, the block was washed with distilled water and immersed in distilled water of 80°C for 24 hours for complete dissolution and washing off of sodium chloride.

From XRD analysis, the calcite content was 100 mass%. The remaining materials after acid dissolution were 0 mass%. The volume was $2.5 \times 10^{-4}$ m$^3$.

The ratio of pore volume which pore diameter was 1 $\mu$m or larger and 6 $\mu$m or shorter with respect to a pore volume which pore diameter was 6 $\mu$m or shorter analyzed by mercury intrusion porosimetry was 0% in the composition.

The composition does not correspond to any of a honeycomb structure comprising a plurality of through-holes extending in one direction, a porous structure comprising a plurality of granules formed by being bonded to each other, and comprising a plurality of through-holes extending in plural directions, and a pore integrated-type porous structure wherein a plurality of pores is integrated to the whole medical composition.

The porosity was 65%. Although standard compressive strength was calculated to be 6 MPa, the compressive strength of the composition was 0.8 MPa and was thus less than the standard compressive strength.

Thus, the produced calcite composition was a material that was not included in the present invention.

From the comparison of this Comparative Example to Examples, it was found that the medical calcium carbonate composition of the present invention is a useful medical calcium carbonate composition excellent in compressive strength.

(Example 13)

The polymer material-containing raw material calcium composition (polymer material-containing calcium hydroxide) produced in Example 9 was used.

<(E1) Extrusion process>

A honeycomb structure formation mold was attached to Labo Plastomill manufactured by Toyo Seiki Seisakusho, Ltd., and extrusion forming was performed to prepare a polymer material-containing raw material calcium composition honeycomb structure in a cylindrical form having a peripheral wall as an intermediate.

<(E2) Forming process after extrusion process>

Before the extruded polymer material-containing raw material calcium composition honeycomb structure was cooled to room temperature, the softening of the honeycomb structure and pressure loading to the honeycomb structure were performed by pressing thereagainst cylindrical stainless of 10 cm in diameter heated to 100°C, to produce a polymer material-containing calcium hydroxide honeycomb structure wherein the diameter of the circle that passed through both ends of any one of the through-holes and a center of the through-hole was 10.5 cm.

<(E3) Removal process of peripheral wall>

The peripheral wall of the honeycomb structure was removed using a dental straight fissure bur. Use of the dental straight fissure bur was not found to form a new peripheral wall.

<(E7) Debindering and carbonation process via calcium oxide>

Subsequently, the honeycomb structure was heat treated and thereby carbonated at the same time with the complete debindering of the polymer material. First, carbon dioxide atmosphere was created in an electric furnace where the honeycomb structure was then heated from room temperature to 700°C, heat treated at 700°C for 48 hours, and furnace cooled (carbonation process).

The cross-sectional area vertical to the through-holes of the produced honeycomb structure was 3.7 cm$^2$.

The peripheral wall was removed and was thus 0 $\mu$m, and the honeycomb structure had a wall thickness of 67 $\mu$m. The diameter of the circle that passed through both ends of any one of the through-holes and a center of the through-hole was 10.2 cm.

As a result of XRD analysis, the inorganic composition was found to be only calcite. The remaining materials after acid dissolution were tested and consequently were 0 mass. The volume of pores which pore diameter was 10 $\mu$m or smaller with respect to a mass of the honeycomb structure analyzed by mercury intrusion porosimetry was 0.05 cm$^3$/g. Therefore, a pure calcium carbonate composition was found to be produced.

<Phosphoric acid component addition process>

Following the finishing process, the medical calcite honeycomb structure was immersed in 1 mol/L aqueous $Na_2HPO_4$ solution of pH 8.9 at 80°C for 3 weeks. It was confirmed by XRD analysis and FT-IR analysis that the medical calcite honeycomb structure was compositionally transformed into carbonate apatite by the addition of phosphoric acid component.

The cross-sectional area vertical to the through-holes of the produced honeycomb structure was 3.7 cm$^2$.

The peripheral wall was removed and was thus 0 $\mu$m, and the honeycomb structure had a wall thickness of 67 $\mu$m. The diameter of the circle that passed through both ends of any one of the through-holes and a center of the through-hole was 10.2 cm.

Thus, the production of a medical carbonate apatite honeycomb structure was confirmed.

(Example 14)

A calcium sulfate hemihydrate powder manufactured by Nacalai Tesque, Inc. was heat treated at 700°C to be calcium sulfate anhydrous, and then pulverized into a mean particle diameter of 1 $\mu$m using a jet mill. The resulting calcium sulfate was mixed with a wax-based polymer material manufactured by Nagamine Manufacturing Co., Ltd. such that the volume ratio of calcium sulfate was 50%, 53%, or 57% with respect to the calcium sulfate and the polymer material.

The following debindering and carbonation process was performed using a raw material calcium composition honeycomb structure produced by the same processes as <(E1) Extrusion process>, <(E2) Forming process after extrusion process>, <(E3) Removal process of peripheral wall>, and <(E4) Forming process after removal process of peripheral wall> of Example 7 except that the raw material calcium composition and the content of the raw material calcium composition were changed.

<(E6) Debindering and carbonation process>

The honeycomb structure was heated to 900°C in the atmosphere such that weight decrease derived from the polymer material was 1 mass%/min or smaller. The honeycomb structure was heat treated at 900°C for 24 hours and furnace cooled.

As a result of XRD analysis, the honeycomb structure consisted of only calcium sulfate anhydrous. The aqueous carbonation solution used was aqueous solution of pH 9 obtained by mixing 2 mol/L aqueous sodium hydrogen carbonate solution with 2 mol/L sodium carbonate. The honeycomb was immersed in the aqueous carbonation solution of 40°C for 4 days. As a result of powder XRD, the composition was pure calcite. The remaining materials after acid dissolution were 0 mass. Therefore, a pure medical calcium carbonate honeycomb structure was found to be produced.

Table 4 summarizes porosity (%), compressive strength (MPa), and the volume (cm$^3$/g) of pores which pore diameter is 10 $\mu$m or smaller with respect to a mass of the honeycomb structure analyzed by mercury intrusion porosimetry in the medical calcite honeycomb structure produced from the sample prepared such that the volume ratio of calcium sulfate was 50%, 53%, or 57% with respect to the calcium sulfate and the wax-based polymer material manufactured by Nagamine Manufacturing Co., Ltd. The remaining materials after acid dissolution were 0 mass% in all the specimens. From these results, it was confirmed that a medical calcium carbonate honeycomb structure was able to be produced.

[Table 4]

| Properties of medical calcite honeycomb structure and medical carbonate apatite honeycomb structure produced in Example 14 | | | | | | | |
|---|---|---|---|---|---|---|---|
| Volume ratio of calcium sulfate (%) | Medical calcite honeycomb structure | | | Medical carbonate apatite honeycomb structure | | | |
| | Porosity (%) | Compressive strength (MPa) | Volume of 10 $\mu$m or smaller pore (cm$^3$/g) | Compositional transformation into apatite (days) | Porosity (%) | Compressive strength (MPa) | Volume of 10 $\mu$m or smaller pore (cm$^3$/g) |
| 50 | 59 | 3.6 | 0.92 | <7 | 50 | 12 | 0.34 |
| 53 | 56 | 11.3 | 0.93 | <7 | 49 | 18 | 0.34 |
| 57 | 52 | 11.4 | 0.79 | <7 | 45 | 23 | 0.39 |

<Phosphoric acid component addition process>

Subsequently, the medical calcium carbonate honeycomb structure was immersed in 1 mol/L aqueous $Na_2HPO_4$ solution of 80°C for 7 days. It was confirmed by XRD analysis and FT-IR analysis that all the medical calcium carbonate honeycomb structures were compositionally transformed into carbonate apatite by the addition of phosphoric acid component. The remaining materials after acid dissolution were 0 mass% in all the specimens.

Thus, a medical carbonate apatite honeycomb structure was confirmed to be produced.

<Verification of usefulness as bone graft material>

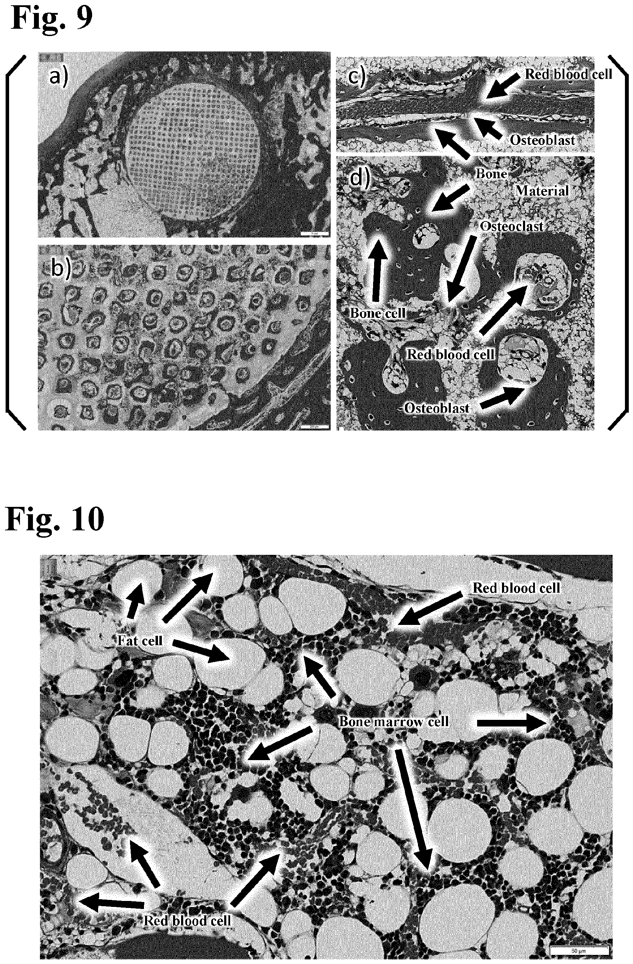

Figure 9 shows a histopathological image obtained by hematoxylin-eosin staining 4 weeks after implantation in the rabbit femur of a medical carbonate apatite honeycomb structure produced in the same manner as in the above described Example 14 from a sample prepared such that the volume ratio of calcium sulfate was 50% with respect to a mixture of the polymer material and the calcium sulfate. Bones were actively formed inside the honeycomb, and osteoblasts, osteoclasts, and bone cells were observed, revealing that the carbonate apatite honeycomb was replaced with new bones by bone remodeling. Red blood cells were also observed, revealing that it was replaced with bones having a Haversian canal structure.

Figure 10 shows a histopathological image obtained by hematoxylin-eosin staining 12 weeks after implantation. Not only red blood cells and fat cells but also bone marrow cells such as myeloblasts were found with high density inside the honeycomb, and 2000 or more bone marrow cells were confirmed within an observed area of 0.2 mm$^2$. Thus, 10000 cells/mm$^2$ of bone marrow cells were confirmed, which exceeded the usefulness criterion of 1000 cells/mm$^2$.

Thus, it was confirmed that a carbonate apatite honeycomb structure is also useful as a scaffold for cell culture, particularly, as a scaffold for cell culture of bone marrow cells or stem cells.

(Example 15)

The production of a pore integrated-type calcite porous structure wherein a plurality of pores which maximum diameter was 50 $\mu$m or longer and 400 $\mu$m or shorter was integrated via partition walls or inter-partition wall through-penetrations, not containing pores which maximum diameter was 800 $\mu$m or longer was studied using calcium sulfate hemihydrate (FUJIFILM Wako Pure Chemical Corp., guaranteed reagent) and spherical phenolic resin (manufactured by Lignyte Inc., LPS-C100) having a particle diameter of 100 $\mu$m as porogen. A spherical phenolic resin-free sample was also produced for comparison.

<Mixing process>

Spherical phenolic resin was mixed at 0, 10, 20, 30, or 40 mass% with calcium sulfate hemihydrate, and the mixture was kneaded using distilled water such that a powder-water ratio was 0.23 to produce paste.

<Compacting process>

The paste was introduced by manual pressing to a split vessel that permitted production of samples having a diameter of 6 mm and a height of 3 mm. The opening was closed with a glass plate, followed by setting reaction for 3 hours.

<Debindering and carbonation process>

The setting product was heated to 300°C at 0.13°C/min in the atmosphere using an electric furnace, heat treated at 300°C for 24 hours, heated to 700°C at 0.13°C/min, heated treated at 700°C for 3 hours, and cooled to room temperature at 5°C/min. As a result of analyzing mass increase and mass decrease under this debindering condition using a thermal mass analysis apparatus, the decrease in the amount of the polymer material was confirmed to be smaller than 1.0 mass%/min.

Subsequently, the setting product was immersed at 90°C for 24 hours in aqueous solution of pH 9 obtained by mixing 2 mol/L aqueous $Na_2CO_3$ solution with 2 mol/L $NaHCO_3$. The product thus immersed was washed with distilled water of 90°C.

As a result of powder XRD analysis, the composition was pure calcite in all the specimens. The remaining materials after acid dissolution were 0 mass in all the specimens.

Table 5 summarizes the properties of the product. In this context, "Ratio of pore volume" means the ratio of pore volume which pore diameter is 1 $\mu$m or larger and 6 $\mu$m or shorter with respect to a pore volume which pore diameter is 6 $\mu$m or shorter analyzed by mercury intrusion porosimetry, which is indicated in %.

[Table 5]

| Properties of medical pore bonded-type calcite porous structure and medical pore bonded-type carbonate apatite porous structure produced in Example 15 (including mass ratio of 0% of non-pore bonded-type LPS-C100) | | | | | | | | | | |
|---|---|---|---|---|---|---|---|---|---|---|
| Mass ratio of LPS-C100 (%) | Medical pore bonded-type calcite porous structure | | | | | Medical pore bonded-type carbonate apatite porous structure | | | | |
| | Porosity (%) | Standard compressive strength (Mpa) | Compressive strength (MPa) | Ratio of pore volume (%) | Volume of 10 $\mu$m or smaller pore ($cm^3$/g) | Compositional transformation into apatite (days) | Porosity (%) | Standard compressive strength (Mpa) | Compressive strength (MPa) | Volume of 10 $\mu$m or smaller pore ($cm^3$/g) |
| 0 | 49 | 18 | 27 | 9 | 0.46 | 7-28 | 49 | 18 | 18 | 0.37 |
| 10 | 59 | 9 | 12 | 44 | 0.83 | <7 | 58 | 10 | 16 | 0.57 |
| 20 | 66 | 6 | 8 | 47 | 1.12 | <7 | 63 | 7 | 14 | 0.82 |
| 30 | 71 | 4 | 2 | 54 | 1.36 | <7 | 68 | 5 | 7 | 1.08 |
| 40 | 79 | 2 | 1 | 40 | 1.85 | <7 | 76 | 3 | 4 | 1.67 |

The sample that was not supplemented with spherical phenolic resin was confirmed to be a medical calcite composition of the present invention which exhibited compressive strength larger than standard compressive strength, not a porous structure wherein "a plurality of pores was integrated via partition walls or inter-partition wall through-penetrations".

The sample produced from calcium sulfate hemihydrate containing 10, 20, 30, or 40 mass% of spherical phenolic resin was confirmed to be the "pore integrated-type calcite porous structure wherein a plurality of pores which maximum diameter was 50 $\mu$m or longer and 400 $\mu$m or shorter was integrated via partition walls or inter-partition wall through-penetrations, not containing pores which maximum diameter was 800 $\mu$m or longer" according to the present invention.

<Phosphoric acid component addition process>

The medical pore integrated-type calcite porous structure produced from calcium sulfate hemihydrate containing 10, 20, 30, or 40 mass% of spherical phenolic resin was immersed in 1 mol/L aqueous $Na_2HPO_4$ solution of 80°C for 7 days. It was confirmed by XRD analysis and FT-IR analysis that the medical pore formed-type calcite porous structure was compositionally transformed into carbonate apatite by the addition of phosphoric acid component.

On the other hand, in the case of immersing the pore integrated-type calcite porous structure produced from spherical phenolic resin-free calcium sulfate hemihydrate in 1 mol/L aqueous $Na_2HPO_4$ solution of 80°C for 7 days, unreacted calcite was found by XRD analysis. When the immersion period was changed to 28 days, it was confirmed by XRD analysis and FT-IR analysis that the medical calcite composition was compositionally transformed into carbonate apatite by the addition of phosphoric acid component.

This demonstrated that a medical calcium carbonate composition that satisfies the condition that a ratio of pore volume which pore diameter is 1 $\mu$m or larger and 6 $\mu$m or shorter with respect to a pore volume which pore diameter is 6 $\mu$m or shorter analyzed by mercury intrusion porosimetry is 10% or more is a medical material having high reactivity.

<Verification of usefulness as bone graft material>

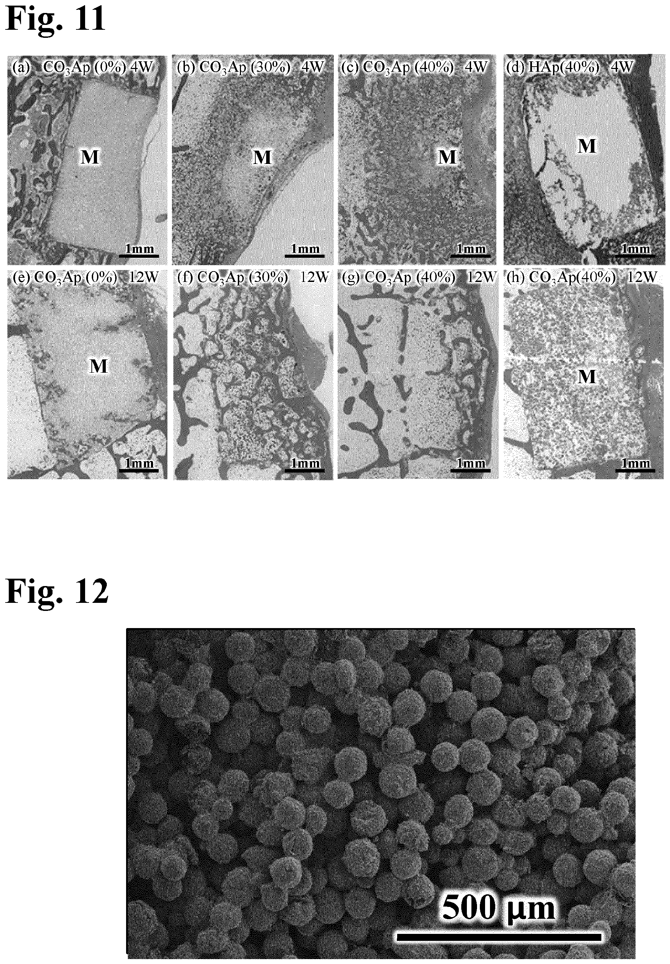

Figure 11 shows a histopathological image obtained by hematoxylin-eosin staining of a material excised together with its surrounding bones 4 weeks (4W) and 12 weeks (12W) after implantation in a rabbit femur defect of a medical carbonate apatite composition having a diameter of 6 mm and a height of 3 mm and a medical pore integrated-type carbonate apatite porous structure produced from calcium sulfate hemihydrate containing 0, 30, or 40 mass% of spherical phenolic resin. In the drawing, carbonate apatite is represented by $CO_3Ap$; the material is represented by M; and the number within the parentheses denotes spherical phenolic resin introduced in raw material. All the specimens exhibited excellent tissue affinity with no inflammatory reaction found. The ratio of pore volume which pore diameter was 1 $\mu$m or larger and 6 $\mu$m or shorter with respect to a pore volume which pore diameter was 6 $\mu$m or shorter analyzed by mercury intrusion porosimetry was 10% or more. As shown in Figures 11(b) and 11(c), it was found that the medical pore integrated-type carbonate apatite porous structure in the bonded form of approximately 100 $\mu$m pores was replaced with bones from the circumference. From high magnified tissue images, osteoclasts, osteoblasts, and red blood cells were observed, and bone replacement adapted to bone remodeling was also confirmed. As shown in Figures 11(f) and 11(g), it was found that on 12 weeks after implantation, the medical pore integrated-type carbonate apatite porous structure was almost completely replaced with bones so as to have a normal bone trabeculae structure.

On the other hand, as shown in Figure 11(a), it was found that the medical carbonate apatite composition wherein the ratio of pore volume which pore diameter was 1 $\mu$m or larger and 6 $\mu$m or shorter with respect to a pore volume which pore diameter was 6 $\mu$m or shorter analyzed by mercury intrusion porosimetry was less than 10% exhibited limited bone replacement on week 4 after implantation. This demonstrated that a medical calcium carbonate composition that satisfies the condition that a ratio of pore volume which pore diameter is 1 $\mu$m or larger and 6 $\mu$m or shorter with respect to a pore volume which pore diameter is 6 $\mu$m or shorter analyzed by mercury intrusion porosimetry is 10% or more is a medical material having high reactivity.

In the case of the medical carbonate apatite composition wherein the ratio of pore volume which pore diameter was 1 $\mu$m or larger and 6 $\mu$m or shorter with respect to a pore volume which pore diameter was 6 $\mu$m or shorter analyzed by mercury intrusion porosimetry was less than 10%, as shown in Figure 11(e), bone replacement was also confirmed on week 12 after implantation to start from the circumference.

(Comparative Example 11)

In order to verify the usefulness of the pore integrated-type carbonate apatite porous structure wherein a plurality of pores which maximum diameter was 50 $\mu$m or longer and 400 $\mu$m or shorter was integrated via partition walls or inter-partition wall through-penetrations, not containing pores which maximum diameter was 800 $\mu$m or longer, which was produced from the pore integrated-type calcite porous structure wherein a plurality of pores which maximum diameter

was 50 μm or longer and 400 μm or shorter was integrated via partition walls or inter-partition wall through-penetrations, not containing pores which maximum diameter was 800 μm or longer, spherical phenolic resin (LPS-C100) was mixed with hydroxyapatite (manufactured by Taihei Chemical Industrial Co., Ltd., HAP-200) such that the LPS-C100 content was 40 mass%. The mixture was uniaxially pressurized at 20 MPa. The obtained compact was heated to 300°C at 0.13°C/min in the atmosphere using an electric furnace, heat treated at 300°C for 24 hours, heated to 1000°C at 0.13°C/min, heated treated at 1000°C for 3 hours, and cooled to room temperature at 5°C/min.

As a result of XRD analysis, the composition was hydroxyapatite. Thus, the material was a material that was not included in the present invention. Hydroxyapatite is a typical bone graft material for clinical application.

Figures 11(d) and 11(h) each show a histopathological image obtained when this material was implanted in the rabbit femur in the same manner as in Example 15. In the drawing, hydroxyapatite is represented by HAp; the material is represented by M; and the number within the parentheses denotes spherical phenolic resin introduced in raw material. This material did not evoke inflammatory reaction, either. The pore integrated-type hydroxyapatite porous structure having approximately 100 μm pores bonded to each other was structurally similar to the medical pore integrated-type carbonate apatite porous structure, and despite this fact, underwent very limited tissue invasion to the inside of the material on week 4 after implantation, as compared with the histopathological image (c) of the pore integrated-type carbonate apatite porous structure. High magnified images revealed that connective tissues more than bone tissues invaded the material. Although tissues including bone tissues invaded the central part of the material on week 12 after implantation, the material kept its original shape, revealing that bone replacement rarely occurred.

The comparison of Example 15 to this Comparative Example revealed that the medical carbonate apatite composition of the present invention is very useful as a bone graft material.

(Example 16)

<Production of CaO granule having sphericity of 0.9 or larger and being hollow in shape>

0.5 mass% of polyvinyl alcohol (Kuraray Poval PVA-205C) was added to calcium hydroxide, and the suspension was spray dried to produce calcium hydroxide hollow spheres. The hollow spherical calcium hydroxide was heated to 1000°C at 50°C/min and sintered at 1000°C for 6 hours to produce CaO hollow spheres, which were then sifted. The hollow structure was confirmed by microCT. The sphericity was 0.98, the average diameter was $1.60 \times 10^{-4}$ m, and the average volume was $1.6 \times 10^{-12}$ m$^3$.

<Placement-closing process>

The CaO hollow spheres were placed in a split reaction vessel having a diameter of 6 mm and a height of 3 mm. The upper and lower openings of the reaction vessel were covered with glass plates, and the reaction vessel was closed with a C-clamp.

<Porous structure producing process>

Subsequently, the reaction vessel was immersed in water. Water was introduced into the reaction vessel through the gap between the glass plates and the split reaction vessel so that the CaO hollow spheres swelled to produce a porous structure comprising a plurality of granules which maximum diameter was approximately 80 μm, formed by being bonded to each other, and comprising a plurality of through-holes extending in plural directions. As a result of XRD analysis, the composition was confirmed to be pure calcium hydroxide. The volume was $2.8 \times 10^{-8}$ m$^3$, and the remaining materials after acid dissolution were 0 mass%.

Thus, a medical calcium hydroxide porous structure was found to be produced.

<Carbonation process>

Subsequently, the upper glass plate was removed from the reaction vessel, which was then placed in the carbonation reaction vessel used in Example 1. A carbonation process was performed for 7 days in the same manner as in Example 1 except that the temperature was set to 15°C.

As a result of microCT analysis and scanning electron microscope image analysis (Figure 12), a granule bonded-porous structure comprising a plurality of granules which maximum diameter was approximately 80 μm, formed by being bonded to each other, and comprising a plurality of through-holes extending in plural directions was found to be produced. The volume was $2.8 \times 10^{-8}$ m$^3$. As a result of powder XRD analysis, pure calcium carbonate having vaterite content of 79 mass% and calcite content of 21 mass% was confirmed. The remaining materials after acid dissolution were tested and consequently were 0 mass%. Therefore, a pure medical calcium carbonate porous structure was found to be produced. It was also found that a medical vaterite composition can be produced even at a temperature set to 15°C.

The porosity was 60%. Although standard compressive strength was calculated to be 0.1 MPa, the compressive strength of the composition was 5 MPa and was thus confirmed to be over the standard compressive strength.

<Phosphoric acid salt addition process>

Subsequently, the medical calcium carbonate porous structure was immersed in 1 mol/L disodium hydrogen phosphate of 80°C for 9 hours.

A granule bonded-porous structure comprising a plurality of granules which maximum diameter was approximately 80 $\mu$m, bonded to each other, and comprising a plurality of through-holes extending in plural directions was found to be produced. The volume was $2.8 \times 10^{-8}$ m$^3$. From XRD analysis and infrared spectroscopic spectra, the composition was confirmed to be pure carbonate apatite. The porosity was 56%, and the compressive strength was 4.1 MPa. Thus, a medical carbonate apatite porous structure was found to be produced.

<Animal experiment>

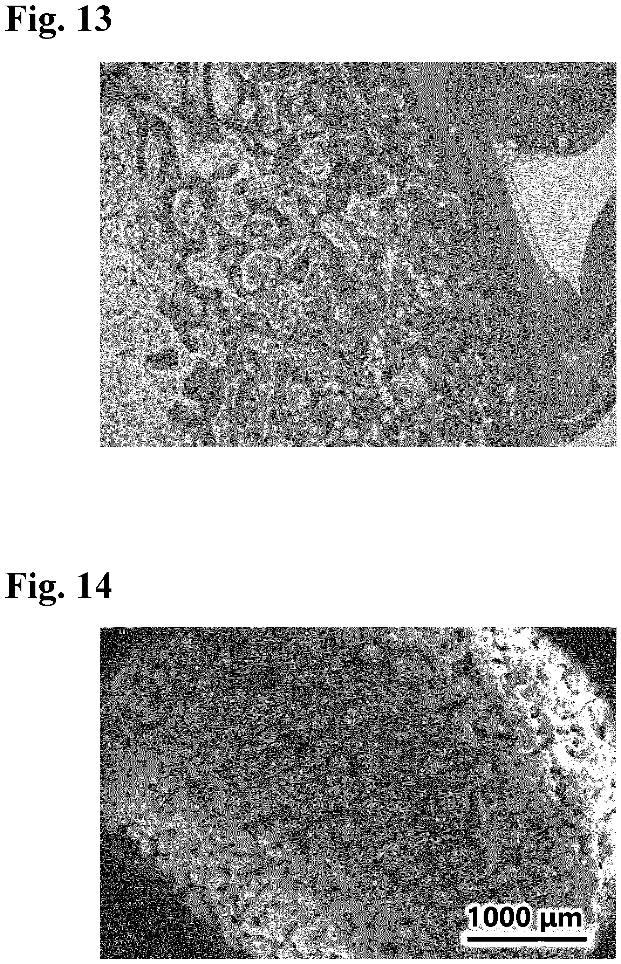

A $\Phi$6 mm bone defect was formed in the rabbit tibia, and the produced medical carbonate apatite porous structure was implanted therein. Figure 13 shows results of isolating the sample together with its surrounding tissues on week 4 after implantation, and conducting histopathological examination. Not only were excellent tissue affinity and osteoconductivity confirmed, but also the medical carbonate apatite porous structure was almost completely replaced with bones.

Such a carbonate apatite composition, which was almost completely replaced with bones on week 4 after implantation, has not been found so far. Thus, the medical carbonate apatite porous structure of the present invention was found to be a medical material very useful as a bone graft material.

(Example 17)

<Calcium sulfate dihydrate production process>

A calcium sulfate hemihydrate powder was kneaded at a powder-water ratio of 0.14, and an excess of water was removed at 20 MPa, followed by setting reaction for 24 hours to produce a block. The block was calcium sulfate dihydrate containing calcium sulfate hemihydrate.

<Calcium sulfate hemihydrate production process>

The block was pulverized and sifted to produce granules having a minor diameter of 100 to 210 $\mu$m. The granules were heat treated at 120°C and thereby dehydrated. From XRD analysis, the composition was confirmed to be pure calcium sulfate hemihydrate. The remaining materials after acid dissolution were 0 mass%, and the volume of representative granules was $1.8 \times 10^{-12}$ m$^3$. It was thus confirmed that a medical calcium sulfate composition was able to be produced.

<Placement process>

The calcium sulfate hemihydrate granules were placed in a split reaction vessel having a diameter of 6 mm and a height of 9 mm. The upper and lower openings of the reaction vessel were covered with glass plates, and the reaction vessel was secured with a C-clamp. The bulk volume of the calcium sulfate hemihydrate granules was set to 120% with respect to the volume of the reaction vessel.

<Porous structure forming process>

Subsequently, the reaction vessel was immersed in water. Water was introduced into the reaction vessel through the gap between the glass plates and the split reaction vessel so that the calcium sulfate hemihydrate granules were hydration hardened to produce a granule bonded-porous structure. As a result of XRD analysis, calcium sulfate dihydrate was confirmed to be formed. The produced calcium sulfate dihydrate porous structure had compressive strength of 1.2 MPa.

<Heat-treatment process>

For the purpose of improving the mechanical strength of the produced calcium sulfate dihydrate granule bonded-porous structure, the porous structure was heated to 900°C at 1°C/min and heat treated at 900°C for 6 hours. As a result of XRD analysis, calcium sulfate anhydrous was confirmed to be formed.

<Carbonation process>

Subsequently, the calcium sulfate anhydrous porous structure was immersed in 1 mol/L aqueous sodium carbonate solution of 80°C for 4 days. The volume was $2.8 \times 10^{-8}$ m$^3$. Figure 14 shows a scanning electron microscope image. From the scanning electron microscope image and microCT analysis, a granule bonded-porous structure having a maximum diameter of 110 to 230 μm was confirmed. From XRD analysis, the composition was confirmed to be calcite. The porosity was 58%. Although standard compressive strength was 9.7 MPa, the compressive strength was 7.9 MPa. The volume of 10 μm or smaller pores in the granule bonded-porous structure analyzed by mercury intrusion porosimetry was 0.65 cm$^3$/g. The remaining materials after acid dissolution were tested and consequently were 0 mass%. Therefore, a pure medical calcium carbonate porous structure was found to be produced.

<Phosphoric acid salt addition process>

Subsequently, the medical calcium carbonate porous structure was immersed in 0.1 mol/L disodium hydrogen phosphate of 60°C for 14 days.
From a scanning electron microscope image and microCT analysis, a granule bonded-porous structure comprising a plurality of granules which maximum diameter was approximately 110 to 230 μm, bonded to each other, and comprising a plurality of through-holes extending in plural directions was found to be produced. The volume was $2.8 \times 10^{-8}$ m$^3$. From XRD analysis pattern and infrared spectroscopic spectra, the composition was confirmed to be pure carbonate apatite. The porosity was 65%, and the compressive strength was 4.9 MPa. The volume of 10 μm or smaller pores in the granule bonded-porous structure analyzed by mercury intrusion porosimetry was 0.42 cm$^3$/g. Thus, a medical carbonate apatite porous structure was found to be produced.

(Example 18)

<Granule bonded-porous structure forming process: production of polymer material-containing raw material calcium composition granule bonded-porous structure>

A calcium hydroxide powder and acrylic resin (Dianal BR-105 manufactured by Mitsubishi Chemical Corp.) were mixed at a ratio of 45:55 and mixed at 170°C for 2 hours. The mixture was pulverized and sifted to produce polymer material-containing calcium hydroxide granules having a minor diameter of 100 μm or larger and 150 μm or smaller.

<Placement process>

Subsequently, the polymer material-containing calcium hydroxide granules were introduced to a reaction vessel having a diameter of 6 mm and a height of 3 mm such that the bulk volume of the granules was 150% with respect to the volume of the reaction vessel. The opening of the reaction vessel was closed.

<Granules bonding process>

Subsequently, the reaction vessel was heated at 150°C for 3 hours so as to soften the polymer material-containing calcium hydroxide granules. Since the reaction vessel was charged with the mixture granules at 150% of the reaction vessel volume, compressive stress was applied to between the mixture granules. In this state, the granules were thermally softened and therefore, a granule bonded-porous structure comprising a plurality of granules formed by being bonded to each other, and comprising a plurality of through-holes extending in plural directions was produced.

<Debindering and carbonation process>

Subsequently, the porous structure was placed in a glass tube having an internal diameter of 10 mm, heated to 650°C at 0.5°C/min under atmosphere containing oxygen flowed at 100 mL/min and carbon dioxide flowed at 400 mL/min, then heat treated at 650°C for 24 hours, and then cooled to room temperature at 5°C/min.
From XRD analysis, the produced porous structure was confirmed to be pure calcite. The remaining materials after acid dissolution were 0 mass%. The porosity was 48%, and the volume of 10 $\mu$m or smaller pores in the granule bonded-porous structure analyzed by mercury intrusion porosimetry was 0.12 cm$^3$/g. Although standard compressive strength was calculated to be 19 MPa, the compressive strength of the composition was 29 MPa and was thus confirmed to be over the standard compressive strength. Thus, a medical calcite porous structure was confirmed to be produced.

<Phosphorylation process>

Subsequently, the medical calcite porous structure was immersed in 2 mol/L disodium hydrogen phosphate of 80°C for 28 days.

From XRD analysis and infrared spectroscopic spectra, the composition was confirmed to be pure carbonate apatite. The remaining materials after acid dissolution were 0 mass%. The porosity was 48%, and the volume of 10 $\mu$m or smaller pores in the granule bonded-porous structure analyzed by mercury intrusion porosimetry was 0.08 cm$^3$/g. Although standard compressive strength was calculated to be 19 MPa, the compressive strength of the composition was 24 MPa and was thus confirmed to be over the standard compressive strength. Thus, a medical carbonate apatite porous structure was confirmed to be produced.

(Example 19)

The same placement process as in Example 18 was performed using the same polymer material-containing calcium hydroxide granules as in Example 18.

<Granules bonding process>

Then, the polymer material-containing calcium hydroxide granules, together with the reaction vessel, were immersed in plasticizer methyl ethyl ketone. 5 seconds later, the reaction vessel was taken out of methyl ethyl ketone, and an excess of methyl ethyl ketone in the reaction vessel was allowed to infiltrate filter paper and thereby removed. By this process, a granule bonded-porous structure comprising a plurality of through-holes extending in plural directions, formed by fusing of the surface of the granules to bond the surface of a plurality of granules one to another, was produced.

<Debindering and carbonation process>

Subsequently, the same debindering and carbonation process as in Example 18 was performed.

From XRD analysis, the produced porous structure was confirmed to be pure calcite. The remaining materials after acid dissolution were 0 mass%. The porosity was 47%, and the volume of 10 $\mu$m or smaller pores in the granule bonded-porous structure analyzed by mercury intrusion porosimetry was 0.12 cm$^3$/g. Although standard compressive strength was calculated to be 21 MPa, the compressive strength of the composition was 33 MPa and was thus confirmed to be over the standard compressive strength. Thus, a medical calcite porous structure was confirmed to be produced.

(Example 20)

Production was performed by the same operation as in Example 19 except that 3 vol% solution of plasticizer dibutyl terephthalate in normal hexane was used instead of methyl ethyl ketone used in Example 19.

From XRD analysis, the produced porous structure was confirmed to be pure calcite. The remaining materials after acid dissolution were 0 mass%. The porosity was 51%, and the volume of 10 $\mu$m or smaller pores in the granule bonded-porous structure analyzed by mercury intrusion porosimetry was 0.14 cm$^3$/g. Although standard compressive strength was calculated to be 16 MPa, the compressive strength of the composition was 22 MPa and was thus confirmed to be over the standard compressive strength. Thus, a medical calcite porous structure was confirmed to be produced.

(Example 21)

50 mg of the carbonate apatite granules produced in Example 2 was immersed in 0.15 mL of water containing 1.5 $\mu$g of dissolved fibroblast growth factor (FGF-2) manufactured by Kaken Pharmaceutical Co., Ltd. at 0°C for 120 minutes. The amount of FGF-2 adsorbed to the carbonate apatite granules was quantified by the BCA method and consequently was 0.87 $\mu$g. The carbonate apatite granules with FGF-2 adsorbed thereto were freeze dried at -80°C and then immersed in physiological saline of 37°C. As a result, FGF-2 was chronologically desorbed, and the amount of FGF-2 desorbed 12 hours later was 1.8% of the amount of FGF-2 adsorbed. From these results, the carbonate apatite of the present invention was confirmed to be useful as a sustained drug release carrier.

(Comparative Example 12)

Hydroxyapatite (HAP-200) manufactured by Taihei Chemical Industrial Co., Ltd. was compacted at 50 MPa and sintered at 1200°C for 12 hours. The sintered body was pulverized to produce sintered hydroxyapatite granules that passed through a sieve having an opening of 2 mm and did not pass through an opening of 1.18 mm. The sintered hydroxyapatite granules were a material that was not included in the present invention.

Then, the same process as in Example 21 was performed. The amount of FGF-2 adsorbed to the hydroxyapatite granules was 0.63 $\mu$g. The carbonate apatite granules with FGF-2 adsorbed thereto were freeze dried at -80°C and then immersed in physiological saline of 37°C. As a result, FGF-2 was chronologically desorbed, and the amount of FGF-2 desorbed 12 hours later was 3.2% of the amount of FGF-2 adsorbed.

As is evident from the comparison of Example 21 to this Comparative Example, the carbonate apatite granules of Example 21 supported a drug in a larger amount and desorbed the drug in a smaller amount. Specifically, the sustained drug release carrier of Example 21 was found to support a drug in a large amount. The amount of the drug desorbed by immersion in physiological saline was small, revealing that it had long-term sustained drug release and support performance.

(Example 22)

The calcium carbonate powders used were Calmaru manufactured by Sakai Chemical Industry Co., Ltd. having Mg content of $1.8 \times 10^{-5}$ mass%, Sr content of $8 \times 10^{-3}$ mass%, a mean particle diameter of 5 $\mu$m, and sphericity of 0.98; a powder of magnesium or strontium solid solution having Mg content of $1.8 \times 10^{-5}$ mass% or Sr content of $8 \times 10^{-3}$ mass% in the high-purity calcium carbonate powder manufactured by Shiraishi Central Laboratories Co., Ltd. used in Example 7; and a calcium carbonate powder having Mg content of $2 \times 10^{-5}$ mass% or smaller, Sr content of $1 \times 10^{-4}$ mass% or smaller, a mean particle diameter of 5 $\mu$m, and sphericity of 0.98 produced from calcium hydroxide (Ube Material Industries, Ltd.) in accordance with the section about calcium carbonate production disclosed in Japanese Patent Laid-Open Publication No. 2016-30708. A calcite honeycomb structure was produced by the same production method as in Example 7 except that the final temperature was set to 600°C. The samples were designated as Calmaru, Sr solid solution, Mg solid solution, and spherical, respectively.

From XRD analysis after <(E5) Debindering and calcium carbonate sintering process>, all the samples were confirmed to consist of calcite.

The calcite composition produced from the Calmaru had Mg content of $1.8 \times 10^{-5}$ mass% and Sr content of $8 \times 10^{-3}$ mass%, which did not change from the values in the raw material. The mean particle diameter based on grain boundary as an interface was 4.8 $\mu$m, and the sphericity was 0.98. The calcite composition produced from the Mg solid solution had Mg content of $1.8 \times 10^{-5}$ mass%, which did not change from the value in the raw material. The calcite composition produced from the Sr solid solution had Sr content of $8 \times 10^{-3}$ mass%, which did not change from the value in the raw material. The calcite composition produced from the spherical sample had sphericity of 0.98 based on grain boundary as an interface, which did not change from the value in the raw material.

Table 6 summarizes porosity, standard compressive strength with respect to the porosity, compressive strength, and the volume of pores which pore diameter is 10 $\mu$m or smaller with respect to a mass of the honeycomb structure analyzed by mercury intrusion porosimetry in the produced calcite honeycomb structure. The remaining materials after acid dissolution were 0 mass% in all the specimens, and no marked grain growth was found.

[Table 6]

| Properties of calcite honeycomb structure and carbonate apatite honeycomb structure produced in Example 22 | | | | | | | | | |
|---|---|---|---|---|---|---|---|---|---|
| Sample | Medical calcite honeycomb structure | | | | Carbonate apatite honeycomb structure | | | | |
| | Porosity (%) | Standard compressive strength (Mpa) | Compressive strength (MPa) | Volume of 10 $\mu$m or s maller pore (cm$^3$/g) | Compositional transformation into apatite (days) | Porosity (%) | Standard compressive strength (Mpa) | Compressive strength (MPa) | Volume of 10 $\mu$m or s maller pore (cm$^3$/g) |
| Calmaru | 59 | 9 | 41 | 0.29 | <7 | 56 | 11 | 58 | 0.19 |
| Sr solid solution | 45 | 23 | 34 | 0.18 | <7 | 37 | 40 | 48 | 0.12 |
| Mg solid solution | 44 | 25 | 33 | 0.17 | <7 | 34 | 50 | 45 | 0.11 |
| Spherical | 54 | 13 | 35 | 0.24 | <7 | 48 | 19 | 50 | 0.16 |

In Comparative Example 4, the volume of 10 μm or smaller pores was as very small as 0.02 cm³/g. By contrast, all the specimens derived from the Calmaru, Sr solid solution, Mg solid solution, and spherical samples were found to have a large volume of the pores and large compressive strength. These results demonstrated that a medical calcium carbonate honeycomb structure that satisfies not only (E) and (I) but also each of (AJ1) to (AJ4), wherein a volume of 10 μm or smaller pores is larger than 0.02 cm³/g, can be produced by using a calcium carbonate powder that satisfies (R1) to (R4) even if the final temperature is set to 600°C.

<Phosphoric acid component addition process>

Subsequently, the medical calcite honeycomb structure was immersed in 1 mol/L aqueous $Na_2HPO_4$ solution of 80°C and pH 8.9 for 7 days. This process is a production process that satisfies any of (AI1) to (AI4). Despite production at the final temperature set to 600°C, it was confirmed by XRD analysis and FT-IR analysis that the medical calcium carbonate honeycomb structure that satisfies any of the (AI1) to (AI4) conditions was compositionally transformed into pure carbonate apatite that satisfied any of the (W4) to (W8) conditions by immersion for 7 days. The carbonate apatite composition produced from the Calmaru had Mg content of $1.6 \times 10^{-5}$ mass% and Sr content of $7 \times 10^{-3}$ mass%, which were slightly decreased from the values in the raw material. The mean particle diameter based on grain boundary as an interface was 4.8 μm, and the sphericity was 0.98. The calcite composition produced from the Mg solid solution had Mg content of $1.5 \times 10^{-5}$ mass%, which was slightly decreased from the value in the raw material. The calcite composition produced from the Sr solid solution had Sr content of $7 \times 10^{-3}$ mass%, which was slightly decreased from the value in the raw material. The calcite composition produced from the spherical sample had sphericity of 0.98 based on grain boundary as an interface, which did not change from the value in the raw material. The carbonate content was 10.8 mass% in all the specimens. The remaining materials after acid dissolution were 0 mass% in all the specimens. The medical carbonate apatite honeycomb structure was also found to exhibit compressive strength larger than standard compressive strength.

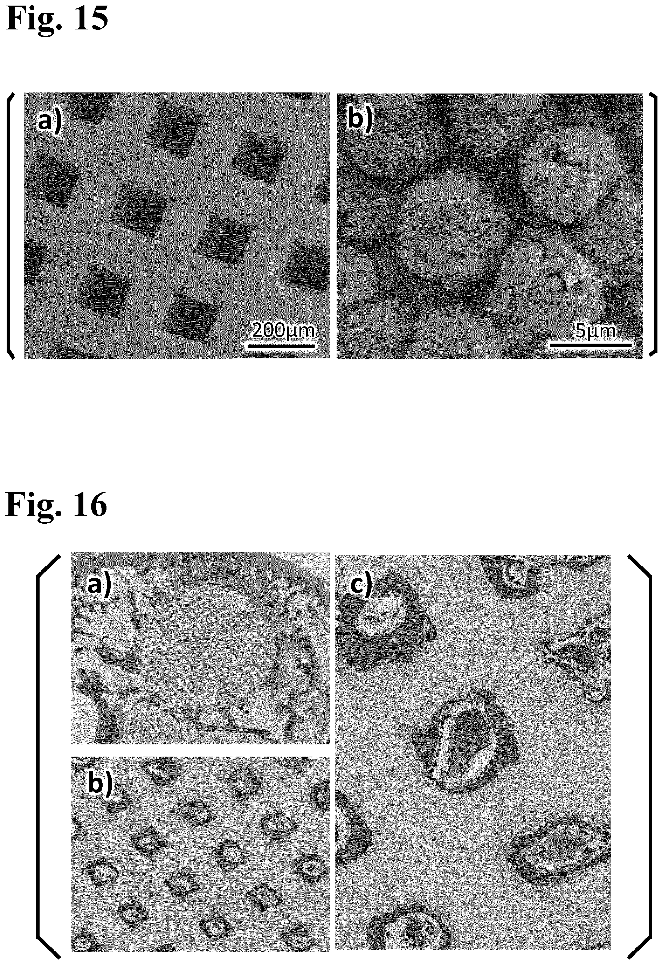

In order to verify the usefulness of the produced medical carbonate apatite honeycomb structure as a bone graft material in rabbits, it was implanted in the distal end of the rabbit femur. Figure 16 shows a histopathological image obtained by hematoxylin-eosin staining on week 4 after implantation. High osteoconductivity can be confirmed from bone conduction up to the central part. Bone formation was found inside all the through-holes, and the area of the formed bones with respect to the pore area was 55%, which was larger than the area of the formed bones in the medical carbonate apatite honeycomb structures produced in Examples 9 and 14.

(Example 23)

<Raw material calcium production process>

The calcium carbonate powder used was Calmaru manufactured by Sakai Chemical Industry Co., Ltd. Calmaru was mixed with silver phosphate such that the silver phosphate content was 0 to 20 mass%. The mixture was compacted at 300 MPa to produce a cylindrical compact having a diameter of 8 mm and a height of 4 mm. The compact was heated to 350°C at 5°C/min and kept for 12 hours for sintering. The material thus heat treated was immersed in saturated aqueous solution of calcium carbonate in 10 times the amount of the material in a glass container and sonicated for 1 minute under conditions involving 28 kHz and an output of 75 W. As a result of comparing the dry weight of the composition thus irradiated to the dry weight before the ultrasonic irradiation, the ratio was 100% in all the specimens. As a result of powder XRD analysis, the polymorph of calcium carbonate was confirmed to be vaterite. As for the samples having silver phosphate content of less than 1 mass%, the remaining materials after acid dissolution were less than 1 mass%. A vaterite block having a volume of $2 \times 10^{-7}$ m³ was able to be produced. The diametral tensile strength was 3 MPa, regardless of the content of silver phosphate, and calculated compressive strength was 15 MPa.

<Process of exposure to aqueous phosphoric acid salt solution>

The vaterite block was immersed in 1 mol/L aqueous $Na_2HPO_4$ solution at 80°C for 7 days. As a result of XRD analysis and FT-IR analysis, compositional transformation into carbonate apatite having carbonate content of 10.8 mass% was found. The diametral tensile strength was 5 MPa, regardless of the content of silver phosphate, and calculated compressive strength was 25 MPa. The concentration of silver phosphate contained in the carbonate apatite composition was 90% which was the original value of the content presumably because the carbonate apatite composition was produced by the addition of phosphoric acid to calcium carbonate. As for the samples derived from the raw material vaterite composition having silver phosphate content of less than 1 mass%, the remaining materials after acid dissolution were less than 1 mass%. The carbonate apatite composition had a volume of $2 \times 10^{-7}$ m³.

<Antimicrobial property test and cytotoxicity test>

The produced silver phosphate-containing carbonate apatite composition was evaluated for its antimicrobial properties by the film covering method. As a result, *Staphylococcus epidermidis* counts for the silver phosphate-containing carbonate apatite compositions having silver phosphate contents of 0 mass%, 0.009 mass%, and 0.09 mass% were $2 \times 10^6$ CFU/mL, $4 \times 10^4$ CFU/mL, and $6 \times 10^3$ CFU/mL, respectively. A *Staphylococcus epidermidis* count for the silver phosphate-containing carbonate apatite composition that contained 0.9 mass% or larger of silver phosphate was $1 \times 10^3$ CFU/mL or lower. Thus, all the silver phosphate-containing carbonate apatite composition samples were confirmed to exhibit an antimicrobial effect.

The produced silver phosphate-containing carbonate apatite composition was also evaluated for its tissue affinity by the cytotoxicity test and resulted in $4000/cm^2$ for the silver phosphate content of 0 mass%, $4000/cm^2$ for 0.09 mass%, $3600/cm^2$ for 0.9 mass%, $3000/cm^2$ for 3.0 mass%, and $200/cm^2$ for 4.5 mass%.

Thus, the silver phosphate-containing carbonate apatite composition that contained 0.01 mass% or larger and 3 mass% or smaller of silver phosphate was confirmed to be a medical carbonate apatite composition having both of an antimicrobial effect and tissue affinity.

(Example 24)

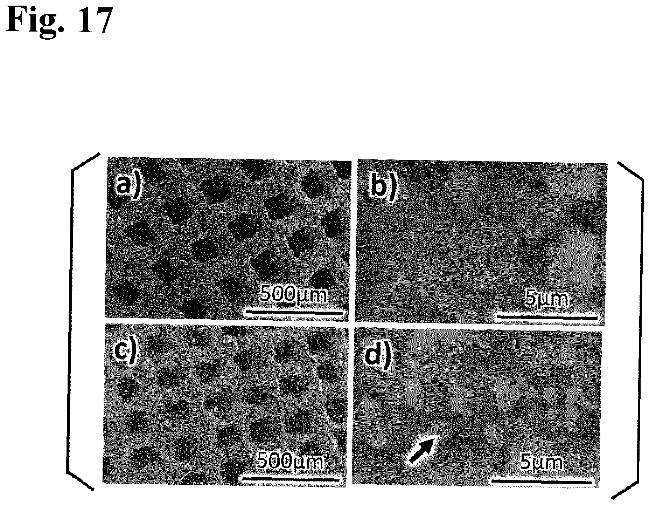

The medical carbonate apatite honeycomb structure of Example 14 produced from the raw material calcium composition containing calcium sulfate at a volume ratio of 50% with respect to a wax-based polymer material was immersed in 0.1 to 5 mmol/L aqueous silver nitrate solution of 25°C for 1 hour. Figure 17 shows a SEM image of the structure before (a and b) and after (c and d) immersion in 1 mmol/L aqueous silver nitrate solution. When the structure was immersed in the aqueous silver nitrate solution, it was found that the honeycomb structure was maintained and crystals were bonded and deposited on the surface of the structure (arrow in Figure 17d). As a result of X-ray photoelectron spectrometry, the crystals were found to be silver phosphate. The amount of silver phosphate formed was 0.04 mass% for the 0.1 mmol/L aqueous silver nitrate solution, 0.2 mass% for the 0.5 mmol/L aqueous silver nitrate solution, 0.4 mass% for the 1 mmol/L aqueous silver nitrate solution, and 2 mass% for the 5 mmol/L aqueous silver nitrate solution.

The medical carbonate apatite honeycomb structure immersed in the 0.1 mmol/L aqueous silver nitrate solution was taken out thereof 1 hour later and immersed in 0.5 mmol/L aqueous silver nitrate solution for 10 minutes. The honeycomb structure was cut in a direction vertical to the pores, and the silver concentration measured at the surface of the honeycomb structure was 3.2 times the silver concentration at a site of 75 $\mu$m from the surface.

The carbonate apatite honeycomb structure had porosity of 50% and compressive strength of 12 MPa in the pore direction. The volume of 10 $\mu$m or smaller pores was 0.34 cm$^3$/g.

The carbonate apatite honeycomb structure was pulverized using a cutting mill manufactured by Fritsch Japan Co., Ltd., and carbonate apatite honeycomb structure granules having a minor diameter of 1 mm or larger and shorter than 5 mm were produced using sieves having opening diameters of 1 mm and 5 mm. The honeycomb structure was strongly anisotropic and therefore had many sharply angled portions at the stage of pulverization using the cutting mill. Specifically, when a circle with a radius of 0.2 mm from any point on a peripheral line of a perspective image was depicted, and at a triangle formed by three points: the vertex point on the peripheral line and two points made by an intersection of the circle and a line of perspective image, a vertex point that the interior angle was 90° or smaller at the triangle existed in the pulverized product. Accordingly, the granules were placed on a sieve having an opening of 0.25 mm and shaken in a sieve shaker manufactured by AS ONE Corp. The sharply angled portions were removed from the granules thus shaken for 3 hours. When a circle with a radius of 0.2 mm from any point on a peripheral line of a perspective image was depicted, and at a triangle formed by three points: the vertex point on the peripheral line and two points made by an intersection of the circle and a line of perspective image, the vertex point that the interior angle was 90° or smaller at the triangle was confirmed to no longer exist.

(Example 25)

The solid portion of a defect reconstruction kit was produced by mixing an aTCP powder (a-TCP-B manufactured by Taihei Chemical Industrial Co., Ltd.) with a vaterite powder (Calmaru manufactured by Sakai Chemical Industry Co., Ltd.) having a mean particle diameter of 5 $\mu$m at a molar ratio of 1:1 (vaterite content: 24 mass%). The liquid portion thereof was produced by mixing 1 mol/L disodium hydrogen phosphate with 1 mol/L aqueous sodium dihydrogen phosphate solution such that pH was 7.0.

The solid portion and the liquid portion of the defect reconstruction kit were kneaded at a mass ratio of 1:0.4, and the setting time of the resulting paste was 10 minutes. The diametral tensile strength obtained 24 hours later was 4 MPa. It was found that carbonate apatite was formed by consuming 65 mass% of vaterite and 18% of aTCP. The ratio of the amount of vaterite consumed to the amount of aTCP consumed was 3.6. The paste immediately after kneading was found to collapse slowly when immersed in water.

(Example 26)

The same defect reconstruction kit as in Example A1 was produced except that carboxymethylcellulose sodium (manufactured by FUJIFILM Wako Pure Chemical Corp.) was added at 0.1 mass% to the liquid portion of Example 25.
The solid portion and the liquid portion of the defect reconstruction kit were kneaded at a mass ratio of 1:0.4, and the resulting paste had better handleability than that of the paste of Example A1, probably because of its viscosity. The setting time of the paste was 10 minutes. The diametral tensile strength obtained 24 hours later was 4 MPa. It was found that carbonate apatite was formed by consuming 64 mass% of vaterite and 17% of aTCP. The ratio of the amount of vaterite consumed to the amount of aTCP consumed was 3.8. The paste immediately after kneading was found to be able to maintain morphology, albeit collapsing, when immersed in water, as compared with Example 25.
From the comparison of Example 25 to this Example, it was found that the improved viscosity of the liquid portion improves the handleability of paste or paste's property of retaining its shape against water and does not largely influence setting time, the mechanical strength or composition of the setting product, etc.

(Example 27)

The same defect reconstruction kit as in Example 26 was produced except that citric acid (manufactured by FUJIFILM Wako Pure Chemical Corp.) was added at 0.2 mass% to the liquid portion of Example 26.
The solid portion and the liquid portion of the defect reconstruction kit were kneaded at a mass ratio of 1:0.4, and the resulting paste had better handleability than that of the paste of Example 25, probably because of its viscosity. The setting time of the paste was 5 minutes. The paste immediately after kneading was found to be able to maintain morphology without collapsing when immersed in water.
The diametral tensile strength obtained 24 hours later was 6 MPa. It was found that carbonate apatite was formed by consuming 71 mass% of vaterite and 33% of aTCP. The ratio of the amount of vaterite consumed to the amount of aTCP consumed was 2.2.
From the comparison of Examples 25 and 26 to this Example, it was found that added citric acid having a plurality of carboxy groups shortens setting time, enhances the mechanical strength of the setting product, and improves paste's property of retaining its shape against water.

(Example 28)

The sintered vaterite block produced by sintering at 350°C in Example 6 was pulverized, and vaterite granules having a minor diameter of 150 $\mu$m or larger and shorter than 200 $\mu$m were produced using sieves having opening diameters of 150 $\mu$m and 200 $\mu$m. The same defect reconstruction kit as in Example 27 was produced except that the vaterite granules were added at 10 mass% to the solid portion of Example 27.
The solid portion and the liquid portion of the defect reconstruction kit were kneaded at a mass ratio of 1:0.4, and the resulting paste was slightly inferior in handleability, albeit of no clinical importance, to the paste of Example 27, presumably due to the vaterite granules. The setting time of the paste was 5 minutes. The diametral tensile strength obtained 24 hours later was 7 MPa. It was found that carbonate apatite was formed by consuming 60 mass% of vaterite and 33% of aTCP. The ratio of the amount of vaterite consumed to the amount of aTCP consumed was 1.8.
From the comparison of Example 27 to this Example, it was found that the mechanical strength of the setting product is enhanced when vaterite having a volume of $10^{-12}$ $m^3$ or larger is contained in the solid portion.

(Comparative Example 13)

The vaterite powder (Calmaru) used in Example 25 was heat treated at 400°C for 48 hours to produce a calcite powder in the same shape. The mean particle diameter was 5 $\mu$m. A bone defect reconstruction kit was produced under the same conditions as in Example 25 except that calcite was used instead of vaterite as calcium carbonate in the solid portion. Kneading and setting were also performed under the same conditions as in Example 25.
The solid portion and the liquid portion of the defect reconstruction kit were kneaded at a mass ratio of 1:0.4, and the resulting paste had the same handleability as in Example 25. The setting time of the paste was 10 minutes. The diametral tensile strength obtained 24 hours later was 3 MPa. It was found that carbonate apatite was formed by consuming 32 mass% of calcite and 25% of aTCP. The ratio of the amount of vaterite consumed to the amount of aTCP consumed was 1.3.
From the comparison of Example 25 to this Comparative Example, it was found that although no marked different is found in setting time between vaterite and calcite used as calcium carbonate, the ratio to the amount of aTCP consumed is larger in vaterite having high solubility, resulting in a large amount of carbonate apatite or a carbonate apatite setting product having large carbonate content. The kit obtained using vaterite was also found to have larger diametral tensile strength, presumably because of the large amount of carbonate apatite formed.

(Comparative Example 14)

A bone defect reconstruction kit was produced under the same conditions as in Example 27 except that the calcite powder produced in Comparative Example 13 was used. Kneading and setting were also performed under the same conditions as in Example 27.

The solid portion and the liquid portion of the defect reconstruction kit were kneaded at a mass ratio of 1:0.4, and the resulting paste had handleability equivalent to Example 27 and better handleability than that of the paste of Comparative Example 13, presumably because of its viscosity. The setting time of the paste was 5 minutes. The diametral tensile strength obtained 24 hours later was 3 MPa. It was found that carbonate apatite was formed by consuming 28 mass% of calcite and 33% of aTCP. The ratio of the amount of vaterite consumed to the amount of aTCP consumed was 0.8.

From the comparison of Example 27 to this Comparative Example, it was found that although no marked different is found in setting time between vaterite and calcite used as calcium carbonate even if carboxymethylcellulose sodium and citric acid having a plurality of carboxy groups are added to the liquid portion, the ratio to the amount of aTCP consumed is larger in vaterite having high solubility, resulting in a large amount of carbonate apatite or a carbonate apatite setting product having large carbonate content. The kit obtained using vaterite was also found to have larger diametral tensile strength, presumably because of the large amount of carbonate apatite formed.

(Example 29)

The same defect reconstruction kit as in Example 25 was produced except that the vaterite powder used was the vaterite powder having a mean particle diameter of 1 $\mu$m produced in Example 6.

The solid portion and the liquid portion of the defect reconstruction kit were kneaded at a mass ratio of 1:0.4, and the setting time of the resulting paste was 5 minutes. The diametral tensile strength obtained 24 hours later was 4 MPa. It was found that carbonate apatite was formed by consuming 57 mass% of vaterite and 15% of aTCP. The ratio of the amount of vaterite consumed to the amount of aTCP consumed was 3.8.

From the comparison of Example 25 to this Example, it was found that use of a vaterite powder having a smaller mean particle diameter shortens setting time.

(Comparative Example 15)

The vaterite powder used in Example 29 was heat treated at 400°C for 48 hours to produce a calcite powder in the same shape. The mean particle diameter was 1 $\mu$m.

The same defect reconstruction kit as in Example 29 was produced except that the calcite powder was used.

The solid portion and the liquid portion of the defect reconstruction kit were kneaded at a mass ratio of 1:0.4, and the setting time of the resulting paste was 5 minutes. The diametral tensile strength obtained 24 hours later was 3 MPa. It was found that carbonate apatite was formed by consuming 17 mass% of vaterite and 30% of aTCP. The ratio of the amount of vaterite consumed to the amount of aTCP consumed was 0.6.

From the comparison of Example 29 to this Example, it was found that although no marked different is found in setting time between vaterite and calcite used as calcium carbonate, the ratio to the amount of aTCP consumed is larger in vaterite having high solubility, resulting in a large amount of carbonate apatite or a carbonate apatite setting product having large carbonate content. The kit obtained using vaterite was also found to have larger diametral tensile strength, presumably because of the large amount of carbonate apatite formed.

**Claims**

1. A medical calcium carbonate composition that satisfies all conditions of the following (A) - (C), and at least one condition selected from the group consisting of (D) - (K):

    (A) a volume is $10^{-12}m^3$ or larger;
    (B) remaining materials after acid dissolution are 1.0 mass% or less.
    (C) it is substantially a pure calcium carbonate as a medical composition, and mainly comprises vaterite or calcite;
    (D) it contains 20 mass% or larger of vaterite;

(E) it is a honeycomb structure comprising a plurality of through-holes extending in one direction, wherein a volume of pores which pore diameter is 10 $\mu$m or smaller with respect to a mass of the honeycomb structure analyzed by mercury intrusion porosimetry is larger than 0.02 cm$^3$/g;

(F) it is a granule bonded-porous structure comprising a plurality of granules which maximum diameter is 50 $\mu$m or longer and 500 $\mu$m or shorter, formed by being bonded to each other, and comprising a plurality of through-holes extending in plural directions, wherein a volume of pores with a pore diameter of 10 $\mu$m or smaller analyzed by mercury intrusion porosimetry is 0.05 cm$^3$/g or more;

(G) it is a pore integrated-type porous structure wherein a plurality of pores which maximum diameter is 50 $\mu$m or longer and 400 $\mu$m or shorter is integrated to the whole medical composition, not containing pores which maximum diameter is 800 $\mu$m or longer, wherein a volume of pores which maximum diameter is 10 $\mu$m or smaller in the pore integrated-type porous structure analyzed by mercury intrusion porosimetry is 0.05 cm$^3$/g or more;

(H) a ratio of pore volume which pore diameter is 1 $\mu$m or larger and 6 $\mu$m or shorter with respect to a pore volume which pore diameter is 6 $\mu$m or shorter analyzed by mercury intrusion porosimetry is 10 % or more;

(I) a maximum compressive strength obtained at any one direction is higher than a standard compressive strength [S] that is calculated by the following equation (with the proviso that a honeycomb structure comprising a plurality of through-holes extending in one direction, wherein a volume of pores with a pore diameter of 10 $\mu$m or smaller with respect to a mass of the honeycomb structure analyzed by mercury intrusion porosimetry is 0.02 cm$^3$/g or smaller is excluded)

$$S = S_0 \times C \times \exp(-b \times P)$$

(wherein $S_0$ and b are constant number, $S_0$ is 500, b is 0.068, and C is a constant number based on polymorph of calcium carbonate; C is 0.01 when the calcium carbonate contains 20 mass% or larger of vaterite, and is 1 when the calcium carbonate does not contain 20 mass% or larger of vaterite; and P is a percentage of pores in the composition.)

(J) it is a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists;

(K) plural composition particles are connected with a fiber.

2. The medical calcium carbonate composition according to claim 1, wherein the above described (D) condition is satisfied, and the medical calcium carbonate composition is a sintered body.

3. The medical calcium carbonate composition according to claim 1 or 2, wherein calcium carbonate powders that satisfy at least one of the following (AJ1) to (AJ4) conditions, are bonded to form the calcium carbonate composition:

(AJ1) a mean particle diameter is 2 $\mu$m or larger, and 8 $\mu$m or smaller;
(AJ2) a sphericity is 0.9 or larger.
(AJ3) Mg content is $5 \times 10^{-4}$ mass% or larger, and $3 \times 10^{-3}$ mass% or smaller;
(AJ4) Sr content is $3 \times 10^{-3}$ mass% or larger, and $1.5 \times 10^{-2}$ mass% or smaller.

4. The medical calcium carbonate composition according to claim 1 or 3, wherein the above described (E) condition is satisfied, which is a bended honeycomb structure wherein a diameter of the circle that passes through both ends of any one of the through-holes and a center of the through-hole, is 1 cm or longer, and 50 cm or shorter.

5. A method for producing the medical calcium carbonate composition according to claim 1 or 3 that satisfies the above described (D) condition, wherein:
in a process of exposing raw material calcium composition which volume is $10^{-12}$m$^3$ or larger to carbon dioxide or carbonate ion, at least one of the following conditions selected from (D1) to (D8) group is satisfied, and optionally comprises the following (D9) to (D12) process:

(D1) a process of inhibiting calcite formation or calcite crystal growth, and relatively promoting vaterite formation;
(D2) a process of exposing of raw material calcium composition to carbon dioxide or carbonate ion, and at least one selected from the group consisting of organic solvent, water soluble organic material, ammonia, and ammonium salt;

(D3) a process of exposing raw material calcium composition that contains at least one selected from the group consisting of organic solvent, water soluble organic material, ammonia, and ammonium salt, to carbon dioxide or carbonate ion, and at least one selected from the group consisting of organic solvent, water soluble organic material, ammonia, and ammonium salt;

(D4) a process of exposing raw material calcium composition to carbon dioxide or carbonate ion, and at least one selected from the group consisting of methanol, ethanol, glycerin, ethylene glycol, and ammonium carbonate.

(D5) a process of exposing raw material calcium composition that contains at least one selected from the group consisting of methanol, ethanol, glycerin, ethylene glycol, and ammonium carbonate, to carbon dioxide or carbonate ion, and at least one selected from the group consisting of methanol, ethanol, glycerin, ethylene glycol, and ammonium carbonate;

(D6) a process of inhibiting transfer from vaterite to calcite;

(D7) a process of removing water from the raw material calcium composition;

(D8) a process of circulating carbon dioxide or carbonate ion containing organic solvent around the raw material calcium composition;

(D9) a process of partial carbonation by exposing raw material calcium composition to carbon dioxide or carbonate ion under gas phase, followed by exposing the raw material calcium composition to carbon dioxide or carbonate ion under liquid phase;

(D10) a process of exposing raw material calcium composition in a mold to carbon dioxide or carbonate ion;

(D11) a process of exposing raw material calcium composition that contains porogen to carbon dioxide or carbonate ion;

(D12) a process of exposing raw material calcium compositions that are connected with a fiber to carbon dioxide or carbonate ion;

6. A method for producing the medical calcium carbonate composition according to claim 1 or 2 that satisfies the above described (D) condition, comprising:
compacting and sintering calcium carbonate powder that contains 20 mass% or larger of vaterite.

7. A method for producing the medical calcium carbonate composition according to claim 1, 3 or 4 that satisfies the above described (E) condition, comprising:

the following process (E1) and one process selected from the group consisting of (E5) to (E9) as essential process, and optionally one process selected from the following (E2) to (E4), and (E10)

(E1) Extrusion process
a process of producing a raw honeycomb structure comprising a plurality of through-holes extending in one direction, having a volume of $3 \times 10^{-11} m^3$ or larger by extruding a raw material calcium composition comprising polymer material through a honeycomb structure forming die;

(E2) Forming process after extrusion process
A process of forming honeycomb structure consisting of a raw material calcium composition comprising polymer material to a desired form by softening by a thermal treatment, followed by pressure loading;

(E3) Removal process of peripheral wall
A process of removing peripheral wall after the extrusion process or the forming process after the extrusion process, and before a debindering and carbonation process;

(E4) Forming process after removal process of peripheral wall A process of forming a honeycomb structure consisting of a raw material calcium composition comprising polymer material to a desired form through softening by thermal treatment, after removal process of peripheral wall;

(E5) Debindering and calcium carbonate sintering process
a process of heat debindering of polymer material-containing calcium carbonate so that remaining materials after acid dissolution is 1 mass% or smaller, and sintering the calcium carbonate;

(E6) Debindering and carbonation process
a process of heat debindering of a polymer material containing-calcium hydroxide porous structure so that remaining materials after acid dissolution is 1 mass% or
smaller under an oxygen concentration of less than 30%, and carbonation at the same time;

(E7) Debindering and carbonation process via calcium oxide
a process of heat debindering a polymer material containing-calcium hydroxide porous structure or polymer material containing-calcium carbonate porous structure so that remaining materials after acid dissolution is 1 mass% or smaller, and to be calcium oxide porous structure, followed by exposing the calcium oxide porous structure to carbon dioxide to be a calcium carbonate porous structure;

(E8) Debindering and carbonation process via calcium carbonate and calcium oxide a process of heat treatment of a polymer material-containing calcium hydroxide under carbon dioxide atmosphere to be a polymer material containing-calcium carbonate porous structure, followed by heat debindering so that remaining materials after acid dissolution is 1 mass% or smaller, and to be a calcium oxide porous structure, followed by exposing the calcium oxide porous structure to carbon dioxide to be a calcium carbonate porous structure;

(E9) Debindering and carbonation process of calcium sulfate

a process of heat debindering of a polymer material containing-calcium sulfate so that remaining materials after acid dissolution is 1 mass% or smaller, followed by adding carbon dioxide or carbonate ion to the produced calcium sulfate porous structure to be a calcium carbonate;

(E10) a process of structure finishing process after debindering and carbonation processes.

8. A method for producing the medical calcium carbonate composition according to claim 7, that satisfies at least one of the conditions selected from the following conditions (E11) to (E14):

(E11) in the above-described "(E1) Extrusion process", honeycomb structure is extruded so that thickness of peripheral wall of the honeycomb structure is thicker than that of the partition wall, and cross-sectional area vertical to the through-holes is 1 cm$^2$ or larger;

(E12) in at least one of the processes of "(E1) Extrusion process", "(E2) Forming process after extrusion process", "(E4) Forming process after removal process of peripheral wall", and "(E10) Structure finishing process after debindering and carbonation processes", a heat softened honeycomb structure comprising raw material calcium composition containing polymer material is bended by applying a pressure so that the diameter of the circle that passes through both ends and the center of one of the through-holes, is 1 cm or longer, and 50 cm or shorter;

(E13) the "(E3) Removal process of peripheral wall" is done with a cutter grinder, and the "(E10) process of structure finishing process after debindering and carbonation processes" is done by polishing;

(E14) a raw material calcium composition of the "(E1) Extrusion process" is calcium sulfate anhydrous.

9. A method for producing the medical calcium carbonate composition according to claim 1 that satisfies the above described (F) condition, comprising:

the following (F1) and (F2) processes, and at least one of the (F3) or (F4) process.

(F1) Placement-closing process

placing calcium oxide granules in a reaction vessel, and closing the opening of the vessel so that the granules are not escaped from the reaction vessel;

(F2) Porous structure producing process

a process of producing a porous structure by adding water or acetic acid to the calcium oxide granules inside the reaction vessel to make calcium hydroxide or calcium acetate;

(F3) Carbonation process

a carbonation process to produce a calcium carbonate porous structure by adding carbon dioxide to calcium hydroxide porous structure at the same time or after the porous structure producing process, or a carbonation process to produce a calcium carbonate porous structure by heat treatment of calcium acetate after porous structure producing process;

(F4) Calcium oxide carbonation process

a carbonation process producing a calcium carbonate porous structure by heat treatment of at least one selected from a group consisting of calcium hydroxide porous structure, calcium carbonate porous structure, and calcium acetate porous structure, followed by exposing the calcium oxide porous structure to carbon dioxide.

10. A method for producing the medical calcium carbonate composition according to claim 1, that satisfies the above described (F) condition, and comprising the following (F5) and (F6) processes; or comprising the following (F5), (F7), and (F9) processes, and optionally comprising the (F8) process:

(F5) Placement process

a process of placing calcium sulfate granules in a reaction vessel;

(F6) Porous structure forming-carbonation process

a process of changing composition to calcium carbonate by reacting calcium sulfate granules in the reaction vessel with carbonate ion, and hardening granules each other to form porous structure;

(F7) Porous structure forming process

a process of forming calcium sulfate dihydrate porous structure by adding water to calcium sulfate hemihydrate granules or calcium sulfate anhydrous granules;

(F8) Heat-treatment process
a process of producing calcium sulfate anhydrous porous structure by heat-treatment of calcium sulfate dihydrate porous structure;
(F9) Carbonation process
a process of changing composition to calcium carbonate by exposing calcium sulfate dihydrate porous structure or calcium sulfate anhydrous porous structure to water containing carbonate ion.

11. A method for producing the medical calcium carbonate composition according to claim 1 that satisfies the above described (F) condition, comprising the following (F10), (F11) and one selected from the group of (F12) to (F16) as essential processes, and optionally comprising the (F17) process:

(F10) Placement process
a process of placing raw material calcium composition granules containing polymer having a volume of $10^{-12}$ $m^3$ or larger in a reaction vessel;
(F11) Porous structure forming process
A process of producing granules bonded-porous structure formed from a plurality of granules which maximum diameter is 50 $\mu$m or longer and 500 $\mu$m or shorter bonded to each another, and comprising a plurality of through-holes extending in plural directions, and having a volume of $3 \times 10^{-11}$ $m^3$ by bonding the granules in the reaction vessel by heat fusing, or by fusing of the surface of granules to bond the surface of the granules one to another, or by fusing the surface of granules one to another with a plasticizer.
(F12) Debindering and calcium carbonate sintering process
a process of heat debindering of polymer material containing-calcium carbonate so that remaining materials after acid dissolution are 1 mass% or smaller, and sintering the calcium carbonate;
(F13) Debindering and carbonation process
a process of heat debindering of polymer material containing calcium hydroxide porous structure so that remaining materials after acid dissolution are 1 mass% or smaller under oxygen concentration of less than 30%, and carbonation at the same time;
(F14) Debindering and carbonation process via calcium carbonate and calcium oxide a process of heat treatment of polymer material containing-calcium hydroxide porous structure or polymer material containing-calcium carbonate porous structure so that remaining materials after acid dissolution are 1 mass% or smaller, and to be calcium oxide porous structure, followed by exposing the calcium oxide porous structure to carbon dioxide to be calcium carbonate porous structure;
(F15) Debindering and carbonation process via calcium carbonate and calcium oxide Debindering and carbonation process via calcium carbonate and calcium oxide, comprising heat treatment of polymer material-containing calcium hydroxide porous structure in the presence of carbon dioxide to produce polymer material-containing calcium carbonate porous structure, followed by heat debindering so that remaining materials after acid dissolution are 1 mass% or smaller, and to be calcium oxide porous structure, followed by exposing the calcium oxide porous structure to carbon dioxide to be calcium carbonate porous structure;
(F16) Calcium sulfate debindering and carbonation process
a debindering and carbonation process of producing calcium carbonate by heat debindering of polymer material-containing calcium sulfate so that remaining materials after acid dissolution are 1 mass% or smaller, followed by adding carbon dioxide or carbonate ion to the produced calcium sulfate porous structure.
(F17) A process of structure finishing process after debindering and carbonation processes.

12. A method for producing the medical calcium carbonate composition according to claim 1 that satisfies the above described (G) condition, comprising (G1) and at least one process selected the group consisting of (D1) to (D10) and (E5) to (E9) as essential process, and optionally comprising (G2), (G3), and (E10):

(G1) Mixing process
A process of mixing raw material calcium composition powder or raw material calcium composition paste, and porogen;
(G2) Compacting process
A process of compacting raw material calcium composition powder or raw material calcium composition paste, and porogen;
(G3) Porogen removal process
A process of removing porogen by dissolving the porogen into a solvent;
(D1) A process of inhibition of calcite formation or calcite crystals' growth, and promoting relative vaterite formation;

(D2) A process comprising exposing raw material calcium composition to carbon dioxide or carbonate ion, and at least one selected from a group of organic solvent, water soluble organic material, ammonia, and ammonium salts;

(D3) A process comprising exposing raw material calcium composition that contains at least one selected from the group consisting of organic solvent, water soluble organic materials, ammonia, and ammonium salts, to carbon dioxide or carbonate ion, and at least one selected from a group of organic solvent, water soluble organic materials, ammonia, and ammonium salts;

(D4) A process comprising exposing raw material calcium composition to carbon dioxide or carbonate ion, and at least one selected from the group consisting of methanol, ethanol, and ammonium carbonate;

(D5) A process comprising exposing raw material calcium composition that contains at least one selected from the group consisting of methanol, ethanol, and ammonium carbonate, to carbon dioxide or carbonate ion, and at least one selected from the group consisting of methanol, ethanol, and ammonium carbonate;.

(D6) A process comprising inhibiting transfer from vaterite to calcite;

(D7) A process comprising removing water from the raw material calcium composition;

(D8) A process comprising circulating carbon dioxide or carbonate ion containing organic solvent around the raw material calcium composition;

(D9) A process comprising partial carbonation by exposing raw material calcium composition to carbon dioxide or carbonate ion under gas phase, followed by exposing the raw material calcium composition to carbon dioxide or carbonate ion under liquid phase;

(D10) A process comprising exposure of raw material calcium composition in a mold to carbon dioxide or carbonate ion;

(E5) Debindering and calcium carbonate sintering process
A process of heat debindering of polymer material containing-calcium carbonate so that remaining materials after acid dissolution is 1 mass% or smaller, and sintering the calcium carbonate;.

(E6) Debindering and carbonation process
A process of heat debindering polymer material containing-calcium hydroxide so that remaining materials after acid dissolution are 1 mass% or smaller under oxygen concentration of less than 30%, and carbonation at the same time.

(E7) Debindering and carbonation process via calcium oxide
A process of heat debindering polymer material containing- calcium hydroxide or calcium carbonate so that remaining materials after acid dissolution are 1 mass% or smaller, and to be calcium oxide porous structure, followed by exposing the calcium oxide porous structure to carbon dioxide to be calcium carbonate porous structure.

(E8) Debindering and carbonation process via calcium carbonate and calcium oxide
A process of heat treatment of polymer material containing-calcium hydroxide under carbon dioxide atmosphere to be polymer material containing-calcium carbonate, followed by heat debindering so that remaining materials after acid dissolution is 1 mass% or smaller, and to be calcium oxide porous structure, followed by exposing the calcium oxide porous structure to carbon dioxide to be calcium carbonate porous structure.

(E9) Debindering and carbonation process of calcium sulfate
A process of heat debindering of polymer material-containing calcium sulfate so that remaining materials after acid dissolution are 1 mass% or smaller, followed by adding carbon dioxide or carbonate ion to the produced calcium sulfate porous structure to be calcium carbonate.

(E10) A process of structure finishing process after debindering and carbonation processes.

13. A method for producing the medical calcium carbonate composition according to any one of claims 7, 8, 11 or 12, wherein:
the above described heat debindering is done at 200 °C or higher, and mass decrease of the polymer material of polymer material containing-calcium composition is smaller than 1 mass%/min,

14. A method for producing the medical calcium carbonate composition according to any one of claims 5 to 13 comprising at least one process selected from a group of below described (L) to (Q) as essential process:

(L) A process of debindering done at an oxygen partial pressure of 30 KPa or higher;

(M) A process of debindering or carbonation done at carbon dioxide partial pressure of 30 KPa or higher;

(N) A process of debindering or carbonation done at 150 KPa or higher under atmosphere that contains oxygen or carbon dioxide;

(O) A process of increasing carbon dioxide concentration in the reaction vessel by replacing air in the reaction vessel partially or completely with carbon dioxide, followed by introduction of carbon dioxide in the reaction vessel;

(P) A process of supplying carbon dioxide so that the pressure of the closed reaction vessel is a constant value;
(Q) A carbonation process of mixing or circulating carbon dioxide in the reaction vessel.

**15.** A method for producing the medical calcium carbonate composition according to any one of claims 5 to 9 and 11 to 14, wherein:
a composition of the raw material calcium composition is one selected from the group consisting of calcium oxide, calcium hydroxide, and calcium carbonate.

**16.** A method for producing the medical calcium carbonate composition according to any one of claims 5 to 15, wherein:
at least one condition selected from the following (R1) to (R4) is satisfied:

(R1) Using calcium carbonate powder with an average particle diameter of 2 $\mu$m and larger, and 8 $\mu$m and smaller;
(R2) Using calcium carbonate powder with a sphericity of 0.9 or higher;
(R3) Using calcium carbon powder containing $5 \times 10^{-4}$ mass% or larger, and $3 \times 10^{-3}$ mass% or smaller of Mg;
(R4) Using calcium carbon powder containing $3 \times 10^{-3}$ mass% or larger, and $1.5 \times 10^{-2}$ mass% or smaller of Sr.

**17.** A medical calcium sulfate setting composition that satisfies all the following (T1) to (T5) conditions.

(T1) The remaining materials after acid dissolution is 1.0 mass% or less;
(T2) The volume is $5 \times 10^{-13}$m$^3$ or larger;
(T3) it is substantially a pure calcium sulfate as a medical composition;
(T4) content of calcium sulfate hemihydrate production is 50 mass% or more;
(T5) Forming a porous structure with compressive strength of 0.3MPa or higher upon setting reaction when immersed in water while the compositions are contacted one another.

**18.** A method for producing the medical calcium sulfate setting composition according to claim 17,
comprising the following (U2) and (U3) as essential processes, and optionally comprising (U1) and/or (U4) to produce calcium sulfate hemihydrate production:

(U1) Polymer debindering process
A process of debindering polymer material-containing calcium sulfate granules or block by thermal treatment so that the remaining materials after acid dissolution are 1.0 mass% or less;
(U2) Calcium sulfate dihydrate production process
A process of producing calcium sulfate dihydrate granules or block by adding water to calcium sulfate anhydrous or hemihydrate granules or block produced by the polymer debindering process, or by adding water to calcium sulfate hemihydrate powder, and followed by hardening;
(U3) Calcium sulfate hemihydrate production process
A process of producing calcium sulfate hemihydrate granules or block by dehydration of calcium sulfate dihydrate granules or block under gaseous phase;
(U4) Granules size adjusting process
A process of adjusting granules size so that the volume is $5 \times 10^{-13}$ m$^3$ or larger.

**19.** A medical calcium phosphate composition that satisfies all the following (V1) to (V3) conditions, and at least one condition selected from the group consisting of (V4) to (V10), and optionally satisfying (V11) or (V12).

(V1) a volume is $1 \times 10^{-12}$ m$^3$ or larger;
(V2) remaining materials after acid dissolution are 1.0 mass% or less;
(V3) it is substantially a pure calcium phosphate as medical composition and is one selected from the group consisting of carbonate apatite, apatite containing $HPO_4$ group, tricalcium phosphate, whitlockite, calcium hydrogen phosphate;
(V4) it is a honeycomb structure comprising a plurality of through-holes extending in one direction (with the proviso that a honeycomb structure that does not satisfy any of the following condition is excluded: a composition is tricalcium phosphate, wherein a volume of pores which pore diameter is 10 $\mu$m or smaller with respect to a mass of the honeycomb structure analyzed by mercury intrusion porosimetry is 0.01 cm$^3$/g or more; and a diameter of the circle that passes through both ends of any one of the through-holes and a center of the through-hole, is 1 cm or longer, and 50 cm or shorter; The surface roughness of the surface of partition wall of honeycomb structure along the through-holes direction in arithmetic average roughness (Ra) is 0.7 $\mu$m or larger.) (V5) it is a granule bonded-porous structure comprising a plurality of granules which maximum diameter is 50 $\mu$m or

longer and 500 $\mu$m or shorter, formed by being bonded to each other, and comprising a plurality of through-holes extending in plural directions, wherein a volume of pores with a pore diameter of 10 $\mu$m or smaller analyzed by mercury intrusion porosimetry is 0.05 cm$^3$/g or more;

(V6) it is a pore integrated-type porous structure wherein a plurality of pores which maximum diameter is 50 $\mu$m or longer and 400 $\mu$m or shorter is integrated to the whole medical composition, not containing pores which maximum diameter is 800 $\mu$m or longer, wherein a volume of pores which maximum diameter is 10 $\mu$m or smaller in the pore integrated-type porous structure analyzed by mercury intrusion porosimetry is 0.05 cm$^3$/g or more (with the proviso that one which composition is tricalcium phosphate is excluded).

(V7) a volume of pores having a pore diameter of 6 $\mu$m or shorter with respect to a volume of pores having a pore diameter of 1 $\mu$m or larger and 6 $\mu$m or shorter analyzed by mercury intrusion porosimetry is 5 % or more;

(V8) a maximum compressive strength obtained at any one direction is higher than a standard compressive strength [S] that is calculated by the following equation (with the proviso that a honeycomb structure comprising a plurality of through-holes extending in one direction, wherein a volume of pores with a pore diameter of 10 $\mu$m or smaller with respect to a mass of the honeycomb structure analyzed by mercury intrusion porosimetry is 0.02 cm$^3$/g or smaller is excluded)

$$S = S_0 \times C \times \exp(-b \times P)$$

(wherein So and b are the constant, and So is 500, and b is 0.068, and C is the constant based on the composition; C is 1 for carbonate apatite, apatite containing $HPO_4$, tricalcium phosphate, and C is 0.5 for whitlockite, and C is 0.1 for calcium hydrogen phosphate; and P is the percentage of pores in the composition.) (V9) it is a honeycomb structure granule which minor diameter is 1 mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists;

(V10) The plural composition granules are connected with a fiber.

(V11) Composition is apatite with carbonate content is 10 mass% or larger.

(V12) Composition is apatite with carbonate content is smaller than 10 mass%.

20. A medical calcium phosphate composition that satisfies the following condition (AG1) or (AG2), and optionally satisfying the following conditions (AG3) to (AG10). (AG1) Composition is selected from the group consisting of carbonate apatite, apatite containing $HPO_4$, whitlockite, and calcium hydrogen phosphate, and granules or block with volume of 10$^{-12}$ m$^3$ or larger, and contains 0.01 mass% or larger and 3 mass % or lower silver or silver compound.

(AG2) Composition is selected from the group consisting of sintered hydroxyapatite, sintered tricalcium phosphate, carbonate apatite, apatite containing $HPO_4$, whitlockite, and calcium hydrogen phosphate, and has silver phosphate crystals bonded on the surface of calcium phosphate, and content of silver phosphate is 0.01 mass% or larger and 3 mass% or smaller.

(AG3) The above described silver compound is silver phosphate.

(AG4) Silver or silver compound is contained at the surface and inside the calcium phosphate composition, and ratio of the silver content at the surface by that at least 50 $\mu$m distant to interior direction is 1.2 or higher.

(AG5) Honeycomb structure comprising a plurality of through-holes extending in one direction

(AG6) Granules bonded porous structure comprising a plurality of granules 50 $\mu$m or longer and 500 $\mu$m or shorter in diameter at the longest, bonded to each other, and comprising a plurality of through-holes extending in plural direction.

(AG7) Pores integrated type porous structure with integrated plurality of pores which maximum diameter is 50 $\mu$m or longer and 400 $\mu$m or shorter, and without containing pores which maximum diameter is 800 $\mu$m or longer.

(AG8) a ratio of pore volume which pore diameter is 1 $\mu$m or larger and 6 $\mu$m or shorter with respect to a pore volume which pore diameter analyzed by mercury intrusion porosimetry is 5 % or more;

(AG9) Maximum compressive strength obtained at one of any direction is higher than the standard compressive strength [S] that is calculated by the following equation;

$$S = S_0 \times C \times \exp(-b \times P)$$

(wherein So and b are the constant, and So is 500, and b is 0.068, and C is the constant based on the composition:

C is 1 for carbonate apatite or apatite containing $HPO_4$, and C is 0.5 for whitlockite, and C is 0.1 for calcium hydrogen phosphate, and C is 2 for sintered hydroxyapatite and sintered tricalcium phosphate; and P is the percentage of pores in the composition)

(AG10) it is a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists.

21. The medical calcium phosphate composition according to claim 19 or 20, that satisfies at least one condition selected from (W1) to (W7):

(W1) a honeycomb structure comprising a plurality of through-holes extending in one direction, wherein ratio of pore volume with pore diameter 10 $\mu$m or smaller against mass of the honeycomb structure analyzed by mercury intrusion porosimetry is 0.01 $cm^3$/g or more;
(W2) a honeycomb structure comprising a plurality of through-holes extending in one direction, wherein a diameter of the circle that passes through both ends of any one of the through-holes and the center of the through-hole, is 1 cm or longer, and 50 cm or shorter;
(W3) a honeycomb structure with surface roughness of the surface of partition wall of honeycomb structure along the through-holes direction in arithmetic average roughness (Ra) is 0.7 $\mu$m or larger;
(W4) an aggregate of calcium phosphate with average diameter of 2 $\mu$m or larger and 8 $\mu$m or smaller;
(W5) an aggregate of calcium phosphate with sphericity of 0.9 or larger;
(W6) Mg content is $5 \times 10^{-4}$ mass% or larger, and $3 \times 10^{-3}$ mass% or smaller;
(W7) Sr content is $3 \times 10^{-3}$ mass% or larger, and $1.5 \times 10^{-2}$ mass% or smaller.

22. A method for producing a medical calcium phosphate composition wherein: the following (AH1) and (AH2) condition are essential condition, and one selected from the group consisting of (AH3) to (AH9) can be added as a selected process:

(AH1) Using raw material calcium composition, containing 0.01 mass% or larger, and 3 mass% or smaller silver or silver composition, that is one selected from the group consisting of calcium carbonate, calcium hydroxide, calcium oxide, calcium sulfate, calcium hydrogen phosphate, and is a granule or block with volume of $10^{-12}$ $m^3$ or larger,

and comprising a process of adding carbonate group to the raw composition when the raw material calcium composition is not calcium carbonate,
and comprising a process of compositional transformation reaction to any one selected from the group consisting of silver or silver compound containing carbonate apatite, apatite with $HPO_4$ group, whitlockite, and calcium hydrogen phosphate by exposure to aqueous phosphoric acid salt solution or mixed aqueous solution of phosphoric acid salt and magnesium salt;
(AH2) A process of immersing the raw material calcium composition selected from the group consisting of apatite, tricalcium phosphate, whitlockite, octacalcium phosphate, calcium hydrogen phosphate; and being granules or block with volume of $10^{-12}$ $m^3$ in aqueous solution containing silver ion, leading formation of silver phosphate to raw material calcium composition, is included;
(AH3) A process of immersing raw material calcium composition with calcium phosphate composition to first aqueous solution containing silver ion to form silver phosphate to raw material calcium composition, followed by immersing to second aqueous solution containing higher concentrated silver ion than first aqueous solution is included;
(AH4) Raw material calcium composition is a honeycomb structure comprising a plurality of through-holes extending in one direction;
(AH5) Raw material calcium composition is a granule bonded porous structure comprising a plurality of granules which maximum diameter is 50 $\mu$m or longer and 500 $\mu$m or shorter, bonded to each other, and comprising a plurality of through-holes extending in plural directions;
(AH6) Raw material calcium composition which is a pore integrated type porous structure with integrated plurality of pores which maximum diameter is 50 $\mu$m or longer and 400 $\mu$m or shorter, and not containing pores which maximum diameter is 800 $\mu$m or longer;
(AH7) Using a raw material calcium composition that a ratio of pore volume which pore diameter is 1 $\mu$m or larger and 6 $\mu$m or shorter with respect to a pore volume which pore diameter is 6 $\mu$m or shorter analysis using mercury intrusion porosimetry is 5 % or more;
(AH8) Using a raw material calcium composition with maximum compressive strength obtained at one of any direction is higher than the standard compressive strength [S] that is calculated by the following equation

$$S= S_0 \times C \times \exp(-b \times P)$$

(wherein So and b are the constant, and So is 500, and b is 0.068, and C is the constant based on composition. C is 1 for carbonate apatite and apatite containing $HPO_4$ group; 0.5 for whitlockite; 0.1 for calcium hydrogen phosphate; 2 for other composition; and P is the percentage of pores in the composition) (AH9) Using a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists.

23. A method for producing the medical calcium carbonate composition according to any one of claims 19 to 21 wherein: one of the following (AI1) to (AI4) condition is satisfied:

(AI1) Using calcium carbonate powder with mean particle diameter is 2 $\mu$m or larger, and 8 $\mu$m or smaller;
(AI2) Using calcium carbonate powder with sphericity 0.9 or larger;
(AI3) Using calcium carbonate powder containing $5 \times 10^{-4}$ mass% or larger, and $3 \times 10^{-3}$ mass% or smaller Mg;
(AI4) Using calcium carbonate powder containing $3 \times 10^{-3}$ mass% or larger, and $1.5 \times 10^{-2}$ mass% Sr.

24. A method for producing the medical phosphate calcium composition according to any one of claims 19 to 21, comprising adding phosphoric acid component to the medical calcium carbonate composition according to any one of claims 1 to 4, or to the medical calcium carbonate composition produced by the method according to any one of claims 5 to 16, wherein the medical calcium carbonate composition is immersed in at least one of the aqueous solution selected from the group of (X1) to (X5), to add phosphoric component to the medical calcium carbonate composition:

(X1) Aqueous solution containing phosphoric acid component with pH 8.5 or higher;
(X2) Aqueous solution containing phosphoric acid component with pH lower than 8.5;
(X3) Aqueous solution containing both phosphoric acid component and carbonate component at a concentration of 0.5 mol/L or lower of pH 8.5 or higher;
(X4) Aqueous solution containing both phosphoric acid component and carbonate component at a concentration of 0.5 mol/L or lower of pH lower than pH 8.5
(X5) Aqueous solution containing both phosphoric acid component and magnesium component.

25. A method for producing the medical calcium phosphate composition according to any one of claims 19 to 21, by adding phosphorous acid component to the medical calcium carbonate composition according to any one of claims 1 to 4, or to the medical calcium carbonate composition produced by the method according to any one of claims 5 to 16 wherein:

the method comprises any one of the process satisfying at least one condition selected from the group of consisting of (Y1) to (Y6) condition.
(Y1) A process of partial or complete replacement of gas in the pore of medical calcium carbonate composition immersed in the aqueous solution containing phosphoric acid component, to aqueous solution containing phosphoric acid component;
(Y2) A process of partial or complete replacement of gas in the pore of medical calcium carbonate composition immersed in the aqueous solution containing phosphoric acid component, to aqueous solution containing phosphoric acid component, using vibration;
(Y3) A process of partial or complete replacement of gas in the pore of medical calcium carbonate composition immersed in the aqueous solution containing phosphoric acid component, to aqueous solution containing phosphoric acid component, by flowing the aqueous solution;
(Y4) A process of partial or complete replacement of gas in the pore of medical calcium carbonate composition immersed in the aqueous solution containing phosphoric acid component, to aqueous solution containing phosphoric acid component, by degasification;
(Y5) A process of partial or complete replacement of gas in the pore of medical calcium carbonate composition immersed in the aqueous solution containing phosphoric acid component, to a gas that has higher solubility in an aqueous solution containing phosphoric acid component;

(Y6) A process of partial or complete replacement of gas in the pore of medical calcium carbonate composition immersed in the aqueous solution containing phosphoric acid component, to a solvent that has smaller contact angle than water and has lower boiling temperature than water.

26. A method of producing a medical calcium phosphate composition wherein:
the following (Z1) to (Z4), or (Z1), (Z3), (Z4) or (Z1) to (Z3) are performed successively in this order and all performed in a same vessel:

(Z1) A process of producing medical calcium carbonate by adding carbonate component to raw material calcium composition;
(Z2) A process of washing medical calcium carbonate composition;
(Z3) A process of adding phosphate component to medical calcium carbonate composition;
(Z4) A process of washing medical calcium phosphate.

27. A medical calcium hydroxide composition that satisfies all the following (AB1) to (AB3) conditions, and at least one condition selected from the group consisting of (AB4) to (AB8):

(AB1) a volume is $10^{-12}m^3$ or larger.
(AB2) remaining materials after acid dissolution are 1.0 mass% or less;
(AB3) it is substantially a pure calcium hydroxide as medical composition;
(AB4) it is a honeycomb structure comprising a plurality of through-holes extending in one direction;
(AB5) granule bonded porous structure comprising a plurality of granules which maximum diameter is 50 $\mu$m or longer and 500 $\mu$m or shorter, bonded to each other, and comprising a plurality of through-holes extending in plural directions;
(AB6) pore integrated type porous structure with integrated plurality of pores which maximum diameter is 50 $\mu$m or longer and 400 $\mu$m or shorter, and not containing pores which maximum diameter is 800 $\mu$m or longer.
(AB7) it is a honeycomb structure granule which minor diameter is 1mm or larger, and shorter than 5 mm, wherein, when a circle with a radius of 0.2 mm from any point on a peripheral line of a perspective image is depicted, and at a triangle formed by three points: the vertex point on the peripheral line and two points made by an intersection of the circle and a line of perspective image, no vertex point that the interior angle is 90° or smaller at the triangle exists;
(AB8) plural composition granules are connected with a fiber.

28. A method for producing the medical calcium hydroxide composition according to claim 27 that satisfies the above described (AB4) condition, wherein:
a raw material calcium composition is calcium hydroxide and comprising the following process (AD1) and one selected from the group consisting of (AD2) to (AD5) are the essential processes, and (AD6) to (AD8) can be added as selection condition:

(AD1) Extrusion process
a process of producing a raw honeycomb structure comprising a plurality of through-holes extending in one direction, having a volume of $3 \times 10^{-11}m^3$ or larger by extruding a raw material calcium composition comprising polymer material through a honeycomb structure forming die;
(AD2) Debindering process
A process of debindering of polymer containing calcium hydroxide porous structure by thermal treatment so that the remaining materials after acid dissolution are 1.0 mass% or less;
(AD3) Hydration process via calcium oxide
A process of producing calcium hydroxide porous structure by debindering of polymer containing calcium hydroxide porous structure by thermal treatment so that the remaining materials after acid dissolution are 1.0 mass% or less, and to be calcium oxide porous structure, followed by hydration;
(AD4) Hydration process via calcium carbonate and calcium oxide
A hydration process via calcium carbonate and calcium oxide for producing calcium hydroxide porous structure by heat treatment of polymer containing calcium hydroxide porous structure in the presence of carbon dioxide, followed by debindering so that the remaining materials after acid dissolution are 1.0 mass% or less, and to be calcium oxide porous structure, followed by hydration;
(AD5) A production process from calcium carbonate porous structure
A process of fabricating calcium hydroxide porous structure by debindering polymer containing calcium hydroxide porous structure so that the remaining materials after acid dissolution is 1.0 mass% or less, and to be calcium oxide porous structure, followed by hydration of calcium oxide porous structure;
(AD6) Forming process after extrusion process
A process of foaming honeycomb structure consisting of polymer containing raw material calcium composition to desired foam through softening by thermal treatment, followed by pressure loading;
(AD7) Removal process of peripheral wall
A process of removing peripheral wall after the extrusion process or the forming process after extrusion process, and before debindering and carbonation process;
(AD8) Forming process after removal process of peripheral wall
A process of foaming honeycomb structure consisting of polymer containing raw material calcium composition to desired foam through softening by thermal treatment, after removal process of peripheral wall.

29. A method for producing the medical calcium hydroxide composition according to claim 27 that satisfies the above described (AB5) condition, comprising the following process (AE1), (AE2) and at least one selected from the group consisting of the following (AD2) to (AD5) as essential processes:

(AE1) Placement process
A process of placing polymer containing calcium hydroxide granules with a volume of $10^{-12} m^3$ or more in a reaction vessel;
(AE2) Granules bonding process
A process of producing granules bonded-porous structure comprising a plurality of through-holes extending in plural directions, formed by bonding a plurality of granules which maximum diameter is 50 $\mu$m or longer and 500 $\mu$m or shorter, and having a volume of $3 \times 10^{-11}$ m$^3$ or larger,
based on heat treatment of the granules in the reaction vessel so that the surface is softened and fused one another, or dissolution of the granule surface so that the surface is bonded to each other, or treatment with a plasticizer so that the granule surface is fused one another;
(AD2) Debindering process
A process of debindering of polymer containing calcium hydroxide porous structure by thermal treatment so that the remaining materials after acid dissolution are 1.0 mass% or less
(AD3) Hydration process via calcium oxide
A process of producing calcium hydroxide porous structure by debindering of polymer containing calcium hydroxide porous structure by thermal treatment so that the remaining materials after acid dissolution is 1.0 mass% or less, and to be calcium oxide porous structure, followed by hydration.
(AD4) Hydration process via calcium carbonate and calcium oxide
A hydration process via calcium carbonate and calcium oxide for producing calcium hydroxide porous structure by heat treatment of polymer containing calcium hydroxide porous structure in the presence of carbon dioxide, followed by debindering so that the remaining materials after acid dissolution is 1.0 mass% or less, and to be calcium oxide porous structure, followed by hydration.
(AD5) A production process from calcium carbonate porous structure
A process of fabricating calcium hydroxide porous structure by debindering polymer containing calcium hydroxide porous structure so that the remaining materials after acid dissolution is 1.0 mass% or less, and to be calcium oxide porous structure, followed by hydration of calcium oxide porous structure.

30. A method for producing the medical calcium hydroxide composition according to claim 27 using calcium hydroxide porous structure or calcium carbonate porous structure, wherein:
calcium oxide porous structure is produced using calcium hydroxide porous structure or calcium carbonate porous structure, followed by producing calcium hydroxide porous structure by its hydration.

31. A method for producing the medical calcium composition according to any one of claims 9 to 11, 14 and 29 that satisfies at least one condition from the following (AF1) to (AF3) in the placement-closing process or placement process:

(AF1) Sphericity of the granules is 0.9 or larger;
(AF2) The granules are hollow;
(AF3) granules with a bulk volume of 105% or more with respect to the reaction vessel are placed in the reaction vessel.

32. A bone defect reconstruction kit comprising a solid portion that contains vaterite and $\alpha$-tricalcium phosphate and a liquid portion that contains phosphoric acid salt, and set to form carbonate apatite when the solid portion and liquid portion are mixed.

33. The bone defect reconstruction kit according to claim 32 wherein: amount of vaterite in the solid portion is 10 mass% or larger and 60 mass% or smaller.

34. The bone defect reconstruction kit according to claim 32 or 33, wherein the liquid portion contains at least one selected from, acid containing plural carboxy groups, hydrogen sulfite salt, cellulose derivative, dextran sulfate salt, chondroitin sulfate salt, alginic acid salt, glucomannan.

35. The bone defect reconstruction kit according to any one of claims 32 to 34, wherein the volume of vaterite in the solid portion is $10^{-12}$ m$^3$ or larger.

36. The bone defect reconstruction kit according to any one of claims 32 to 34, wherein the vaterite's average diameter is 6 $\mu$m or smaller.

Fig. 1

Fig. 2

Fig. 3

$2\theta$(degree)

Fig. 4

$2\theta$(degree)

Fig. 5

Fig. 6

# Fig. 7

# Fig. 8

# Fig. 9

# Fig. 10

# Fig. 11

(a) CO₃Ap (0%) 4W    (b) CO₃Ap (30%) 4W    (c) CO₃Ap (40%) 4W    (d) HAp(40%) 4W

(e) CO₃Ap (0%) 12W   (f) CO₃Ap (30%) 12W   (g) CO₃Ap (40%) 12W   (h) CO₃Ap(40%) 12W

# Fig. 12

Fig. 13

Fig. 14

# Fig. 15

# Fig. 16

Fig. 17

## INTERNATIONAL SEARCH REPORT

| | International application No. |
|---|---|
| | PCT/JP2020/032323 |

**A. CLASSIFICATION OF SUBJECT MATTER**
A61L 27/02(2006.01)i; A61K 47/02(2006.01)i; A61L 27/12(2006.01)i; A61L 27/56(2006.01)i; C01B 25/32(2006.01)i; C01F 11/18(2006.01)i; C12M 3/00(2006.01)i
FI:     A61L27/02; C1F11/18 B; C01F11/18 H; A61L27/12; A61L27/56; A61K47/02; C12M3/00 A; C01B25/32
According to International Patent Classification (IPC) or to both national classification and IPC

**B. FIELDS SEARCHED**

Minimum documentation searched (classification system followed by classification symbols)
A61L27/02; A61K47/02; A61L27/12; A61L27/56; C01B25/32; C01F11/18; C12M3/00

Documentation searched other than minimum documentation to the extent that such documents are included in the fields searched
Published examined utility model applications of Japan       1922-1996
Published unexamined utility model applications of Japan     1971-2020
Registered utility model specifications of Japan             1996-2020
Published registered utility model applications of Japan     1994-2020

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN); Science Direct

**C. DOCUMENTS CONSIDERED TO BE RELEVANT**

| Category* | Citation of document, with indication, where appropriate, of the relevant passages | Relevant to claim No. |
|---|---|---|
| Y | WO 2016/035751 A1 (KYUSHU UNIVERSITY) 10 March 2016 (2016-03-10) claim 52, example 11, paragraphs [0182], [0304] | 1-3, 5-6, 14-16, 31 |
| Y | WO 2015/005205 A1 (NAGOYA INSTITUTE OF TECHNOLOGY) 15 January 2015 (2015-01-15) paragraph [0013] | 1-3, 5-6, 14-16, 31 |
| A | KR 10-2012-0046529 A (KOREA ADVANCED INSTITUTE OF SCIENCE AND TECHNOLOGY) 10 May 2012 (2012-05-10) | 1-3, 5-6, 14-16, 31 |
| A | US 2015/0307400 A1 (CALERA CORPORATION) 29 October 2015 (2015-10-29) | 1-3, 5-6, 14-16, 31 |
| A | KR 10-2018-0029782 A (RESEARCH INSTITUTE OF INDUSTRIAL SCIENCE & TECHNOLOGY) 21 March 2018 (2018-03-21) | 1-3, 5-6, 14-16, 31 |

☐ Further documents are listed in the continuation of Box C.      ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 November 2020 (17.11.2020) | 24 November 2020 (24.11.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
|---|
| PCT/JP2020/032323 |

**Box No. II       Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III       Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: Parts of 1-3, 5-6, 14-16, and 31

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2020/032323

<Continuation of Box No. III>

(Invention 1) Parts of claims 1-3, 5-6, 14-16, and 31

Document 1 indicates that calcium sulfate interconnected porous body granules were immersed in a sodium carbonate aqueous solution at a 2 molar concentration at 4 °C to obtain a calcium carbonate composition consisting of vaterite and calcite. In addition, document 1 also indicates that the volume was $2 \times 10^{-9}$ m$^3$ (example 11). Furthermore, document 1 also indicates that the obtained material is used for a medical use (claim 52). In document 1, the obtained calcium carbonate composition is recognized to have an acid-dissolved residue of 1 mass% or less from the production method.

Document 1 does not indicate that the calcium carbonate composition comprises 20 mass% or more of vaterite as specified in claim 1 (D) of the present application. Therefore, said (D) comprising 20 mass% or more of vaterite is considered a special technical feature, and thus the invention satisfying conditions (A)-(D) of the invention in claim 1, the invention in claims 2-3 and 5-6, the invention referring to claims 5 and 6 of the invention in claims 14-16, and parts referring to claim 14 referring to claims 5 and 6 of the invention in claim 31 are classified as invention 1.

(Invention 2) Parts of claims 1, 3-4, 7-8, 13-16, and 31

The invention satisfying conditions (A)-(C) and (E) of the invention in claim 1 cannot be said to have technical features identical or corresponding to those of the invention satisfying conditions (A)-(D) of the invention in claim 1 classified as invention 1. In addition, the invention satisfying conditions (A)-(C) and (E) of the invention in claim 1 is not dependent on, and is not substantially identical or equivalent to any of the claims classified as invention 1.

Therefore, the invention satisfying conditions (A)-(C) and (E) of the invention in claim 1 cannot be classified as invention 1.

In addition, the invention satisfying conditions (A)-(C) and (E) of the invention in claim 1 has the special technical feature of a honeycomb structure comprising a plurality of through holes extending in one direction, wherein in the fine pore distribution measurement by a mercury intrusion method, the fine pore volume having a fine pore diameter of 10 μm or less with respect to the mass of the honeycomb structure is greater than 0.02 cm$^3$/g.

Therefore, the invention satisfying conditions (A)-(C) and (E) of the invention in claim 1, the invention in claims 3-4, 7-8, and 13, the invention referring to claims 7-8 and 13 of the invention in claims 14-16, and parts referring to claim 14 referring to claims 7-8 and 13 of the invention in claim 31 are classified as invention 2.

(Invention 3) Parts of claims 1, 9-11, 13-16, and 31

The invention satisfying conditions (A)-(C) and (F) of the invention in claim 1 cannot be said to have technical features identical or corresponding to those of invention 1 and invention 2. In addition, the invention satisfying conditions (A)-(C) and (F) of the invention in claim 1 is not dependent on, and is not substantially identical or equivalent to any of each claim classified as invention 1 and invention 2.

Therefore, the invention satisfying conditions (A)-(C) and (F) of the invention in claim 1 cannot be classified as invention 1 and invention 2.

In addition, the invention satisfying conditions (A)-(C) and (F) of the invention in claim 1 has the special technical feature of a granule binding porous body which comprises a plurality of through holes extending in multiple directions and which is formed by the binding of a plurality of granules having a maximum diameter length of 50 μm to 500 μm, wherein the fine pore volume having a fine pore diameter of 10 μm or less of said granule binding porous body is 0.05 cm$^3$/g or more as measured by the mercury intrusion method. Therefore, the invention satisfying conditions (A)-(C) and (F) of the invention in claim 1, the invention in claims 9-11, and 13, the invention referring to claims 9-11 and 13 of the invention in claims 14-16, and parts referring to claim 14 referring to claims 9-11 and 13 of the invention in claim 31 are classified as invention 3.

(Invention 4) Parts of claims 1, 12-16, and 31
　　　The invention satisfying conditions (A)-(C) and (G) of the invention in claim 1 cannot be said to have technical features identical or corresponding to those of inventions 1 to 3. In addition, the invention satisfying conditions (A)-(C) and (G) of the invention in claim 1 is not dependent on, and is not substantially identical or equivalent to any of each claim classified as inventions 1 to 3.
　　　Therefore, the invention satisfying conditions (A)-(C) and (G) of the invention in claim 1 cannot be classified as inventions 1 to 3. In addition, the invention satisfying conditions (A)-(C) and (G) of the invention in claim 1 has the special technical feature of a pore integrated porous body in which, in the entire medical composition, a plurality of pores having a maximum diameter length of 50 μm to 400 μm are integrated and which do not include pores having a maximum diameter length of 800 μm or more, wherein the fine pore volume having a fine pore diameter of 10 μm or less of said pore integrated porous body is 0.05 cm$^3$/g or more as measured by the mercury intrusion method.
　　　Therefore, the invention satisfying conditions (A)-(C) and (G) of the invention in claim 1, the invention in claims 12-13, the invention referring to claims 9-11 and 13 of the invention in claims 14-16, and parts referring to claim 14 referring to claims 12-13 of the invention in claim 31 are classified as invention 4.

(Invention 5) Part of claim 1
　　　The invention satisfying conditions (A)-(C) and (H) of the invention in claim 1 cannot be said to have technical features identical or corresponding to those of inventions 1 to 4.
　　　In addition, the invention satisfying conditions (A)-(C) and (H) of the invention in claim 1 is not dependent on, and is not substantially identical or equivalent to any of each claim classified as inventions 1 to 4. Therefore, the invention satisfying conditions (A)-(C) and (H) of the invention in claim 1 cannot be classified as inventions 1 to 4.
　　　In addition, the invention satisfying conditions (A)-(C) and (H) of the invention in claim 1 has the special technical feature in that the ratio of the fine pore volume having a fine pore diameter of 1 μm to 6 μm with respect to the fine pore volume having a fine pore diameter of 6 μm or less is 10% or more as measured by the mercury intrusion method, and thus the part satisfying conditions (A)-(C) and (H) of the invention in claim 1 is classified as invention 5.

(Invention 6) Part of claim 1

The invention satisfying conditions (A)-(C) and (I) of the invention in claim 1 cannot be said to have technical features identical or corresponding to those of inventions 1 to 5.

In addition, the invention satisfying conditions (A)-(C) and (I) of the invention in claim 1 is not dependent on, and is not substantially identical or equivalent to any of each claim classified as inventions 1 to 5. Therefore, the invention satisfying conditions (A)-(C) and (I) of the invention in claim 1 cannot be classified as inventions 1 to 5.

In addition, the invention satisfying conditions (A)-(C) and (I) of the invention in claim 1 has the special technical feature in that a maximum compressive strength obtained from a certain direction is equal to or greater than a standard compressive strength [S] calculated by the following formula (however, excluding a honeycomb structure comprising a plurality of through holes extending in one direction, wherein as measured by a mercury intrusion method, the fine pore volume having a fine pore diameter of 10 μm or less with respect to the mass of the honeycomb structure is 0.02 $cm^3$/g or less), and thus the part satisfying conditions (A)-(C) and (I) of the invention in claim 1 is classified as invention 6.

(Invention 7) Part of claim 1

The invention satisfying conditions (A)-(C) and (J) of the invention in claim 1 cannot be said to have technical features identical or corresponding to those of inventions 1 to 6.

In addition, the invention satisfying conditions (A)-(C) and (J) of the invention in claim 1 is not dependent on, and is not substantially identical or equivalent to any of each claim classified as inventions 1 to 6.

Therefore, the invention satisfying conditions (A)-(C) and (J) of the invention in claim 1 cannot be classified as inventions 1 to 6.

In addition, the invention satisfying conditions (A)-(C) and (J) of the invention in claim 1 has the special technical feature of a honeycomb structure granule having a short diameter of 1 mm to 5 mm (exclusive of 5), wherein in a perspective chart of the composition, a circle having a radius of 0.2 mm is drawn from any point on a peripheral line in the perspective chart, and in a triangle formed by three points on which the circle and the peripheral line in the perspective chart cross, there is no point having an angle of 90° or less, the angle being formed when any point on the peripheral line in the perspective chart is the apex, and thus the part satisfying conditions (A)-(C) and (J) of the invention in claim 1 is classified as invention 7.

(Invention 8) Part of claim 1

The invention satisfying conditions (A)-(C) and (K) of the invention in claim 1 cannot be said to have technical features identical or corresponding to those of inventions 1 to 7.

In addition, the invention satisfying conditions (A)-(C) and (K) of the invention in claim 1 is not dependent on, and is not substantially identical or equivalent to any of each claim classified as inventions 1 to 7.

Therefore, the invention satisfying conditions (A)-(C) and (K) of the invention in claim 1 cannot be classified as inventions 1 to 7.

In addition, the invention satisfying conditions (A)-(C) and (K) of the invention in claim 1 has the special technical feature in that a plurality of composition particles are connected with a fiber, and thus the part satisfying conditions (A)-(C) and (K) of the invention in claim 1 is classified as invention 8.

Form PCT/ISA/210 (extra sheet) (January 2015)

| INTERNATIONAL SEARCH REPORT | International application No. |
|---|---|
| | PCT/JP2020/032323 |

(Invention 9) Claims 17-18
Claims 17-18 cannot be said to have technical features identical or corresponding to those of inventions 1-8.
Furthermore, claims 17-18 are not dependent on, and are not substantially identical or equivalent to any of each claim classified as inventions 1 to 8.
Therefore, claims 17-18 cannot be classified as inventions 1 to 8.
In addition, claims 17-18 have the special technical feature of a calcium sulfate curable composition for a medical use, and thus the invention in claims 17-18 is classified as invention 9.

(Invention 10) Claims 19-21 and 23
Claims 19-21 and 23 cannot be said to have technical features identical or corresponding to those of inventions 1 to 9.
Furthermore, claims 19-21 and 23 are not dependent on, and are not substantially identical or equivalent to any of each claim classified as inventions 1 to 9.
Therefore, claims 19-21 and 23 cannot be classified as inventions 1 to 9.
In addition, claims 19-21 and 23 have the special technical feature of a calcium phosphate composition for a medical use or a production method thereof, and thus the invention in claims 19-21 and 23 is classified as invention 9.

(Invention 11) Claim 22
Claim 22 cannot be said to have technical features identical or corresponding to those of inventions 1-10. Furthermore, claim 22 are not dependent on, and are not substantially identical or equivalent to any of each claim classified as inventions 1 to 10. Thus, claim 22 cannot be classified as inventions 1 to 10.
In addition, claim 22 has the special technical feature of a method for producing a calcium phosphate composition for a medical use, and thus the invention in claim 22 is classified as invention 12.

(Invention 12) Claims 24-26
Claims 24-26 cannot be said to have technical features identical or corresponding to those of inventions 1-11. Furthermore, claims 24-26 are not dependent on, and are not substantially identical or equivalent to any of each claim classified as inventions 1 to 11.
Therefore, claims 24-26 cannot be classified as inventions 1 to 11.
In addition, claims 24-26 have the special technical feature of a method for producing a calcium phosphate composition for a medical use, and thus the invention in claims 24-26 is classified as invention 12.

(Invention 13) Claims 27-30
Claims 27-30 cannot be said to have technical features identical or corresponding to those of inventions 1 to 12.
Furthermore, claims 27-30 are not dependent on, and are not substantially identical or equivalent to any of each claim classified as inventions 1 to 12.
Therefore, claims 27-30 cannot be classified as inventions 1 to 12.
In addition, claims 27-30 have the special technical feature of a calcium hydroxide composition for a medical use or a production method thereof, and thus the invention in claims 27-30 is classified as invention 13.

Form PCT/ISA/210 (extra sheet) (January 2015)

(Invention 14) Claims 32-36

Claims 32-36 cannot be said to have technical features identical or corresponding to those of inventions 1-13.

Furthermore, claims 32-36 are not dependent on, and are not substantially identical or equivalent to any of each claim classified as inventions 1 to 13.

Therefore, claims 32-36 cannot be classified as inventions 1 to 13.

In addition, claims 32-36 have the special technical feature of a kit for reconstruction treatment of bone defects, and thus the invention in claims 32-36 is classified as invention 14.

Form PCT/ISA/210 (extra sheet) (January 2015)

INTERNATIONAL SEARCH REPORT
Information on patent family members

International application No.

PCT/JP2020/032323

| Patent Documents referred in the Report | Publication Date | Patent Family | Publication Date |
|---|---|---|---|
| WO 2016/035751 A1 | 10 Mar. 2016 | US 2017/0252480 A1 claim 52, example 11, paragraphs [0313], [0624] EP 3190089 A1 | |
| WO 2015/005205 A1 | 15 Jan. 2015 | US 2016/0121024 A1 paragraph [0014] EP 3020424 A1 | |
| KR 10-2012-0046529 A | 10 May 2012 | (Family: none) | |
| US 2015/0307400 A1 | 29 Oct. 2015 | (Family: none) | |
| KR 10-2018-0029782 A | 21 Mar. 2018 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004112856 A
- JP S5792520 A
- JP S6090822 A
- JP S54150397 A
- JP 2011126741 A
- JP 2011157245 A
- JP H11314915 A
- WO 2016035751 A

- JP 2016552061 A
- JP 2018140890 A
- JP H7223871 A
- WO 2018074429 A
- JP 2004298407 A
- JP 2005152006 A
- JP 2018000193 W
- JP 2016030708 A

### Non-patent literature cited in the description

- *Journal of the Adhesion Society of Japan,* 1986, vol. 22 (11), 573-579
- *The Journal Of the Society Of Materials Science, Japan,* 1986, vol. 30 (336), 6-10

- *Journal of Biomedical Research,* vol. 29, 1537-1543
- *Journal of American Ceramics Society,* vol. 36 (2), 68